# EUROPEAN PATENT APPLICATION

(11) **EP 3 511 318 A1**
(43) Date of publication of application: **17.07.2019**
(21) Application number: 19154529.2
(22) Date of filing: 28.10.2016
(51) Int. Cl.: C07C 233/49, A61K 31/195

(54) **IMMUNOSTIMULATING AGENT**

(30) Priority: 28.10.2015 JP 2015212222; 25.04.2016 JP 2016091808
(62) Divisional of application: 16819376.1
(71) Applicant: AJINOMOTO CO., INC., Chuo-ku Tokyo 104-8315 (JP)
(72) Inventor: TANAKA, Kenji, Kawasaki-shi, Kanagawa 210-8681 (JP); KURIHARA, Shigekazu, Kawasaki-shi, Kanagawa 210-8681 (JP); KUROSAWA, Wataru, Kawasaki-shi, Kanagawa 210-8681 (JP); UMISHIO, Hiroshi, Kawasaki-shi, Kanagawa 210-8681 (JP)
(74) Representative: Mewburn Ellis LLP

(57) **Abstract**

The present invention aims to provide an immunostimulating agent superior in an immunostimulatory effect, particularly a compound useful as a vaccine adjuvant, a pharmaceutical composition containing the compound, a vaccine containing the compound and an antigen.

The present invention relates to an immunostimulating agent containing at least one kind of a compound represented by the formula (I): 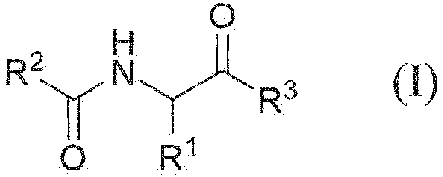 wherein each symbol is as defined in the DESCRIPTION, or a salt thereof, or at least one kind of a compound represented by the formula (II): 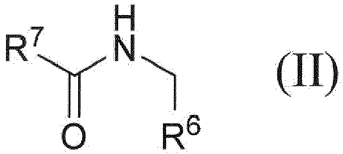 wherein each symbol is as defined in the DESCRIPTION, or a salt thereof.

## Description

### Technical Field

The present invention relates to an immunostimulating agent superior in an immunostimulatory effect, particularly a compound useful as a vaccine adjuvant, a pharmaceutical composition containing the compound, a vaccine containing the compound and an antigen and the like.

### Background Art

Vaccine includes live vaccine wherein pathogen is attenuated, whole particle vaccine wherein pathogen is inactivated, and split vaccine wherein pathogen is decomposed and only a particular component is extracted and purified. Of these, split vaccine requires addition of a compound or composition called adjuvant to enhance immunostimulatory ability thereof. Also, it is said that mucosal vaccine, cancer vaccine and vaccine for a certain kind of allergy that are being increasingly researched and developed in recent years also require addition of an adjuvant for the expression of the effects thereof. Examples of the adjuvants approved inside the country at present include aluminum salt (aluminum hydroxide gel etc.) as precipitated adjuvant, squalane as oil adjuvant, MPL that is a variant of lipopolysaccharide LPS which is a constituent component of gram negative bacteria cell wall outer membrane intrinsically having immunogenicity. The research and development of adjuvant at the global level are also advancing taking note of nucleic acid derived from CpG and Poly (I:C) and the like, variants of bacteria constituent components that activate Toll-like receptor (TLR), variants of cytokines that stimulate immune system and the like. However, these existing adjuvants including those already approved inside the country and those under research and development have the following problems.

As for aluminum salt which is a precipitated adjuvant, the adjuvant effect is questioned in some vaccines such as influenza HA vaccine, foot-and-mouth disease vaccine and the like. Also from the safety aspect, aluminum salt is known to often show granulation in the inoculation site, cause hyperimmunoglobulinemia E and the like. As for oil adjuvant such as squalene and the like, inoculation may be sometimes painful since viscosity increases by emulsifying, and inoculation site sometimes indurates since it has property to resist dispersion in the body and stay at the inoculation site. On the other hand, since MPL is a variant of LPS having immunogenicity, simultaneous inoculation with vaccine sometimes initiates strong inflammatory reaction, and sometimes accompanies pain and fever. Furthermore, adjuvants under development are also held to have safety problems such as allergy induction, strong inflammation reaction, fever initiation and the like. As for nucleic acid adjuvant, new problems are surfacing such as problems in synthesizing as a pharmaceutical product, for example, difficulty in chemical synthesis up to a chain length considered to afford an effective adjuvant effect and the like. Although adjuvants are requested to simultaneously show effectiveness and safety, conventional adjuvants already approved inside the country and those under research and development fail to completely satisfy such request as the situation stands.

In the meantime, National Institute of Allergy and Infectious Diseases (NIAID) indicates the following 12 points regarding the safety of vaccine adjuvant. 1) free of induction of autoimmune response, 2) free of antigen having crossreactivity with human antigen, 3) free of induction of allergic hypersensitive reaction, 4) should be synthesized chemically pure, 5) free of carcinogenicity, 6) free of induction of response other than the object immune response, 7) should be a substance to be quickly metabolized in the body, 8) should be safe irrespective of inoculation method, 9) should be free of teratogenicity and reproductive toxicity, 10) should have preservation stability for at least one year, 11) should be selected for the object, 12) should tolerate side reaction developed at low frequency. Also, in the guideline of EMA (European Medicines Agency) which is an organization responsible for examination of vaccine in Europe, 1) histological damage and granuloma formation of inoculation spot, 2) hypersensitivity and anaphylaxis, 3) pyrogenicity, 4) systemic toxicity, 5) reproduction toxicity, 6) genotoxicity (synthetic adjuvant alone) are recited as non-clinical toxicity test of adjuvant alone. While some parts are common or not common between US and Europe, at least the vaccine adjuvants to be developed from now should satisfy these requests.

On the other hand, while patent document 1 discloses N-(tetracosenoyl)-L-lysine, N-(tetracosenoyl)-L-methionine, and N-(tetracosenoyl)-L-threonine as antiinflammatory agents and immunomodulatory agents, their immunoregulating effect (e.g., enhancement of antibody production etc.) has not been demonstrated. Patent documents 2 and 3 disclose N-palmitoyl-L-serine, N-lauroyl-L-serine and methyl ester form thereof, N-oleoyl-L-serine, N-palmitoleyl-L-serine, N-palmitoyl-L-cysteine, N-palmitoyl-L-glycine, N-lauroyl-L-glycine and methyl ester form thereof, N-lauroyl-4-hydroxy-L-proline as therapeutic drugs for diseases relating to the anomalousness of a peripheral receptor to cannabinoid (e.g., disease related to immune system disorder, inflammatory diseases); however, their immunoregulating effect (e.g., enhancement of antibody production etc.) has not been demonstrated. Patent documents 4 and 5 disclose that a mixture of leucine and ω-3 and/or ω-6 polyvalent unsaturated fatty acid has an enhancing effect on immunofunctions (activation of NK cell, enhancement of antibody production); however, they do not disclose such effect on a condensed compound.

Also, as a compound having an immunoregulating effect, diacylcystine derivatives are known. Example 25 of patent document 6 discloses a diacylcystine compound wherein an acyl group is a carbonyl group which is substituted by an undecyl group (C₁₁ alkyl), and Example 3 of patent document 7 discloses a diacylcystine compound wherein an acyl group is a carbonyl group which is substituted by an unsaturated hydrocarbon group (C₁₇ alkenyl). Both are different from the compound of the present invention, and their adjuvant activities thereof are not sufficient.

It has been reported in non-patent document 1 that hydroxypropyl-β-cyclodextrin (hereinafter to be also referred to as HP-β-CD) potentiates immune responses. Also, patent document 8 describes that HP-β-CD potentiates immune responses by chitin particles as adjuvant, and patent documents 9 and 10 describe that HP-β-CD has a vaccine antigen (influenza vaccine and polio virus)-stabilizing effect. Furthermore, patent document 11 also describes that HP-β-CD is known as a base (Solubilizer) that increases solubility of a triazole antifungal agent, and patent document 12 also describes that HP-β-CD dissolves QS-21 known as a saponin adjuvant. However, a combination of acylagmatine and HP-β-CD has not been known before, and it is not known heretofore that acylagmatine in a dispersion state can be dissolved in the co-presence of HP-β-CD, and still shows a high immunostimulatory effect even in such dissolution state.

### Document List

### Patent documents

patent document 1: WO 02/94764
patent document 2: WO 96/18600
patent document 3: WO 03/06007
patent document 4: WO 2009/157759
patent document 5: WO 2009/157767
patent document 6: JP-A-4-230359
patent document 7: JP-A-59-219262
patent document 8: WO 2009/142988
patent document 9: WO 2015/034924
patent document 10: WO 2016/012385
patent document 11: WO 2014/191080
patent document 12: WO 99/10008

### non-patent document

non-patent document 1: M. Onishi et al., J. Immunol. 2015, 194, 2673-82

### Summary of The Invention

### Problems to be Solved by the Invention

An object of the present invention is to provide an immunostimulating agent superior in an immunostimulatory effect, particularly a compound useful as a vaccine adjuvant, a pharmaceutical composition containing the compound, and a vaccine containing the compound and an antigen.

### Means of Solving the Problems

The present inventors have conducted intensive studies in an attempt to solve the aforementioned problems and found that a compound represented by the following formula (I) (hereinafter sometimes to be referred to as compound (I)) and a salt thereof, and a compound represented by the formula (II) (hereinafter sometimes to be referred to as compound (II)) and a salt thereof have a superior immunostimulatory effect that enables enhancement of antigen-specific IgG1 subclass antibody production. They have conducted further studies based on the finding and completed the present invention. Also, the present inventors have found that compound (I), compound (II) and a salt thereof can enhance production of an antigen specific IgG2a subclass antibody and can sometimes suppress induction of IgE antibody production. Furthermore, the present inventors have also found that compound (II) or a salt thereof in a dispersion state can be in a dissolution state in the co-presence of hydroxypropyl-β-cyclodextrin, and can show a high immunostimulatory effect even in such a dissolution state.

Therefore, the present invention provides the following.
[1] An immunostimulating agent comprising at least one kind of a compound represented by the formula (I): 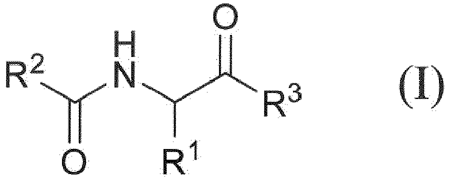 wherein
   R¹ is an amino acid side chain (excluding a cystine side chain);
   R² is a C₁₋₃₇ alkyl group; and
   R³ is a hydroxyl group, a C₁₋₆ alkoxy group or -NR⁴R⁵ wherein R⁴ and R⁵ are the same or different and each is a hydrogen atom or a C₁₋₆ alkyl group,
   or a salt thereof.
[2] The immunostimulating agent of [1], wherein R¹ is selected from the group consisting of an arginine side chain, a glutamine side chain, a glutamic acid side chain, a hydrogen atom, an isoleucine side chain, a leucine side chain, a lysine side chain, a phenylalanine side chain, and a valine side chain.
[3] The immunostimulating agent of [2], wherein R¹ is selected from the group consisting of an arginine side chain, and a glutamine side chain.
[4] The immunostimulating agent of [1], wherein R¹ is selected from the group consisting of a histidine side chain, a proline side chain, and a serine side chain.
[5] The immunostimulating agent of any of [1] - [4], wherein R² is a C₁₂₋₂₄ alkyl group.
[6] The immunostimulating agent of any of [1] - [5], wherein R³ is a hydroxyl group or a C₁₋₆ alkoxy group.
[7] The immunostimulating agent of any of [1] - [5], wherein R³ is a hydroxyl group, a C₁₋₆ alkoxy group or a -NR⁴R⁵ wherein R⁴ and R⁵ are the same or different and each is a hydrogen atom or a C₁₋₄ alkyl group.
[8] The immunostimulating agent of any of [1], [2] and [5] - [7], wherein the compound represented by the formula (I) or a salt thereof is selected from the group consisting of
   N²-hexadecanoyl-L-arginine,
   N²-hexadecanoyl-L-arginine methyl ester,
   N²-octadecanoyl-L-glutamine tert-butyl ester,
   N²-octadecanoyl-L-glutamine,
   N-docosanoyl glycine methyl ester,
   N-docosanoyl-L-leucine methyl ester,
   N-docosanoyl-L-phenylalanine methyl ester,
   N-docosanoyl-L-glutamic acid 1-methyl ester,
   N²-docosanoyl-L-lysine methyl ester,
   N-docosanoyl-L-isoleucine methyl ester,
   N-docosanoyl-L-valine methyl ester,
   N-hexadecanoyl glycine methyl ester,
   N-hexadecanoyl-L-leucine methyl ester,
   N-hexadecanoyl-L-phenylalanine methyl ester,
   N²-hexadecanoyl-L-lysine methyl ester,
   N-hexadecanoyl-L-glutamic acid 1-methyl ester,
   and a salt thereof.
[9] The immunostimulating agent of [1] or [7], wherein the compound represented by the formula (I) or a salt thereof is selected from the group consisting of
   N-acetyl-L-leucine methyl ester,
   N-hexanoyl-L-leucine methyl ester,
   N-(2-octadecyleicosanoyl)-L-leucine methyl ester,
   N-hexadecanoyl-L-leucine tert-butyl ester,
   N-hexadecanoyl-L-leucinamide,
   N²-hexadecanoyl-N¹,N¹-diethyl-L-leucinamide,
   N-hexadecanoyl-L-histidine methyl ester,
   N-hexadecanoyl-L-proline methyl ester,
   N-hexadecanoyl-L-serine methyl ester,
   N-hexadecanoyl-L-leucine hexyl ester,
   N²-hexadecanoyl-L-arginine hexyl ester,
   N²-docosanoyl-N¹,N¹-diethyl-L-glutamic acid 1-amide,
   N²-hexadecanoyl-N¹,N¹-diethyl-L-arginine amide,
   N²-docosanoyl-L-arginine hexyl ester,
   N-docosanoyl-L-leucine hexyl ester,
   N²-docosanoyl-N¹,N¹-diethyl-L-leucinamide,
   N²-docosanoyl-L-arginine amide,
   N²-docosanoyl-N¹,N¹-diethyl-L-arginine amide,
   N²-hexanoyl-L-arginine hexyl ester,
   N-hexadecanoyl-L-glutamic acid hexyl ester,
   N-docosanoyl-L-glutamic acid hexyl ester,
   N²-hexadecanoyl-L-glutamine methyl ester,
   and a salt thereof.
[10] An immunostimulating agent comprising at least one kind of a compound represented by the formula (II): 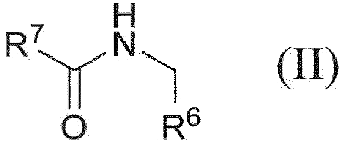 wherein
   R⁶ is an arginine side chain; and
   R⁷ is a C₁₋₃₇ alkyl group,
   or a salt thereof.
[11] The immunostimulating agent of [10], wherein R⁷ is a C₁₂₋₂₄ alkyl group.
[12] The immunostimulating agent of [10] or [11], wherein the compound represented by the formula (II) or a salt thereof is N-hexadecanoylagmatine or a salt thereof.
[13] The immunostimulating agent of [10] or [11], wherein the compound represented by the formula (II) or a salt thereof is N-docosanoylagmatine or a salt thereof.
[14] The immunostimulating agent of any of [1] - [13], wherein the aforementioned immunostimulating agent is a vaccine adjuvant.
[15] A pharmaceutical composition comprising at least one kind of a compound represented by the formula (I): 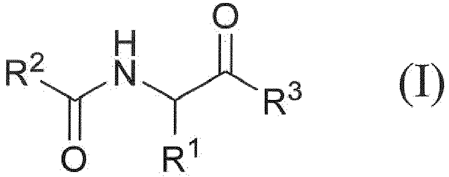 wherein
   R¹ is an amino acid side chain (excluding a cystine side chain);
   R² is a C₁₋₃₇ alkyl group; and
   R³ is a hydroxyl group, a C₁₋₆ alkoxy group or -NR⁴R⁵ wherein R⁴ and R⁵ are the same or different and each is a hydrogen atom or a C₁₋₆ alkyl group,
   or a salt thereof, and
   α -cyclodextrin.
[16] The pharmaceutical composition of [15], wherein R¹ is selected from the group consisting of an arginine side chain, a glutamine side chain, a glutamic acid side chain, a hydrogen atom, an isoleucine side chain, a leucine side chain, a lysine side chain, a phenylalanine side chain, and a valine side chain.
[17] The pharmaceutical composition of [15], wherein R¹ is selected from the group consisting of a histidine side chain, a proline side chain, and a serine side chain.
[18] The pharmaceutical composition of any of [15] - [17], wherein R² is a C₁₂₋₂₄ alkyl group.
[19] The pharmaceutical composition of any of [15] - [18], wherein R³ is a hydroxyl group or a C₁₋₆ alkoxy group.
[20] The pharmaceutical composition of any of [15] - [18], wherein R³ is a hydroxyl group, a C₁₋₆ alkoxy group or a -NR⁴R⁵ wherein R⁴ and R⁵ are the same or different and each is a hydrogen atom or a C₁₋₄ alkyl group.
[21] The pharmaceutical composition of any of [15], [16] and [18] - [20], wherein the compound represented by the formula (I) or a salt thereof is selected from the group consisting of
   N²-hexadecanoyl-L-arginine,
   N²-hexadecanoyl-L-arginine methyl ester,
   N²-octadecanoyl-L-glutamine tert-butyl ester,
   N²-octadecanoyl-L-glutamine,
   N-docosanoyl glycine methyl ester,
   N-docosanoyl-L-leucine methyl ester,
   N-docosanoyl-L-phenylalanine methyl ester,
   N-docosanoyl-L-glutamic acid 1-methyl ester,
   N²-docosanoyl-L-lysine methyl ester,
   N-docosanoyl-L-isoleucine methyl ester,
   N-docosanoyl-L-valine methyl ester,
   N-hexadecanoyl glycine methyl ester,
   N-hexadecanoyl-L-leucine methyl ester,
   N-hexadecanoyl-L-phenylalanine methyl ester,
   N²-hexadecanoyl-L-lysine methyl ester,
   N-hexadecanoyl-L-glutamic acid 1-methyl ester,
   and a salt thereof.
[22] The pharmaceutical composition of [15] or [20], wherein the compound represented by the formula (I) or a salt thereof is selected from the group consisting of
   N-acetyl-L-leucine methyl ester,
   N-hexanoyl-L-leucine methyl ester,
   N-(2-octadecyleicosanoyl)-L-leucine methyl ester,
   N-hexadecanoyl-L-leucine tert-butyl ester,
   N-hexadecanoyl-L-leucinamide,
   N²-hexadecanoyl-N¹,N¹-diethyl-L-leucinamide,
   N-hexadecanoyl-L-histidine methyl ester,
   N-hexadecanoyl-L-proline methyl ester,
   N-hexadecanoyl-L-serine methyl ester,
   N-hexadecanoyl-L-leucine hexyl ester,
   N²-hexadecanoyl-L-arginine hexyl ester,
   N²-docosanoyl-N¹,N¹-diethyl-L-glutamic acid 1-amide,
   N²-hexadecanoyl-N¹,N¹-diethyl-L-arginine amide,
   N²-docosanoyl-L-arginine hexyl ester,
   N-docosanoyl-L-leucine hexyl ester,
   N²-docosanoyl-N¹,N¹-diethyl-L-leucinamide,
   N²-docosanoyl-L-arginine amide,
   N²-docosanoyl-N¹,N¹-diethyl-L-arginine amide,
   N²-hexanoyl-L-arginine hexyl ester,
   N-hexadecanoyl-L-glutamic acid hexyl ester,
   N-docosanoyl-L-glutamic acid hexyl ester,
   N²-hexadecanoyl-L-glutamine methyl ester,
   and a salt thereof.
[23] A pharmaceutical composition comprising at least one kind of a compound represented by the formula (II): 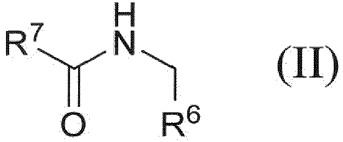 wherein
   R⁶ is an arginine side chain; and
   R⁷ is a C₁₋₃₇ alkyl group,
   or a salt thereof, and
   α -cyclodextrin.
[24] The pharmaceutical composition of [23], wherein R⁷ is a C₁₂₋₂₄ alkyl group.
[25] The pharmaceutical composition of [23] or [24], wherein the compound represented by the formula (II) or a salt thereof is N-hexadecanoylagmatine or a salt thereof.
[26] The pharmaceutical composition of [23] or [24], wherein the compound represented by the formula (II) or a salt thereof is N-docosanoylagmatine or a salt thereof.
[27] A pharmaceutical composition comprising at least one kind of a compound represented by the formula (II): 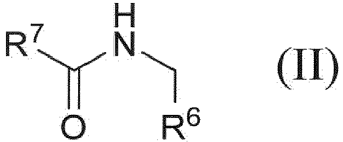 wherein
   R⁶ is an arginine side chain; and
   R⁷ is a C₁₋₃₇ alkyl group,
   or a salt thereof, and
   hydroxypropyl-β-cyclodextrin.
[28] The pharmaceutical composition of [27], wherein R⁷ is a C₁₂₋₂₄ alkyl group.
[29] The pharmaceutical composition of [27] or [28], wherein the compound represented by the formula (II) or a salt thereof is N-hexadecanoylagmatine or a salt thereof.
[30] The pharmaceutical composition of [27] or [28], wherein the compound represented by the formula (II) or a salt thereof is N-docosanoylagmatine or a salt thereof.
[31] A vaccine comprising at least one kind of a compound represented by the formula (I): 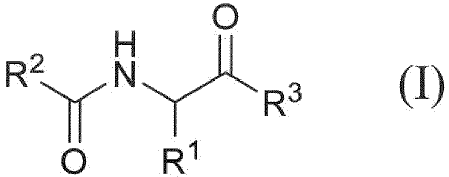 wherein
   R¹ is an amino acid side chain (excluding a cystine side chain);
   R² is a C₁₋₃₇ alkyl group; and
   R³ is a hydroxyl group, a C₁₋₆ alkoxy group or -NR⁴R⁵ wherein R⁴ and R⁵ are the same or different and each is a hydrogen atom or a C₁₋₆ alkyl group,
   or a salt thereof, and
   antigen.
[32] The vaccine of [31], wherein R¹ is selected from the group consisting of an arginine side chain, a glutamine side chain, a glutamic acid side chain, a hydrogen atom, an isoleucine side chain, a leucine side chain, a lysine side chain, a phenylalanine side chain, and a valine side chain.
[33] The vaccine of [31], wherein R¹ is selected from the group consisting of histidine side chain, a proline side chain, and a serine side chain.
[34] The vaccine of any of [31] - [33], wherein R² is a C₁₂₋₂₄ alkyl group.
[35] The vaccine of any of [31] - [34], wherein R³ is a hydroxyl group or a C₁₋₆ alkoxy group.
[36] The vaccine of any of [31] - [34], wherein R³ is a hydroxyl group, a C₁₋₆ alkoxy group or a -NR⁴R⁵ wherein R⁴ and R⁵ are the same or different and each is a hydrogen atom or a C₁₋₄ alkyl group.
[37] The vaccine of any of [31], [32] and [34] - [36], wherein the compound represented by the formula (I) or a salt thereof is selected from the group consisting of
   N²-hexadecanoyl-L-arginine,
   N²-hexadecanoyl-L-arginine methyl ester,
   N²-octadecanoyl-L-glutamine tert-butyl ester,
   N²-octadecanoyl-L-glutamine,
   N-docosanoyl glycine methyl ester,
   N-docosanoyl-L-leucine methyl ester,
   N-docosanoyl-L-phenylalanine methyl ester,
   N-docosanoyl-L-glutamic acid 1-methyl ester,
   N²-docosanoyl-L-lysine methyl ester,
   N-docosanoyl-L-isoleucine methyl ester,
   N-docosanoyl-L-valine methyl ester,
   N-hexadecanoyl glycine methyl ester,
   N-hexadecanoyl-L-leucine methyl ester,
   N-hexadecanoyl-L-phenylalanine methyl ester,
   N²-hexadecanoyl-L-lysine methyl ester,
   N-hexadecanoyl-L-glutamic acid 1-methyl ester,
   and a salt thereof.
[38] The vaccine of [31] or [36], wherein the compound represented by the formula (I) or a salt thereof is selected from the group consisting of
   N-acetyl-L-leucine methyl ester,
   N-hexanoyl-L-leucine methyl ester,
   N-(2-octadecyleicosanoyl)-L-leucine methyl ester,
   N-hexadecanoyl-L-leucine tert-butyl ester,
   N-hexadecanoyl-L-leucinamide,
   N²-hexadecanoyl-N¹,N¹-diethyl-L-leucinamide,
   N-hexadecanoyl-L-histidine methyl ester,
   N-hexadecanoyl-L-proline methyl ester,
   N-hexadecanoyl-L-serine methyl ester,
   N-hexadecanoyl-L-leucine hexyl ester,
   N²-hexadecanoyl-L-arginine hexyl ester,
   N²-docosanoyl-N¹,N¹-diethyl-L-glutamic acid 1-amide,
   N²-hexadecanoyl-N¹,N¹-diethyl-L-arginine amide,
   N²-docosanoyl-L-arginine hexyl ester,
   N-docosanoyl-L-leucine hexyl ester,
   N²-docosanoyl-N¹,N¹-diethyl-L-leucinamide,
   N²-docosanoyl-L-arginine amide,
   N²-docosanoyl-N¹,N¹-diethyl-L-arginine amide,
   N²-hexanoyl-L-arginine hexyl ester,
   N-hexadecanoyl-L-glutamic acid hexyl ester,
   N-docosanoyl-L-glutamic acid hexyl ester,
   N²-hexadecanoyl-L-glutamine methyl ester,
   and a salt thereof.
[39] A vaccine comprising at least one kind of a compound represented by the formula (II): 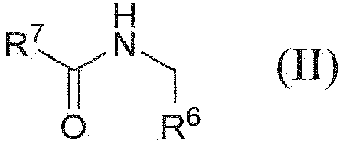 wherein
   R⁶ is an arginine side chain; and
   R⁷ is a C₁₋₃₇ alkyl group,
   or a salt thereof, and
   antigen.
[40] The vaccine of [39], wherein R⁷ is a C₁₂₋₂₄ alkyl group.
[41] The vaccine of [39] or [40], wherein the compound represented by the formula (II) or a salt thereof is N-hexadecanoylagmatine or a salt thereof.
[42] The vaccine of [39] or [40], wherein the compound represented by the formula (II) or a salt thereof is N-docosanoylagmatine or a salt thereof.
[43] The vaccine of any of [31] - [42], which is for subcutaneous administration or transnasal administration.

### Effect of the Invention

Since compound (I), compound (II) and a salt thereof have an antigen-specific IgG1 subclass antibody production-enhancing effect (immunostimulatory effect), they are useful as immunostimulating agents. Particularly, compound (I), compound (II) and a salt thereof have an immunostimulatory effect equivalent to or not less than that of conventional aluminum gel adjuvants. In addition, compound (I), compound (II) and a salt thereof also show an IgG2a subclass antibody production-enhancing effect (immunostimulatory effect). Furthermore, since compound (I), compound (II) and a salt thereof suppress induction of IgE antibody production and sometimes suppress problematic allergy inducing activity of conventional aluminum gel adjuvants, they can be effective and safe adjuvants.

According to the present invention, moreover, a pharmaceutical composition containing compound (I) or a salt thereof, or compound (II) or a salt thereof, and α-cyclodextrin, which can be easily dissolved or dispersed in saline can be provided. Also, a pharmaceutical composition containing compound (II) or a salt thereof, and hydroxypropyl-β-cyclodextrin, which can be easily dissolved or dispersed in saline can be provided. The pharmaceutical composition is superior in an antigen-specific IgG1 subclass antibody production-enhancing effect (immunostimulatory effect), and can be used as an immunostimulating agent.

According to the present invention, a vaccine comprising compound (I) or a salt thereof, or compound (II) or a salt thereof, and an antigen is provided.

### Brief Description of The Drawings

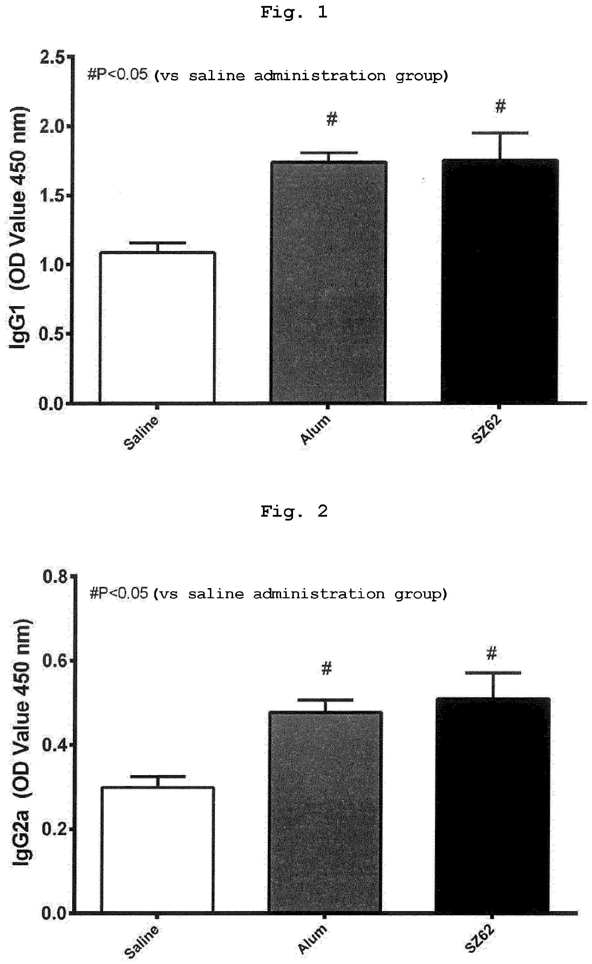

Fig. 1 is a graph showing the results of the evaluation test of adjuvant activity (IgG1 production amount) of N-hexadecanoylagmatine (SZ62: compound wherein the group corresponding to R⁷ in the formula (II) is C₁₅ alkyl group) in Example 1, wherein "Saline" shows OVA single administration group, "Alum" shows Alum administration group, and "SZ62" shows SZ62 administration group.
Fig. 2 is a graph showing the results of the evaluation test of adjuvant activity (IgG2a production amount) of N-hexadecanoylagmatine (SZ62: compound wherein the group corresponding to R⁷ in the formula (II) is C₁₅ alkyl group) in Example 2, wherein "Saline" shows OVA single administration group, "Alum" shows Alum administration group, and "SZ62" shows SZ62 administration group.

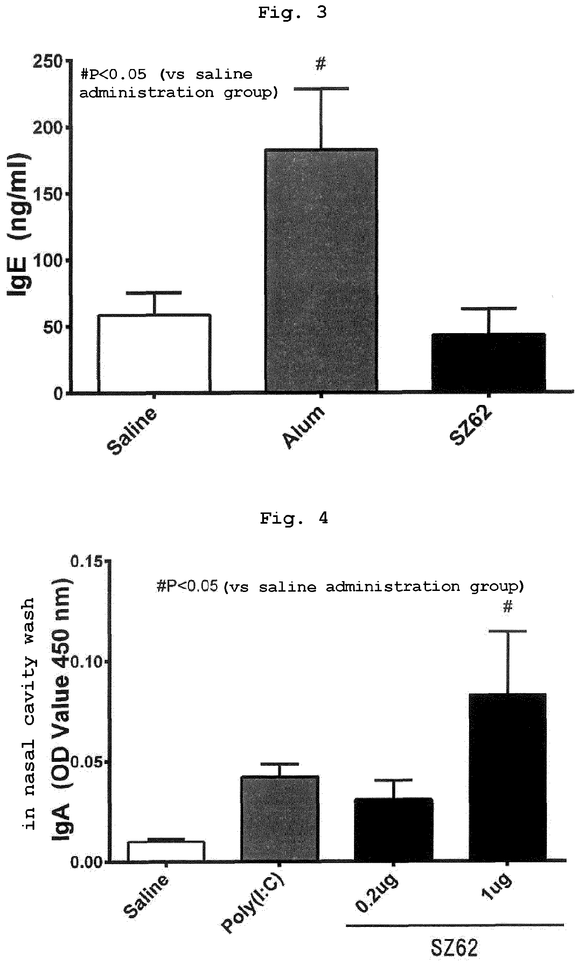

Fig. 3 is a graph showing the results of the evaluation test of allergy inducing activity of N-hexadecanoylagmatine (SZ62: compound wherein the group corresponding to R⁷ in the formula (II) is C₁₅ alkyl group) in Example 3, wherein "Saline" shows OVA single administration group, "Alum" shows Alum administration group, and "SZ62" shows SZ62 administration group.
Fig. 4 is a graph showing the results of the evaluation test of transnasal influenza vaccine adjuvant activity of N-hexadecanoylagmatine (SZ62: compound wherein the group corresponding to R⁷ in the formula (II) is C₁₅ alkyl group) in Example 4, wherein "Saline" shows influenza vaccine single administration group, "Poly(I:C)" shows Poly(I:C) administration group, "0.2 µg" shows SZ62 (0.2 µg) administration group, and "1 µg" shows SZ62 (1 µg) administration group.

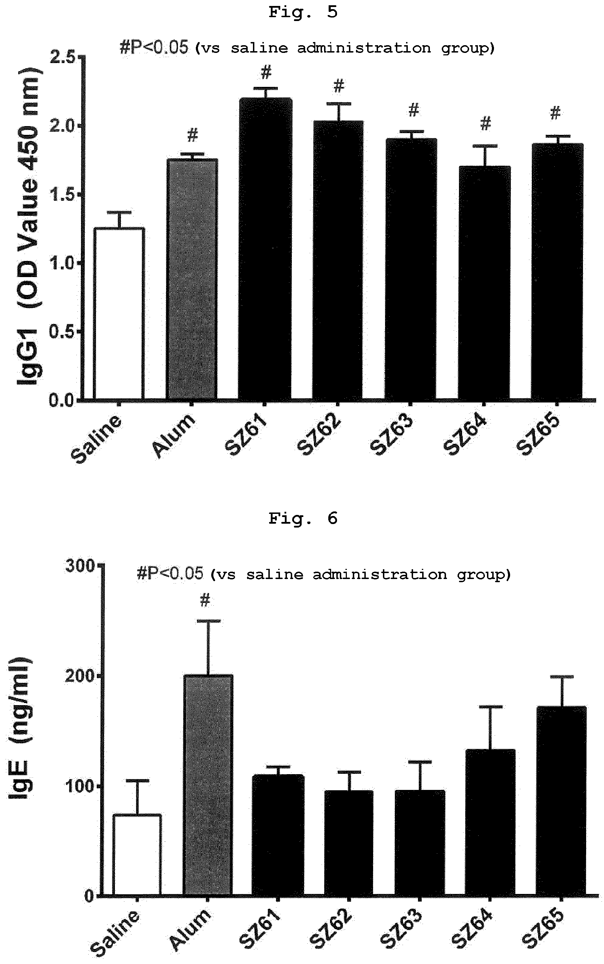

Fig. 5 is a graph showing the results of the evaluation test of adjuvant activity of N²-hexadecanoyl-L-arginine (SZ61: compound wherein, in the formula (I), the group corresponding to R¹ is arginine side chain, the group corresponding to R² is C₁₅ alkyl group, and the group corresponding to R³ is hydroxyl group), N-hexadecanoylagmatine (SZ62: compound wherein the group corresponding to R⁷ in the formula (II) is C₁₅ alkyl group), N²-hexadecanoyl-L-arginine methyl ester hydrochloride (SZ63: salt of compound wherein, in the formula (I), the group corresponding to R¹ is arginine side chain, the group corresponding to R² is C₁₅ alkyl group, and the group corresponding to R³ is methoxy), N²-octadecanoyl-L-glutamine tert-butyl ester (SZ64: compound wherein, in the formula (I), the group corresponding to R¹ is glutamine side chain, the group corresponding to R² is C₁₇ alkyl group, and the group corresponding to R³ is tert-butyloxy), N²-octadecanoyl-L-glutamine (SZ65: compound wherein, in the formula (I), the group corresponding to R¹ is glutamine side chain, the group corresponding to R² is C₁₇ alkyl group, and the group corresponding to R³ is hydroxyl group), in Example 5, wherein "Saline" shows OVA single administration group, "Alum" shows Alum administration group, "SZ61" shows SZ61 administration group, "SZ62" shows SZ62 administration group, "SZ63" shows SZ63 administration group, "SZ64" shows SZ64 administration group, and "SZ65" shows SZ65 administration group.
Fig. 6 is a graph showing the results of the evaluation test of allergy inducing activity of N²-hexadecanoyl-L-arginine (SZ61: compound wherein, in the formula (I), the group corresponding to R¹ is arginine side chain, the group corresponding to R² is C₁₅ alkyl group, and the group corresponding to R³ is hydroxyl group), N-hexadecanoylagmatine (SZ62: compound wherein the group corresponding to R⁷ in the formula (II) is C₁₅ alkyl group), N²-hexadecanoyl-L-arginine methyl ester hydrochloride (SZ63: salt of compound wherein, in the formula (I), the group corresponding to R¹ is arginine side chain, the group corresponding to R² is C₁₅ alkyl group, and the group corresponding to R³ is methoxy), N²-octadecanoyl-L-glutamine tert-butyl ester (SZ64: compound wherein, in the formula (I), the group corresponding to R¹ is glutamine side chain, the group corresponding to R² is C₁₇ alkyl group, and the group corresponding to R³ is tert-butyloxy), N²-octadecanoyl-L-glutamine (SZ65: compound wherein, in the formula (I), the group corresponding to R¹ is glutamine side chain, the group corresponding to R² is C₁₇ alkyl group, and the group corresponding to R³ is hydroxyl group), in Example 6, wherein "Saline" shows OVA single administration group, "Alum" shows Alum administration group, "SZ61" shows SZ61 administration group, "SZ62" shows SZ62 administration group, "SZ63" shows SZ63 administration group, "SZ64" shows SZ64 administration group, and "SZ65" shows SZ65 administration group.

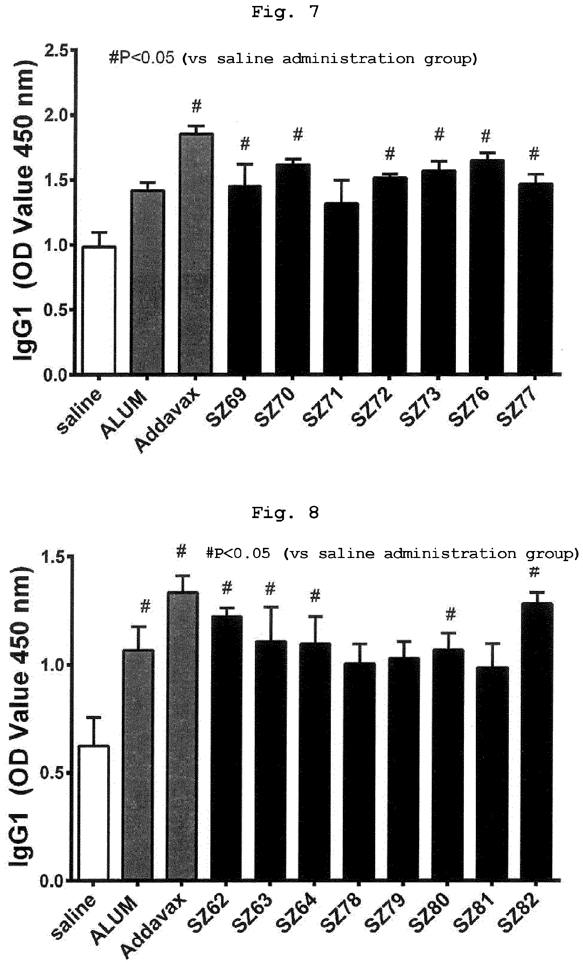

Fig. 7 is a graph showing the results of the evaluation test of adjuvant activity of N-docosanoyl glycine methyl ester (SZ69: compound wherein, in the formula (I), the group corresponding to R¹ is hydrogen atom, the group corresponding to R² is C₂₁ alkyl group, and the group corresponding to R³ is methoxy), N-docosanoyl-L-leucine methyl ester (SZ70: compound wherein, in the formula (I), the group corresponding to R¹ is leucine side chain, the group corresponding to R² is C₂₁ alkyl group, and the group corresponding to R³ is methoxy), N-docosanoyl-L-phenylalanine methyl ester (SZ71: compound wherein, in the formula (I), the group corresponding to R¹ is phenylalanine side chain, the group corresponding to R² is C₂₁ alkyl group, and the group corresponding to R³ is methoxy), N-docosanoyl-L-glutamic acid 1-methyl ester (SZ72: compound wherein, in the formula (I), the group corresponding to R¹ is glutamic acid side chain, the group corresponding to R² is C₂₁ alkyl group, and the group corresponding to R³ is methoxy), N²-docosanoyl-L-lysine methyl ester trifluoroacetic acid salt (SZ73: salt of compound wherein, in the formula (I), the group corresponding to R¹ is lysine side chain, the group corresponding to R² is C₂₁ alkyl group, and the group corresponding to R³ is methoxy), N-docosanoyl-L-isoleucine methyl ester (SZ76: compound wherein, in the formula (I), the group corresponding to R¹ is isoleucine side chain, the group corresponding to R² is C₂₁ alkyl group, and the group corresponding to R³ is methoxy), and N-docosanoyl-L-valine methyl ester (SZ77: compound wherein, in the formula (I), the group corresponding to R¹ is valine side chain, the group corresponding to R² is C₂₁ alkyl group, and the group corresponding to R³ is methoxy), in Example 7, wherein "saline" shows OVA single administration group, "ALUM" shows Alum administration group, "Addavax" shows Addavax™ administration group, "SZ69" shows SZ69 administration group, "SZ70" shows SZ70 administration group, "SZ71" shows SZ71 administration group, "SZ72" shows SZ72 administration group, "SZ73" shows SZ73 administration group, "SZ76" shows SZ76 administration group, and "SZ77" shows SZ77 administration group.
Fig. 8 is a graph showing the results of the evaluation test of adjuvant activity of N-hexadecanoylagmatine (SZ62: compound wherein the group corresponding to R⁷ in the formula (II) is C₁₅ alkyl group), N²-hexadecanoyl-L-arginine methyl ester hydrochloride (SZ63: salt of compound wherein, in the formula (I), the group corresponding to R¹ is arginine side chain, the group corresponding to R² is C₁₅ alkyl group, and the group corresponding to R³ is methoxy), N²-octadecanoyl-L-glutamine tert-butyl ester (SZ64: compound wherein, in the formula (I), the group corresponding to R¹ is glutamine side chain, the group corresponding to R² is C₁₇ alkyl group, and the group corresponding to R³ is tert-butyloxy), N-hexadecanoyl glycine methyl ester (SZ78: compound wherein, in the formula (I), the group corresponding to R¹ is hydrogen atom, the group corresponding to R² is C₁₅ alkyl group, and the group corresponding to R³ is methoxy), N-hexadecanoyl-L-leucine methyl ester (SZ79: compound wherein, in the formula (I), the group corresponding to R¹ is leucine side chain, the group corresponding to R² is C₁₅ alkyl group, and the group corresponding to R³ is methoxy), N-hexadecanoyl-L-phenylalanine methyl ester (SZ80: compound wherein, in the formula (I), the group corresponding to R¹ is phenylalanine side chain, the group corresponding to R² is C₁₅ alkyl group, and the group corresponding to R³ is methoxy), N²-hexadecanoyl-L-lysine methyl ester trifluoroacetic acid salt (SZ81: salt of compound wherein, in the formula (I), the group corresponding to R¹ is lysine side chain, the group corresponding to R² is C₁₅ alkyl group, and the group corresponding to R³ is methoxy), and N-hexadecanoyl-L-glutamic acid 1-methyl ester (SZ82: compound wherein, in the formula (I), the group corresponding to R¹ is glutamic acid side chain, the group corresponding to R² is C₁₅ alkyl group, and the group corresponding to R³ is methoxy), wherein "saline" shows OVA single administration group, "ALUM" shows Alum administration group, "Addavax" shows Addavax™ administration group, "SZ62" shows SZ62 administration group, "SZ63" shows SZ63 administration group, "SZ64" shows SZ64 administration group, "SZ78" shows SZ78 administration group, "SZ79" shows SZ79 administration group, "SZ80" shows SZ80 administration group, "SZ81" shows SZ81 administration group, and "SZ82" shows SZ82 administration group.

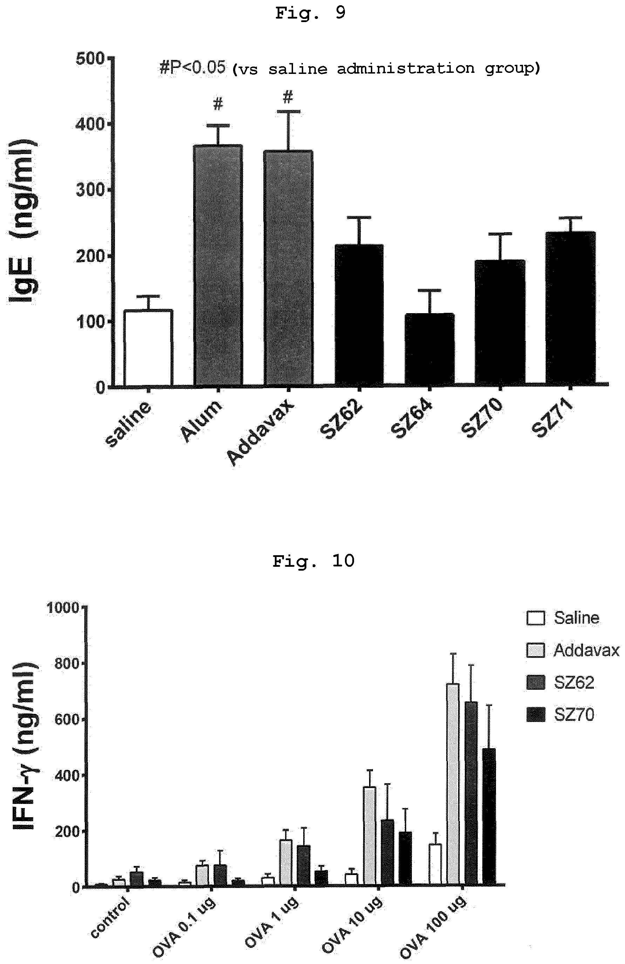

Fig. 9 is a graph showing the results of the evaluation test of allergy inducing activity of N-hexadecanoylagmatine (SZ62: compound wherein the group corresponding to R⁷ in the formula (II) is C₁₅ alkyl group), N²-octadecanoyl-L-glutamine tert-butyl ester (SZ64: compound wherein, in the formula (I), the group corresponding to R¹ is glutamine side chain, the group corresponding to R² is C₁₇ alkyl group, and the group corresponding to R³ is tert-butyloxy), N-docosanoyl-L-leucine methyl ester (SZ70: compound wherein, in the formula (I), the group corresponding to R¹ is leucine side chain, the group corresponding to R² is C₂₁ alkyl group, and the group corresponding to R³ is methoxy), N-docosanoyl-L-phenylalanine methyl ester (SZ71: compound wherein, in the formula (I), the group corresponding to R¹ is phenylalanine side chain, the group corresponding to R² is C₂₁ alkyl group, and the group corresponding to R³ is methoxy), in Example 9, wherein "saline" shows OVA single administration group, "Alum" shows Alum administration group, "Addavax" shows Addavax™ administration group, "SZ62" shows SZ62 administration group, "SZ64" shows SZ64 administration group, "SZ70" shows SZ70 administration group, and "SZ71" shows SZ71 administration group.
Fig. 10 is a graph showing the results of the evaluation test of Th1 response of N-hexadecanoylagmatine (SZ62: compound wherein the group corresponding to R⁷ in the formula (II) is C₁₅ alkyl group), and N-docosanoyl-L-leucine methyl ester (SZ70: compound wherein, in the formula (I), the group corresponding to R¹ is leucine side chain, the group corresponding to R² is C₂₁ alkyl group, and the group corresponding to R³ is methoxy), in Example 10, wherein "Saline" shows OVA single administration group, "Addavax" shows Addavax™ administration group, "SZ62" shows SZ62 administration group, and "SZ70" shows SZ70 administration group. This graph repeatedly shows "Saline", "Addavax", "SZ62" and "SZ70" in this order from the left.

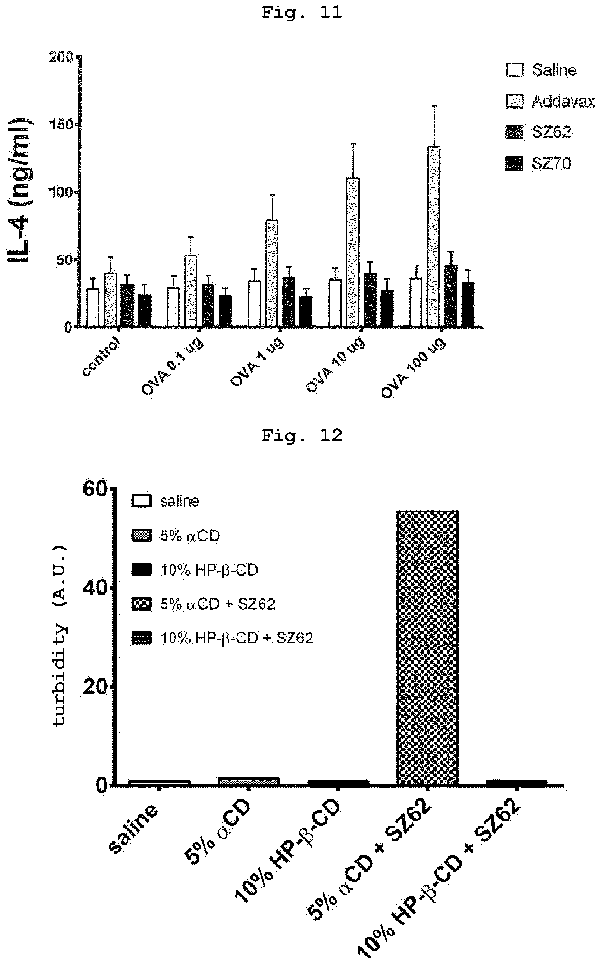

Fig. 11 is a graph showing the results of the evaluation test of Th2 response of N-hexadecanoylagmatine (SZ62: compound wherein the group corresponding to R⁷ in the formula (II) is C₁₅ alkyl group), and N-docosanoyl-L-leucine methyl ester (SZ70: compound wherein, in the formula (I), the group corresponding to R¹ is leucine side chain, the group corresponding to R² is C₂₁ alkyl group, and the group corresponding to R³ is methoxy), in Example 10, wherein "Saline" shows OVA single administration group, "Addavax" shows Addavax™ administration group, "SZ62" shows SZ62 administration group, and "SZ70" shows SZ70 administration group. This graph repeatedly shows "Saline", "Addavax", "SZ62" and "SZ70" in this order from the left.
Fig. 12 is a graph showing the results of the dispersibility consideration test of N-hexadecanoylagmatine (SZ62: compound wherein the group corresponding to R⁷ in the formula (II) is C₁₅ alkyl group) in Example 11, wherein "saline" shows saline, "5% αCD" shows 5% α-cyclodextrin (αCD)-added saline, "10% HP-β-CD" shows 10% 2-hydroxypropyl-β-CD(HP-β-CD)-added saline, "5% αCD+SZ62" shows a sample prepared by adding 5% αCD-added saline to N-hexadecanoylagmatine (SZ62: compound wherein the group corresponding to R⁷ in the formula (II) is C₁₅ alkyl group) and stirring the mixture, and "10% HP-β-CD+SZ62" shows a sample prepared by adding 10% HP-β-CD-added saline to SZ62 and stirring the mixture.

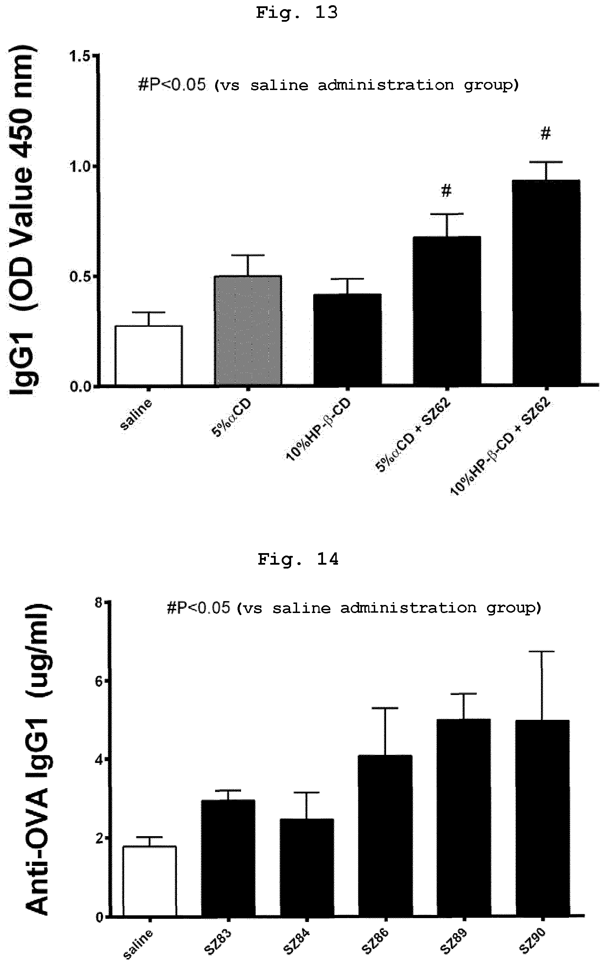

Fig. 13 is a graph showing the results of the evaluation test of adjuvant activity of N-hexadecanoylagmatine (SZ62: compound wherein the group corresponding to R⁷ in the formula (II) is C₁₅ alkyl group) in Example 12 (5% αCD-added saline, 10% HP-β-CD-added saline), wherein "saline" shows OVA single administration group, "5% αCD" shows 5% αCD-added saline administration group, "10% HP-β-CD" shows 10% HP-β-CD-added saline administration group, "5% αCD+SZ62" shows a group administered with a sample prepared by adding 5% αCD-added saline to SZ62 and stirring the mixture, and "10% HP-β-CD+SZ62" shows a group administered with a sample prepared by adding 10% HP-β-CD-added saline to SZ62 and stirring the mixture.
Fig. 14 is a graph showing the results of the evaluation test of adjuvant activity after primary immunization of N-acetyl-l-leucine methyl ester (SZ83: compound wherein, in the formula (I), the group corresponding to R¹ is leucine side chain, the group corresponding to R² is C₁ alkyl group, the group corresponding to R³ is methoxy), N-hexanoyl-L-leucine methyl ester (SZ84: compound wherein, in the formula (I), the group corresponding to R¹ is leucine side chain, the group corresponding to R² is C₅ alkyl group, the group corresponding to R³ is methoxy), N-(2-octadecyleicosanoyl)-L-leucine methyl ester (SZ86: compound wherein, in the formula (I), the group corresponding to R¹ is leucine side chain, the group corresponding to R² is C₃₇ alkyl group, the group corresponding to R³ is methoxy), N-hexadecanoyl-L-leucine tert-butyl ester (SZ89: compound wherein, in the formula (I), the group corresponding to R¹ is leucine side chain, the group corresponding to R² is C₁₅ alkyl group, the group corresponding to R³ is tert-butoxy), N-hexadecanoyl-L-leucinamide (SZ90: compound wherein, in the formula (I), the group corresponding to R¹ is leucine side chain, the group corresponding to R² is C₁₅ alkyl group, the group corresponding to R³ is amino), in Example 13, wherein "saline" shows OVA single administration group, "SZ83" shows SZ83 administration group, "SZ84" shows SZ84 administration group, "SZ86" shows SZ86 administration group, "SZ89" shows SZ89 administration group, "SZ90" shows SZ90 administration group.

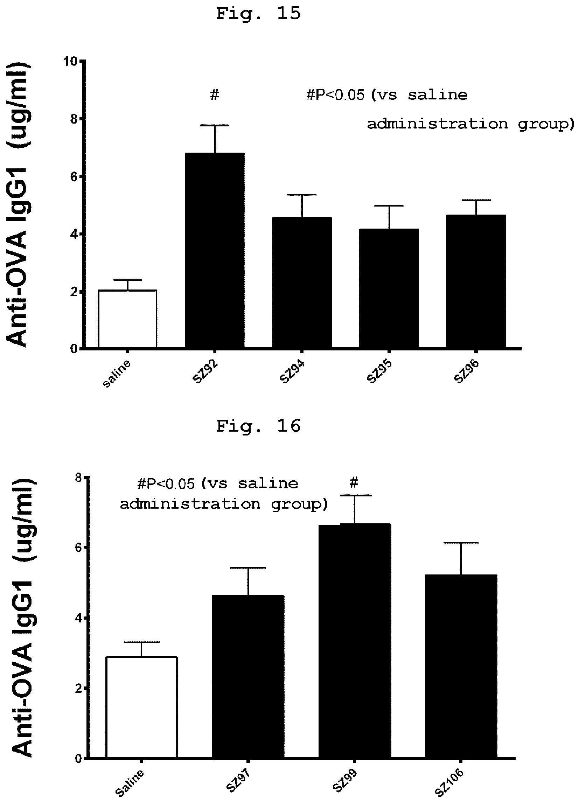

Fig. 15 is a graph showing the results of the evaluation test of adjuvant activity after primary immunization of N²-hexadecanoyl-N¹,N¹-diethyl-L-leucinamide (SZ92: compound wherein, in the formula (I), the group corresponding to R¹ is leucine side chain, the group corresponding to R² is C₁₅ alkyl group, the group corresponding to R³ is diethylamino), N-hexadecanoyl-L-histidine methyl ester (SZ94: compound wherein, in the formula (I), the group corresponding to R² is histidine side chain, the group corresponding to R² is C₁₅ alkyl group, the group corresponding to R³ is methoxy), N-hexadecanoyl-L-proline methyl ester (SZ95: compound wherein, in the formula (I), the group corresponding to R¹ is proline side chain, the group corresponding to R² is C₁₅ alkyl group, the group corresponding to R³ is methoxy), N-hexadecanoyl-L-serine methyl ester (SZ96: compound wherein, in the formula (I), the group corresponding to R¹ is serine side chain, the group corresponding to R² is C₁₅ alkyl group, the group corresponding to R³ is methoxy), in Example 14, wherein "saline" shows OVA single administration group, "SZ92" shows SZ92 administration group, "SZ94" shows SZ94 administration group, "SZ95" shows SZ95 administration group, "SZ96" shows SZ96 administration group.
Fig. 16 is a graph showing the results of the evaluation test of adjuvant activity after primary immunization of N-hexadecanoyl-L-leucine hexyl ester (SZ97: compound wherein, in the formula (I), the group corresponding to R¹ is leucine side chain, the group corresponding to R² is C₁₅ alkyl group, the group corresponding to R³ is hexyloxy), N²-hexadecanoyl-L-arginine hexyl ester hydrochloride (SZ99: salt of compound wherein, in the formula (I), the group corresponding to R¹ is arginine side chain, the group corresponding to R² is C₁₅ alkyl group, the group corresponding to R³ is hexyloxy), N²-docosanoyl-N¹,N¹-diethyl-L-glutamic acid 1-amide (SZ106: compound wherein, in the formula (I), the group corresponding to R¹ is glutamic acid side chain, the group corresponding to R² is C₂₁ alkyl group, the group corresponding to R³ is diethylamino), in Example 15, wherein "Saline" shows OVA single administration group, "SZ97" shows SZ97 administration group, "SZ99" shows SZ99 administration group, "SZ106" shows SZ106 administration group.

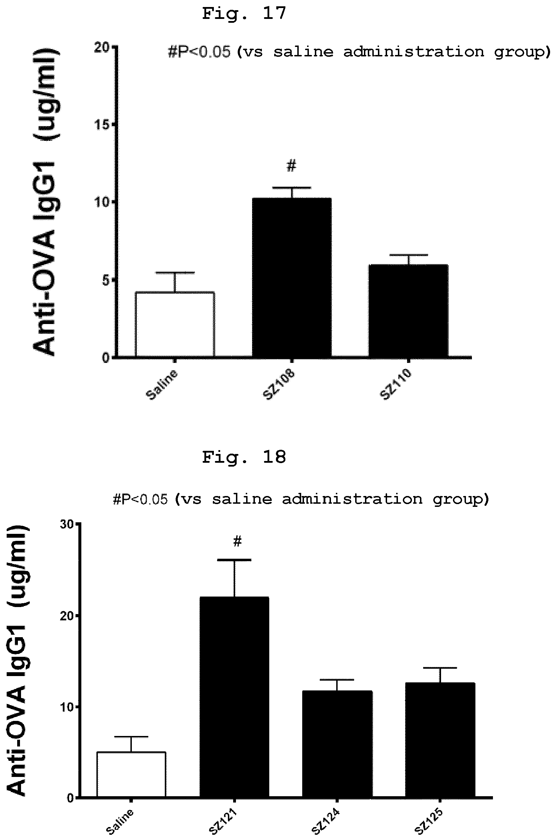

Fig. 17 is a graph showing the results of the evaluation test of adjuvant activity after primary immunization of N-docosanoylagmatine hydrochloride (SZ108: salt of compound wherein the group corresponding to R⁷ in the formula (II) is C₂₁ alkyl group), N²-hexadecanoyl-N¹,N¹-diethyl-L-arginine amide hydrochloride (SZ110: compound wherein, in the formula (I), the group corresponding to R¹ is arginine side chain, the group corresponding to R² is C₁₅ alkyl group, the group corresponding to R³ is diethylamino), in Example 16, wherein "Saline" shows OVA single administration group, "SZ108" shows SZ108 administration group, "SZ110" shows SZ110 administration group.
Fig. 18 is a graph showing the results of the evaluation test of adjuvant activity after primary immunization of N²-docosanoyl-L-arginine hexyl ester hydrochloride (SZ121: salt of compound wherein, in the formula (I), the group corresponding to R¹ is arginine side chain, the group corresponding to R² is C₂₁ alkyl group, the group corresponding to R³ is hexyloxy), N-docosanoyl-L-leucine hexyl ester (SZ124: compound wherein, in the formula (I), the group corresponding to R¹ is leucine side chain, the group corresponding to R² is C₂₁ alkyl group, the group corresponding to R³ is hexyloxy), N²-docosanoyl-N¹,N¹-diethyl-L-leucinamide (SZ125: compound wherein, in the formula (I), the group corresponding to R¹ is leucine side chain, the group corresponding to R² is C₂₁ alkyl group, the group corresponding to R³ is diethylamino), in Example 17, wherein "Saline" shows OVA single administration group, "SZ121" shows SZ121 administration group, "SZ124" shows SZ124 administration group, "SZ125" shows SZ125 administration group.

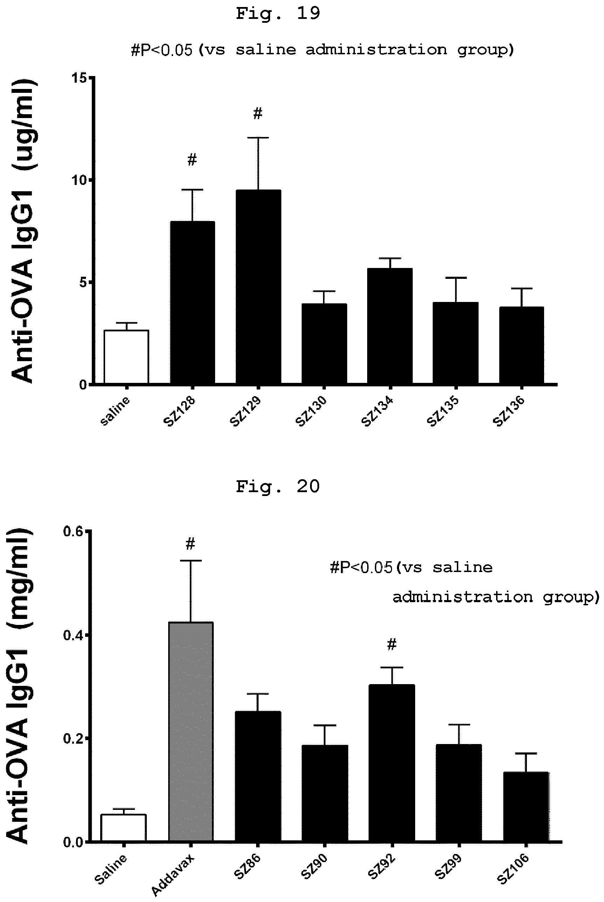

Fig. 19 is a graph showing the results of the evaluation test of adjuvant activity after primary immunization of N²-docosanoyl-L-arginine amide hydrochloride (SZ128: salt of compound wherein, in the formula (I), the group corresponding to R¹ is arginine side chain, the group corresponding to R² is C₂₁ alkyl group, the group corresponding to R³ is amino), N²-docosanoyl-N¹,N¹-diethyl-L-arginine amide hydrochloride (SZ129: salt of compound wherein, in the formula (I), the group corresponding to R¹ is arginine side chain, the group corresponding to R² is C₂₁ alkyl group, the group corresponding to R³ is diethylamino), N²-hexanoyl-L-arginine hexyl ester hydrochloride (SZ130: salt of compound wherein, in the formula (I), the group corresponding to R¹ is arginine side chain, the group corresponding to R² is C₅ alkyl group, the group corresponding to R³ is hexyloxy), N-hexadecanoyl-L-glutamic acid hexyl ester (SZ134: compound wherein, in the formula (I), the group corresponding to R¹ is glutamic acid side chain, the group corresponding to R² is C₁₅ alkyl group, the group corresponding to R³ is hexyloxy), N-docosanoyl-L-glutamic acid hexyl ester (SZ135: compound wherein, in the formula (I), the group corresponding to R¹ is glutamic acid side chain, the group corresponding to R² is C₂₁ alkyl group, the group corresponding to R³ is hexyloxy), N²-hexadecanoyl-L-glutamine methyl ester (SZ136: compound wherein, in the formula (I), the group corresponding to R¹ is glutamine side chain, the group corresponding to R² is C₁₅ alkyl group, the group corresponding to R³ is methoxy), in Example 18, wherein "saline" shows OVA single administration group, "SZ128" shows SZ128 administration group, "SZ129" shows SZ129 administration group, "SZ130" shows SZ130 administration group, "SZ134" shows SZ134 administration group, "SZ135" shows SZ135 administration group, "SZ136" shows SZ136 administration group.
Fig. 20 is a graph showing the results of the evaluation test of adjuvant activity after secondary immunization of N-(2-octadecyleicosanoyl)-L-leucine methyl ester (SZ86: compound wherein, in the formula (I), the group corresponding to R¹ is leucine side chain, the group corresponding to R² is C₃₇ alkyl group, the group corresponding to R³ is methoxy), N-hexadecanoyl-L-leucinamide (SZ90: compound wherein, in the formula (I), the group corresponding to R¹ is leucine side chain, the group corresponding to R² is C₁₅ alkyl group, the group corresponding to R³ is amino), N²-hexadecanoyl-N¹,N¹-diethyl-L-leucinamide (SZ92: compound wherein, in the formula (I), the group corresponding to R¹ is leucine side chain, the group corresponding to R² is C₁₅ alkyl group, the group corresponding to R³ is diethylamino), N²-hexadecanoyl-L-arginine hexyl ester hydrochloride (SZ99: salt of compound wherein, in the formula (I), the group corresponding to R¹ is arginine side chain, the group corresponding to R² is C₁₅ alkyl group, the group corresponding to R³ is hexyloxy), N²-docosanoyl-N¹,N¹-diethyl-L-glutamic acid 1-amide (SZ106: compound wherein, in the formula (I), the group corresponding to R¹ is glutamic acid side chain, the group corresponding to R² is C₂₁ alkyl group, the group corresponding to R³ is diethylamino), in Example 19, wherein "Saline" shows OVA single administration group, "Addavax" shows Addavax™ administration group, "SZ86" shows SZ86 administration group, "SZ90" shows SZ90 administration group, "SZ92" shows SZ92 administration group, "SZ99" shows SZ99 administration group, "SZ106" shows SZ106 administration group.

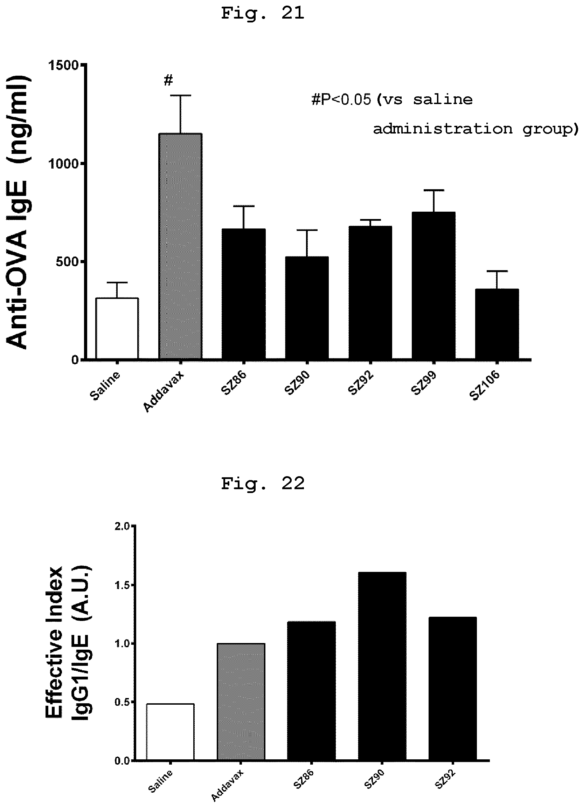

Fig. 21 is a graph showing the results of the evaluation test of allergy inducing activity after secondary immunization of N-(2-octadecyleicosanoyl)-L-leucine methyl ester (SZ86: compound wherein, in the formula (I), the group corresponding to R¹ is leucine side chain, the group corresponding to R² is C₃₇ alkyl group, the group corresponding to R³ is methoxy), N-hexadecanoyl-L-leucinamide (SZ90: compound wherein, in the formula (I), the group corresponding to R¹ leucine side chain, the group corresponding to R² is C₁₅ alkyl group, the group corresponding to R³ is amino), N²-hexadecanoyl-N¹,N¹-diethyl-L-leucinamide (SZ92: compound wherein, in the formula (I), the group corresponding to R¹ is leucine side chain, the group corresponding to R² is C₁₅ alkyl group, the group corresponding to R³ is diethylamino), N²-hexadecanoyl-L-arginine hexyl ester hydrochloride (SZ99: salt of compound wherein, in the formula (I), the group corresponding to R¹ is arginine side chain, the group corresponding to R² is C₁₅ alkyl group, the group corresponding to R³ is hexyloxy), N²-docosanoyl-N¹,N¹-diethyl-L-glutamic acid 1-amide (SZ106: compound wherein, in the formula (I), the group corresponding to R¹ is glutamic acid side chain, the group corresponding to R² is C₂₁ alkyl group, the group corresponding to R³ is diethylamino), in Example 19, wherein "Saline" shows OVA single administration group, "Addavax" shows Addavax™ administration group, "SZ86" shows SZ86 administration group, "SZ90" shows SZ90 administration group, "SZ92" shows SZ92 administration group, "SZ99" shows SZ99 administration group, "SZ106" shows SZ106 administration group.
Fig. 22 is a graph showing the relative values between adjuvant activity and allergy inducing activity after secondary immunization of N-(2-octadecyleicosanoyl)-L-leucine methyl ester (SZ86: compound wherein, in the formula (I), the group corresponding to R¹ is leucine side chain, the group corresponding to R² is C₃₇ alkyl group, the group corresponding to R³ is methoxy), N-hexadecanoyl-L-leucinamide (SZ90: compound wherein, in the formula (I), the group corresponding to R¹ is leucine side chain, the group corresponding to R² is C₁₅ alkyl group, the group corresponding to R³ is amino), N²-hexadecanoyl-N¹,N¹-diethyl-L-leucinamide (SZ92: compound wherein, in the formula (I), the group corresponding to R¹ leucine side chain, the group corresponding to R² is C₁₅ alkyl group, the group corresponding to R³ is diethylamino), in Example 19, wherein "Saline" shows OVA single administration group, "Addavax" shows Addavax™ administration group, "SZ86" shows SZ86 administration group, "SZ90" shows SZ90 administration group, "SZ92" shows SZ92 administration group.

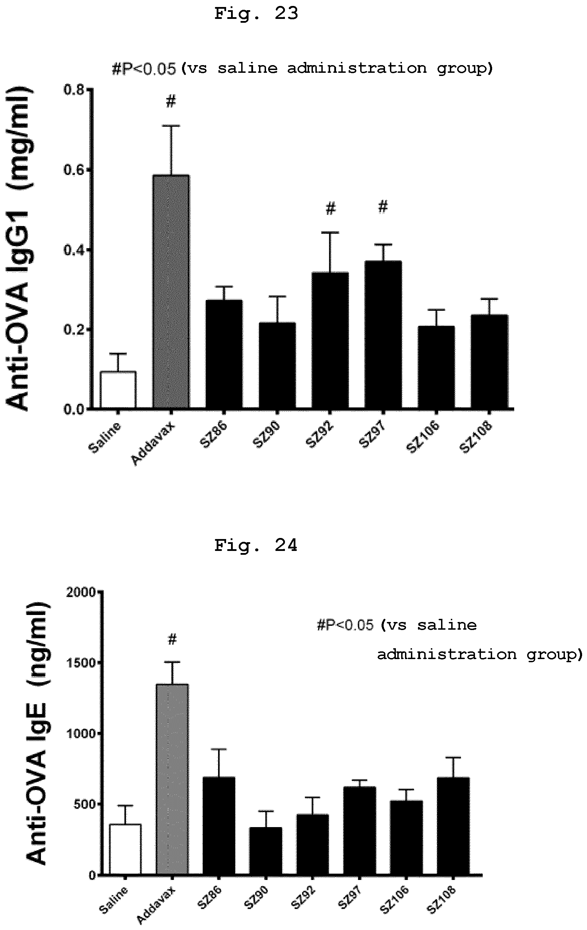

Fig. 23 is a graph showing the results of the evaluation test of adjuvant activity after secondary immunization of N-(2-octadecyleicosanoyl)-L-leucine methyl ester (SZ86: compound wherein, in the formula (I), the group corresponding to R¹ is leucine side chain, the group corresponding to R² is C₃₇ alkyl group, the group corresponding to R³ is methoxy), N-hexadecanoyl-L-leucinamide (SZ90: compound wherein, in the formula (I), the group corresponding to R¹ is leucine side chain, the group corresponding to R² is C₁₅ alkyl group, the group corresponding to R³ is amino), N²-hexadecanoyl-N¹,N¹-diethyl-L-leucinamide (SZ92: compound wherein, in the formula (I), the group corresponding to R¹ is leucine side chain, the group corresponding to R² is C₁₅ alkyl group, the group corresponding to R³ is diethylamino), N-hexadecanoyl-L-leucine hexyl ester (SZ97: compound wherein, in the formula (I), the group corresponding to R¹ is leucine side chain, the group corresponding to R² is C₁₅ alkyl group, the group corresponding to R³ is hexyloxy), N²-docosanoyl-N¹,N¹-diethyl-L-glutamic acid 1-amide (SZ106: compound wherein, in the formula (I), the group corresponding to R¹ is glutamic acid side chain, the group corresponding to R² is C₂₁ alkyl group, the group corresponding to R³ is diethylamino), N-docosanoylagmatine hydrochloride (SZ108: salt of compound wherein the group corresponding to R⁷ in the formula (II) is C₂₁ alkyl group), in Example 20, wherein "Saline" shows OVA single administration group, "Addavax" shows Addavax™ administration group, "SZ86" shows SZ86 administration group, "SZ90" shows SZ90 administration group, "SZ92" shows SZ92 administration group, "SZ97" shows SZ97 administration group, "SZ106" shows SZ106 administration group, "SZ108" shows SZ108 administration group.
Fig. 24 is a graph showing the results of the evaluation test of allergy inducing activity after secondary immunization of N-(2-octadecyleicosanoyl)-L-leucine methyl ester (SZ86: compound wherein, in the formula (I), the group corresponding to R¹ is leucine side chain, the group corresponding to R² is C₃₇ alkyl group, the group corresponding to R³ is methoxy), N-hexadecanoyl-L-leucinamide (SZ90: compound wherein, in the formula (I), the group corresponding to R¹ is leucine side chain, the group corresponding to R² is C₁₅ alkyl group, the group corresponding to R³ is amino), N²-hexadecanoyl-N¹,N¹-diethyl-L-leucinamide (SZ92: compound wherein, in the formula (I), the group corresponding to R¹ is leucine side chain, the group corresponding to R² is C₁₅ alkyl group, the group corresponding to R³ is diethylamino), N-hexadecanoyl-L-leucine hexyl ester (SZ97: compound wherein, in the formula (I), the group corresponding to R¹ is leucine side chain, the group corresponding to R² is C₁₅ alkyl group, the group corresponding to R³ is hexyloxy), N²-docosanoyl-N¹,N¹-diethyl-L-glutamic acid 1-amide (SZ106: compound wherein, in the formula (I), the group corresponding to R¹ is glutamic acid side chain, the group corresponding to R² is C₂₁ alkyl group, the group corresponding to R³ is diethylamino), N-docosanoylagmatine hydrochloride (SZ108: salt of compound wherein the group corresponding to R⁷ in the formula (II) is C₂₁ alkyl group), in Example 20, wherein "Saline" shows OVA single administration group, "Addavax" shows Addavax™ administration group, "SZ86" shows SZ86 administration group, "SZ90" shows SZ90 administration group, "SZ92" shows SZ92 administration group, "SZ97" shows SZ97 administration group, "SZ106" shows SZ106 administration group, "SZ108" shows SZ108 administration group.

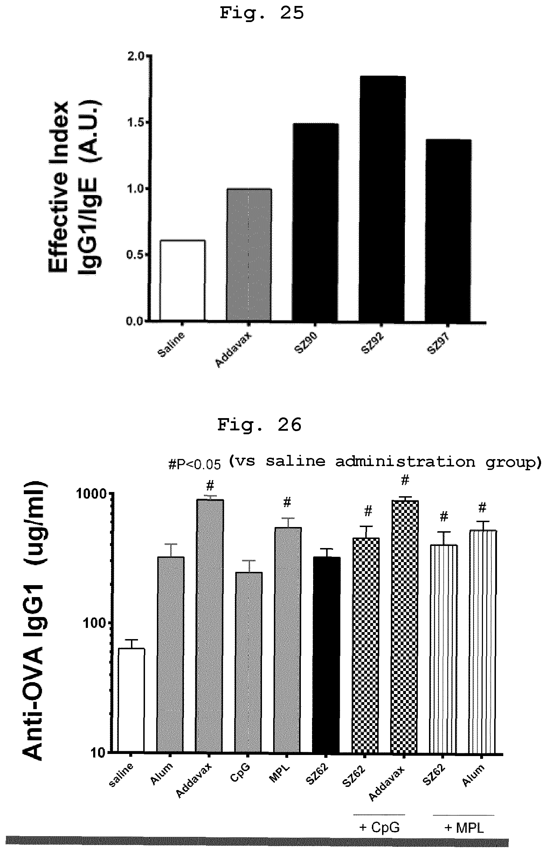

Fig. 25 is a graph showing the relative values between adjuvant activity and allergy inducing activity after secondary immunization of N-hexadecanoyl-L-leucinamide (SZ90: compound wherein, in the formula (I), the group corresponding to R¹ is leucine side chain, the group corresponding to R² is C₁₅ alkyl group, the group corresponding to R³ is amino), N²-hexadecanoyl-N¹,N¹-diethyl-L-leucinamide (SZ92: compound wherein, in the formula (I), the group corresponding to R¹ is leucine side chain, the group corresponding to R² is C₁₅ alkyl group, the group corresponding to R³ is diethylamino), N-hexadecanoyl-L-leucine hexyl ester (SZ97: compound wherein, in the formula (I), the group corresponding to R¹ is leucine side chain, the group corresponding to R² is C₁₅ alkyl group, the group corresponding to R³ is hexyloxy), in Example 20, wherein "Saline" shows OVA single administration group, "Addavax" shows Addavax™ administration group, "SZ90" shows SZ90 administration group, "SZ92" shows SZ92 administration group, "SZ97" shows SZ97 administration group.
Fig. 26 is a graph showing the adjuvant activity (anti-OVA-specific IgG1 subclass antibody) after secondary immunization of N-hexadecanoylagmatine (SZ62: compound wherein the group corresponding to R⁷ in the formula (II) is C₁₅ alkyl group), in Example 21, wherein "saline" shows OVA single administration group, "Alum" shows Alum administration group, "Addavax" shows Addavax™ administration group, "CpG" shows CpG-ODN™ administration group, "MPL" shows MPL administration group, "SZ62" shows SZ62 administration group, "SZ62+CpG" shows SZ62 and CpG administration group, "Addavax+CpG" shows Addavax™ and CpG administration group, "SZ62+MPL" shows SZ62 and MPL administration group, "Alum+MPL" shows ALUM and MPL administration group.

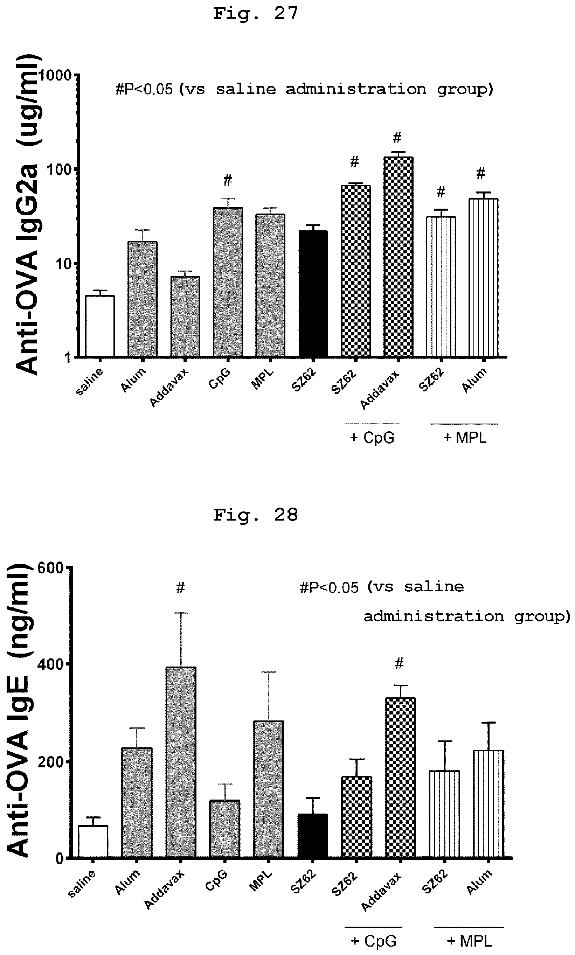

Fig. 27 is a graph showing the adjuvant activity (anti-OVA-specific IgG2a subclass antibody) after secondary immunization of N-hexadecanoylagmatine (SZ62: compound wherein the group corresponding to R⁷ in the formula (II) is C₁₅ alkyl group), in Example 21, wherein "saline" shows OVA single administration group, "Alum" shows Alum administration group, "Addavax" shows Addavax™ administration group, "CpG" shows CpG-ODN™ administration group, "MPL" shows MPL administration group, "SZ62" shows SZ62 administration group, "SZ62+CpG" shows SZ62 and CpG administration group, "Addavax+CpG" shows Addavax™ and CpG administration group, "SZ62+MPL" shows SZ62 and MPL administration group, "Alum+MPL" shows ALUM and MPL administration group.
Fig. 28 is a graph showing the results of the evaluation test of allergy inducing activity after secondary immunization of N-hexadecanoylagmatine (SZ62: compound wherein the group corresponding to R⁷ in the formula (II) is C₁₅ alkyl group), in Example 21, wherein "saline" shows OVA single administration group, "Alum" shows Alum administration group, "Addavax" shows Addavax™ administration group, "CpG" shows CpG-ODN™ administration group, "MPL" shows MPL administration group, "SZ62" shows SZ62 administration group, "SZ62+CpG" shows SZ62 and CpG administration group, "Addavax+CpG" shows Addavax™ and CpG administration group, "SZ62+MPL" shows SZ62 and MPL administration group, "Alum+MPL" shows ALUM and MPL administration group.

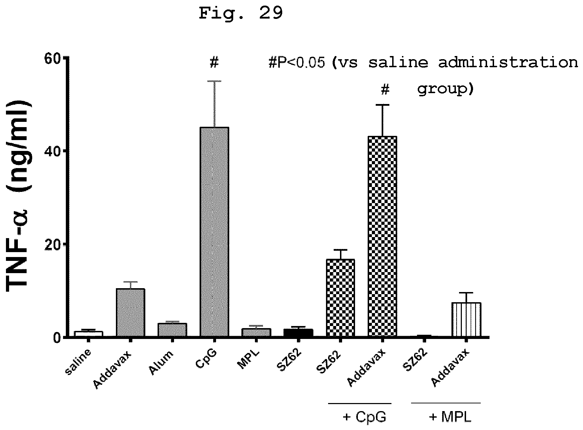

Fig. 29 is a graph showing the results of the evaluation test of systemic inflammation after primary immunization of N-hexadecanoylagmatine (SZ62: compound wherein the group corresponding to R⁷ in the formula (II) is C₁₅ alkyl group), in Example 22, wherein "saline" shows OVA single administration group, "Alum" shows Alum administration group, "Addavax" shows Addavax™ administration group, "CpG" shows CpG-ODN™ administration group, "MPL" shows MPL administration group, "SZ62" shows SZ62 administration group, "SZ62+CpG" shows SZ62 and CpG administration group, "Addavax+CpG" shows Addavax™ and CpG administration group, "SZ62+MPL" shows SZ62 and MPL administration group, "Addavax+MPL" shows Addavax™ and MPL administration group.

### Description of Embodiments

The immunostimulating agent of the present invention comprises at least one kind of a compound represented by the following formula (I) or a salt thereof, or at least one kind of a compound represented by the following formula (II) or a salt thereof. 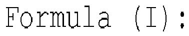 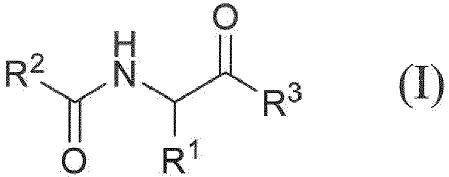 wherein
R¹ is an amino acid side chain (excluding a cystine side chain);
R² is a C₁₋₃₇ alkyl group; and
R³ is a hydroxyl group, a C₁₋₆ alkoxy group or -NR⁴R⁵ wherein R⁴ and R⁵ are the same or different and each is a hydrogen atom or a C₁₋₆ alkyl group. 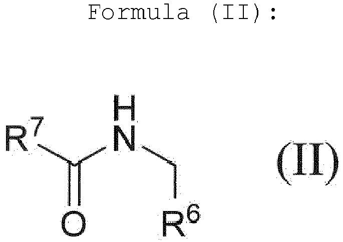
wherein
R⁶ is an arginine side chain; and
R⁷ is a C₁₋₃₇ alkyl group.

Each group in the formula (I) and the formula (II) is explained below.

R¹ in the formula (I) is an amino acid side chain (excluding a cystine side chain).

The "amino acid side chain" for R¹ is an R moiety of α-amino acid represented by R-CH(NH₂)-COOH. Examples thereof include an alanine side chain (methyl), an arginine side chain (3-guanidylpropyl), an asparagine side chain (carbamoylmethyl), an aspartic acid side chain (carboxymethyl), a cysteine side chain (sulfhydrylmethyl), a glutamine side chain (2-carbamoylethyl), a glutamic acid side chain (2-carboxyethyl), a hydrogen atom (glycine side chain), a histidine side chain (1H-imidazol-4-ylmethyl), an isoleucine side chain (sec-butyl), a leucine side chain (isobutyl), a lysine side chain (4-aminobutyl), a methionine side chain (2-(methylthio)ethyl), a phenylalanine side chain (benzyl), a serine side chain (hydroxymethyl), a threonine side chain (1-hydroxyethyl), a tryptophan side chain (3-indolylmethyl), a tyrosine side chain (4-hydroxybenzyl), a valine side chain (isopropyl) and the like. The "amino acid side chain" for R¹ includes a proline side chain (propyl bonded to N in the formula (I) to form a 5-membered ring). Of these, an arginine side chain, a glutamine side chain, a glutamic acid side chain, a hydrogen atom, an isoleucine side chain, a leucine side chain, a lysine side chain, a phenylalanine side chain, and a valine side chain are preferable, an arginine side chain, a glutamine side chain, an isoleucine side chain, a leucine side chain, a phenylalanine side chain, and a valine side chain are more preferable; an arginine side chain, a glutamine side chain, and a leucine side chain are more preferable, an arginine side chain, and a glutamine side chain are particularly preferable, and an arginine side chain is most preferable, since they are superior in an immunostimulatory effect and show a lower allergy inducing activity. An arginine side chain, a glutamine side chain, a glutamic acid side chain, a histidine side chain, a leucine side chain, a serine side chain, a proline side chain are preferable. A histidine side chain, a serine side chain, a proline side chain are preferable.

The "amino acid side chain" for R¹ preferablyexcludes a cystine side chain and a cysteine side chain.

R² in the formula (I) is a C₁₋₃₇ alkyl group.

The "C₁₋₃₇ alkyl group" for R² is a straight chain or branched chain alkyl group having 1 - 37 carbon atoms and includes, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, dodecyl, tridecyl, tetradecyl, pentadecyl, hexadecyl, heptadecyl, 2-heptyldecyl, 4,6,6-trimethyl-1-(1,3,3-trimethylbutyl)heptyl, octadecyl, nonadecyl, icosyl, eicosyl, henicosyl, heneicosyl, docosyl, tricosyl, tetracosyl, pentacosyl, hexacosyl, heptacosyl, octacosyl, nonacosyl, 1-tetradecylpentadecyl, triacontyl, hentriacontyl, dotriacontyl, tritriacontyl, 1-hexadecylheptadecyl, tetratriacontyl, pentatriacontyl, hexatriacontyl, heptatriacontyl, 1-octadecylnonadecyl and the like. Of these, a C₁₂₋₂₄ alkyl group is preferable, a C₁₅₋₂₁ alkyl group is more preferable, a straight chain C₁₅₋₂₁ alkyl group is particularly preferable, and pentadecyl, heptadecyl, henicosyl are most preferable.

R³ in the formula (I) is a hydroxyl group, a C₁₋₆ alkoxy group or -NR⁴R⁵ wherein R⁴ and R⁵ are the same or different and each is a hydrogen atom or a C₁₋₆ alkyl group.

The "C₁₋₆ alkoxy group" for R³ is a straight chain or branched chain alkoxy group having 1 - 6 carbon atoms and, for example, methoxy, ethoxy, propoxy, isopropyloxy, butoxy, isobutyloxy, sec-butyloxy, tert-butyloxy, pentyloxy, isopentyloxy, neopentyloxy, hexyloxy and the like can be mentioned. Of these, a C₁₋₄ alkoxy group is preferable, and methoxy is more preferable, from the aspects of easy availability and the like of amino acid alkyl ester as a starting material.

The "C₁₋₆ alkyl group" for R⁴ or R⁵ is a straight chain or branched chain alkyl group having 1 - 6 carbon atoms, and examples thereof include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like. Among these, a C₁₋₄ alkyl group is preferable, tert-butyl, ethyl, methyl is more preferable, and ethyl, methyl is particularly preferable, from the aspect of easy availability and low cost.

R⁴ and R⁵ are preferably the same or different and each is a hydrogen atom or a C₁₋₄ alkyl group, and more preferably, they are the same or different and each is a hydrogen atom or a methyl group.

While R⁴ and R⁵ may be the same or different, they are preferably the same.

R⁴ and R⁵ are preferably the same or different and each is a hydrogen atom or a C₁₋₄ alkyl group, and more preferably, they are the same or different and each is a hydrogen atom or an ethyl group.

R³ is preferably a hydroxyl group or a C₁₋₆ alkoxy group, more preferably a hydroxyl group or a C₁₋₄ alkoxy group, particularly preferably a hydroxyl group or methoxy, most preferably methoxy.

R³ is preferably a C₁₋₆ alkoxy group or -NR⁴R⁵ wherein R⁴ and R⁵ are the same or different and each is a hydrogen atom or a C₁₋₆ alkyl group, more preferably a C₁₋₆ alkoxy group or -NR⁴R⁵ wherein R⁴ and R⁵ are the same or different and each is a hydrogen atom or a C₁₋₄ alkyl group, particularly preferably a C₁₋₄ alkoxy group or -NR⁴R⁵ wherein R⁴ and R⁵ are the same or different and each is a hydrogen atom or ethyl, most preferably methoxy, amino, diethylamino.

R⁶ in the formula (II) is an arginine side chain.

R⁷ in the formula (II) is a C₁₋₃₇ alkyl group.

The "C₁₋₃₇ alkyl group" for R⁷ is a straight chain or branched chain alkyl group having 1 - 37 carbon atoms and, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl, undecyl, dodecyl, tridecyl, tetradecyl, pentadecyl, hexadecyl, heptadecyl, 2-heptyldecyl, 4,6,6-trimethyl-1-(1,3,3-trimethylbutyl)heptyl, octadecyl, nonadecyl, icosyl, eicosyl, henicosyl, heneicosyl, docosyl, tricosyl, tetracosyl, pentacosyl, hexacosyl, heptacosyl, octacosyl, nonacosyl, 1-tetradecylpentadecyl, triacontyl, hentriacontyl, dotriacontyl, tritriacontyl, 1-hexadecylheptadecyl, tetratriacontyl, pentatriacontyl, hexatriacontyl, heptatriacontyl, 1-octadecylnonadecyl and the like can be mentioned. Of these, a C₁₂₋₂₄ alkyl group is preferable, a C₁₅₋₂₁ alkyl group is more preferable, a straight chain C₁₅₋₂₁ alkyl group is particularly preferable, and pentadecyl is most preferable.

Preferable compound (I) is compound (I), wherein
R¹ is an arginine side chain, a glutamine side chain, a glutamic acid side chain, a hydrogen atom, an isoleucine side chain, a leucine side chain, a lysine side chain, a phenylalanine side chain or a valine side chain,
R² is a C₁₂₋₂₄ alkyl group, and
R³ is a hydroxyl group or a C₁₋₆ alkoxy group.

More preferable compound (I) is compound (I), wherein
R¹ is an arginine side chain, a glutamine side chain, an isoleucine side chain, a leucine side chain, a phenylalanine side chain or a valine side chain,
R² is a C₁₂₋₂₄ alkyl group, and
R³ is a hydroxyl group or a C₁₋₆ alkoxy group.

Particularly preferable compound (I) is compound (I), wherein
R¹ is an arginine side chain, a glutamine side chain or a leucine side chain,
R² is a C₁₂₋₂₄ alkyl group, and
R³ is a hydroxyl group or a C₁₋₆ alkoxy group.

Most preferable compound (I) is compound (I), wherein
R¹ is an arginine side chain,
R² is a C₁₂₋₂₄ alkyl group, and
R³ is a hydroxyl group or a C₁₋₆ alkoxy group.

Other preferable compound (I) is compound (I), wherein
R¹ is an arginine side chain, a glutamine side chain, a glutamic acid side chain, a histidine side chain, a leucine side chain, a serine side chain or a proline side chain,
R² is a C₁₋₃₇ alkyl group, and
R³ is a hydroxyl group, a C₁₋₆ alkoxy group or -NR⁴R⁵ wherein R⁴ and R⁵ are the same or different and each is a hydrogen atom or a C₁₋₆ alkyl group.

Other more preferable compound (I) is compound (I), wherein
R¹ is an arginine side chain, a glutamine side chain, a glutamic acid side chain, a histidine side chain, a leucine side chain, a serine side chain or a proline side chain,
R² is a C₁₋₃₇ alkyl group, and
R³ is a hydroxyl group, a C₁₋₆ alkoxy group or -NR⁴R⁵ wherein R⁴ and R⁵ are the same or different and each is a hydrogen atom or a C₁₋₄ alkyl group.

Other particularly preferable compound (I) is compound (I), wherein
R¹ is an arginine side chain, a glutamine side chain, a glutamic acid side chain, a histidine side chain, a leucine side chain, a serine side chain or a proline side chain,
R² is a C₁₂₋₂₄ alkyl group, and
R³ is a hydroxyl group, a C₁₋₆ alkoxy group or -NR⁴R⁵ wherein R⁴ and R⁵ are the same or different and each is a hydrogen atom or a C₁₋₆ alkyl group.

Other most preferable compound (I) is compound (I), wherein
R¹ is an arginine side chain, a glutamine side chain, a glutamic acid side chain, a histidine side chain, a leucine side chain, a serine side chain or a proline side chain,
R² is a C₁₂₋₂₄ alkyl group, and
R³ is a hydroxyl group, a C₁₋₆ alkoxy group or -NR⁴R⁵ wherein R⁴ and R⁵ are the same or different and each is a hydrogen atom or ethyl.

A salt of compound (I) or compound (II) is not particularly limited as long as it is pharmacologically acceptable. Examples thereof include metal salt, ammonium salt, salts with organic bases, salts with inorganic acids, salts with organic acids and the like. Preferable examples of the metal salt include alkali metal salts such as sodium salt, potassium salt and the like; alkaline earth metal salts such as calcium salt, barium salt and the like; magnesium salt, aluminum salt and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine or the like. Preferable examples of the salt with inorganic acid include salts with hydrochloric acid, hydrobromic acid, nitric acid, sulfuric acid, phosphoric acid or the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid or the like. Of these, salt with hydrochloric acid, salt with acetic acid, and sodium salt are preferable from the aspect of practicability of pharmaceutical product.

Specific examples of preferable compound (I) or a salt thereof include
N²-hexadecanoyl-L-arginine,
N²-hexadecanoyl-L-arginine methyl ester,
N²-octadecanoyl-L-glutamine tert-butyl ester,
N²-octadecanoyl-L-glutamine,
N-docosanoyl glycine methyl ester,
N-docosanoyl-L-leucine methyl ester,
N-docosanoyl-L-phenylalanine methyl ester,
N-docosanoyl-L-glutamic acid 1-methyl ester,
N²-docosanoyl-L-lysine methyl ester,
N-docosanoyl-L-isoleucine methyl ester,
N-docosanoyl-L-valine methyl ester,
N-hexadecanoyl glycine methyl ester,
N-hexadecanoyl-L-leucine methyl ester,
N-hexadecanoyl-L-phenylalanine methyl ester,
N²-hexadecanoyl-L-lysine methyl ester,
N-hexadecanoyl-L-glutamic acid 1-methyl ester,
and a salt thereof and the like.

Of these,
N²-hexadecanoyl-L-arginine,
N²-hexadecanoyl-L-arginine methyl ester salt (preferably, hydrochloride),
N²-octadecanoyl-L-glutamine tert-butyl ester,
N²-octadecanoyl-L-glutamine,
N-docosanoyl-L-leucine methyl ester,
N-docosanoyl-L-phenylalanine methyl ester,
N-docosanoyl-L-isoleucine methyl ester,
N-docosanoyl-L-valine methyl ester,
N-hexadecanoyl-L-leucine methyl ester, and
N-hexadecanoyl-L-phenylalanine methyl ester are preferable,
N²₋hexadecanoyl-L-arginine,
N²-hexadecanoyl-L-arginine methyl ester salt (preferably, hydrochloride),
N²-octadecanoyl-L-glutamine tert-butyl ester,
N²-octadecanoyl-L-glutamine,
N-docosanoyl-L-leucine methyl ester, and
N-hexadecanoyl-L-leucine methyl ester are more preferable, and
N²₋hexadecanoyl-L-arginine, and
N²-hexadecanoyl-L-arginine methyl ester salt (preferably, hydrochloride)
are particularly preferable, since they are superior in an immunostimulatory effect and have a lower allergy inducing activity.

Other specific examples of preferable compound (I) or a salt thereof include
N-acetyl-L-leucine methyl ester,
N-hexanoyl-L-leucine methyl ester,
N-(2-octadecyleicosanoyl)-L-leucine methyl ester,
N-hexadecanoyl-L-leucine tert-butyl ester,
N-hexadecanoyl-L-leucinamide,
N²-hexadecanoyl-N¹,N¹-diethyl-L-leucinamide,
N-hexadecanoyl-L-histidine methyl ester,
N-hexadecanoyl-L-proline methyl ester,
N-hexadecanoyl-L-serine methyl ester,
N-hexadecanoyl-L-leucine hexyl ester,
N²-hexadecanoyl-L-arginine hexyl ester,
N²-docosanoyl-N¹,N¹-diethyl-L-glutamic acid 1-amide,
N²-hexadecanoyl-N¹,N¹-diethyl-L-arginine amide,
N²-docosanoyl-L-arginine hexyl ester,
N-docosanoyl-L-leucine hexyl ester,
N²-docosanoyl-N¹,N¹-diethyl-L-leucinamide,
N²-docosanoyl-L-arginine amide,
N²-docosanoyl-N¹,N¹-diethyl-L-arginine amide,
N²-hexanoyl-L-arginine hexyl ester,
N-hexadecanoyl-L-glutamic acid hexyl ester,
N-docosanoyl-L-glutamic acid hexyl ester,
N²-hexadecanoyl-L-glutamine methyl ester,
and a salt thereof and the like.

Of these,
N-acetyl-L-leucine methyl ester,
N-hexanoyl-L-leucine methyl ester,
N-(2-octadecyleicosanoyl)-L-leucine methyl ester,
N-hexadecanoyl-L-leucine tert-butyl ester,
N-hexadecanoyl-L-leucinamide,
N²-hexadecanoyl-N¹,N¹-diethyl-L-leucinamide,
N-hexadecanoyl-L-histidine methyl ester,
N-hexadecanoyl-L-proline methyl ester,
N-hexadecanoyl-L-serine methyl ester,
N-hexadecanoyl-L-leucine hexyl ester,
N²-hexadecanoyl-L-arginine hexyl ester salt (preferably, hydrochloride),
N²-docosanoyl-N¹,N¹-diethyl-L-glutamic acid 1-amide,
N²-hexadecanoyl-N¹,N¹-diethyl-L-arginine amide salt (preferably, hydrochloride),
N²-docosanoyl-L-arginine hexyl ester salt (preferably, hydrochloride),
N-docosanoyl-L-leucine hexyl ester,
N²-docosanoyl-N¹,N¹-diethyl-L-leucinamide,
N²-docosanoyl-L-arginine amide salt (preferably, hydrochloride),
N²-docosanoyl-N¹,N¹-diethyl-L-arginine amide salt (preferably, hydrochloride),
N²-hexanoyl-L-arginine hexyl ester salt (preferably, hydrochloride),
N-hexadecanoyl-L-glutamic acid hexyl ester,
N-docosanoyl-L-glutamic acid hexyl ester,
N²-hexadecanoyl-L-glutamine methyl ester,
are preferable,
N-hexadecanoyl-L-leucine tert-butyl ester,
N-hexadecanoyl-L-leucinamide,
N²-hexadecanoyl-N¹,N¹-diethyl-L-leucinamide,
N-hexadecanoyl-L-histidine methyl ester,
N-hexadecanoyl-L-proline methyl ester,
N-hexadecanoyl-L-serine methyl ester,
N-hexadecanoyl-L-leucine hexyl ester,
N²-hexadecanoyl-L-arginine hexyl ester salt (preferably, hydrochloride),
N²-docosanoyl-N¹,N¹-diethyl-L-glutamic acid 1-amide,
N²-hexadecanoyl-N¹,N¹-diethyl-L-arginine amide salt (preferably, hydrochloride),
N²-docosanoyl-L-arginine hexyl ester salt (preferably, hydrochloride),
N-docosanoyl-L-leucine hexyl ester,
N²-docosanoyl-N¹,N¹-diethyl-L-leucinamide,
N²-docosanoyl-L-arginine amide salt (preferably, hydrochloride),
N²-docosanoyl-N¹,N¹-diethyl-L-arginine amide salt (preferably, hydrochloride),
are more preferable, since they are superior in an immunostimulatory effect and have a lower allergy inducing activity.

Specific examples of preferable compound (II) or a salt thereof include N-hexadecanoylagmatine, a salt thereof and the like.

Other specific examples of preferable compound (II) or a salt thereof include N-docosanoylagmatine, a salt (preferably, hydrochloride) thereof and the like.

### (Synthesis of compound (I), compound (II) and a salt thereof)

While the production method of compound (I), compound (II) and a salt thereof (hereinafter sometimes to be generically referred to as the compound of the present invention) is not particularly limited, they can be produced by a known method or an appropriate combination of a method analogous thereto.

For example, compound (I) can be produced by reacting amino acid or amino acid alkyl ester, wherein a side chain is protected as necessary, with R²-COCl wherein R² is as defined above in the presence of a base, or reacting amino acid alkyl ester with R²-COOH wherein R² is as defined above in the presence of a condensing agent (or sometimes in the presence of a base), which is followed by deprotection of the protecting group of the side chain or esterification as necessary; and compound (II) can be produced by reacting agmatine, wherein a side chain is protected as necessary, with R⁷-COCl wherein R⁷ are as defined above in the presence of a base, or with R⁷-COOH wherein R⁷ are as defined above in the presence of a condensing agent (or sometimes in the presence of a base), which is followed by deprotection of the protecting group of the side chain as necessary.

The amount of R²-COCl to be used is generally 1 - 5 equivalents, preferably 1 - 2 equivalents, relative to 1 equivalent of the amino acid or amino acid alkyl ester.

The amount of R²-COOH to be used is generally 1 - 5 equivalents, preferably 1 - 2 equivalents, relative to 1 equivalent of the amino acid alkyl ester.

The amount of R⁷-COCl to be used is generally 1 - 5 equivalents, preferably 1 - 2 equivalents, relative to 1 equivalent of the agmatine.

The amount of R⁷-COOH to be used is generally 1 - 5 equivalents, preferably 1 - 2 equivalents, relative to 1 equivalent of the agmatine.

Examples of the condensing agent include carbodiimides such as 1,3-dicyclohexylcarbodiimide, 1-cyclohexyl-3-morpholinoethylcarbodiimide, 1-cyclohexyl-3-(4-diethylaminocyclohexyl)carbodiimide, 1,3-diethylcarbodiimide, 1,3-diisopropylcarbodiimide, 1-ethyl-3-(3-dimethylaminopropyl)carbodiimide and the like or a salt thereof and the like.

The amount of the condensing agent to be used is generally 1 - 5 equivalents, preferably 1 - 2 equivalents, relative to 1 equivalent of the amino acid alkyl ester or agmatine.

Examples of the base include alkali metal hydroxides (e.g., lithium hydroxide, sodium hydroxide, potassium hydroxide etc.), alkaline earth metal hydroxides (e.g., magnesium hydroxide, calcium hydroxide etc.), alkali metal carbonates (e.g., sodium carbonate, potassium carbonate etc.), alkali metal hydrogen carbonates (e.g., sodium hydrogen carbonate, potassium hydrogen carbonate etc.), organic bases (e.g., trimethylamine, triethylamine, diisopropylethylamine, pyridine, 4-dimethylaminopyridine, picoline, N-methylpyrrolidine, N-methylmorpholine, N,N-dimethylaniline, 1,5-diazabicyclo[4.3.0]-5-nonene, 1,4-diazabicyclo[2.2.2]octane, 1,8-diazabicyclo[5.4.0]-7-undecene, tetramethylguanidine etc.), organic lithiums (e.g., methyllithium, n-butyllithium, sec-butyllithium, tert-butyllithium etc.), lithium amides (e.g., lithium diisopropylamide etc.) and the like.

The amount of the base to be used is generally 1 - 10 equivalents, preferably 1 - 4 equivalents, relative to 1 equivalent of the amino acid or amino acid alkyl ester or agmatine.

Of the above-mentioned reactions, the reaction using R²-COOH or R⁷-COOH may be performed, when desired, in the presence of a condensation promoter.

Examples of the condensation promoter include 1-hydroxybenzotriazole (HOBt), a hydrate thereof and the like.

The amount of the condensation promoter to be used is generally 0.01 - 10 equivalents, preferably 1 - 2 equivalents, relative to 1 equivalent of the amino acid alkyl ester or agmatine.

In the above-mentioned reactions, a mixed acid anhydride of R²-COOH or a mixed acid anhydride of R⁷-COOH may also be used instead of R²-COCl or R⁷-COCl. The mixed acid anhydride can be obtained, for example, by reacting R²-COOH or R⁷-COOH with alkyl chlorocarbonate (e.g., methyl chlorocarbonate, ethyl chlorocarbonate, isobutyl chlorocarbonate) and the like in the presence of a base.

The above-mentioned reaction is preferably performed in a solvent inert to the reaction. While such solvent is not particularly limited as long as the reaction proceeds, examples thereof include ethers (e.g., 1,4-dioxane, tetrahydrofuran, diethyl ether, tert-butyl methyl ether, diisopropyl ether, ethylene glycol dimethylether), esters (e.g., ethyl formate, ethyl acetate, butyl acetate), halogenated hydrocarbons (e.g., dichloromethane, chloroform, carbon tetrachloride, trichloroethylene), hydrocarbons (e.g., hexane, benzene, toluene), amides (e.g., N,N-dimethylformamide, N,N-dimethylacetamide), sulfoxides (e.g., dimethyl sulfoxide) and the like. Two or more kinds of these solvents may be mixed and used at an appropriate ratio. As a solvent for a reaction using R²-COCl wherein R² is as defined above or R-COCl wherein R⁷ is as defined above, from the above-mentioned reactions, an alcohol solvent (isopropyl alcohol and the like) can also be mentioned.

The reaction temperature is generally -80 - 150°C, preferably 10 - 100°C.

The reaction time generally 0.5 - for 48 hr, preferably 10 - for 30 hr.

A production method of compound (I) wherein R³ is -NR⁴R⁵ includes a method comprising hydrolyzing an ester moiety of an acylamino acid alkyl ester form wherein a side chain is protected as necessary, which is obtained by a method similar to the above-mentioned method, converting same to an amide form with NHR⁴R⁵ wherein R⁴ and R⁵ are as defined above, and eliminating the protecting group of the side chain as necessary, and a method comprising converting an amino acid wherein a side chain is protected as necessary to an amide form with NHR⁴R⁵, acylating same by the above-mentioned method, and eliminating the protecting group of the side chain as necessary. As the production method, a method comprising converting an amino acid wherein a side chain is protected as necessary and an amino group is protected to an amide form with NHR⁴R⁵, eliminating the amino-protecting group, acylating the form by the above-mentioned method, and eliminating the protecting group of the side chain as necessary can also be mentioned.

When the compound of the present invention produced by the above-mentioned method is a free form, it can be converted to a salt with, for example, inorganic acid, organic acid, inorganic base, organic base or the like according to a conventional method; when the compound of the present invention is a salt form, it can also be converted to a free form or other salt according to a conventional method.

The compound of the present invention produced by a method such as the above can be isolated and purified by, for example, general separation means such as column chromatography, recrystallization, solvent washing and the like.

When the compound of the present invention contains an optical isomer, a stereoisomer, a positional isomer or a rotamer, these are also included as the compound of the present invention, and each can be obtained as a single product by a synthesis method and a separation method known per se (concentration, solvent extraction, column chromatography, recrystallization, solvent washing etc.). For example, when an optical isomer is present in the compound of the present invention, an optical isomer resolved from the compound is also encompassed in the compound of the present invention.

An optical isomer of the compound of the present invention can be produced by a method known per se. Specifically, an optical isomer is obtained by using an optically active synthetic intermediate, or optical resolution of the final product racemate according to a conventional method.

The compound of the present invention may be a crystal, and is encompassed in the compound of the present invention whether the crystal form is single or a crystal mixture. A crystal can be produced by crystallization by applying a crystallization method known per se.

The compound of the present invention may be any of a hydrate, a non-hydrate, a solvate and a non-solvate.

Compound (I) and compound (II) labeled with an isotope (e.g., ²H, ³H, ¹³C, ¹⁴C, ¹⁵N, ³⁵S) and the like are also encompassed in the compound of the present invention.

Since the compound of the present invention has an antigen-specific IgG1 subclass antibody production-enhancing effect (immunostimulatory effect), it is useful as an immunostimulating agent. Also, it can enhance production of an antigen-specific IgG2a subclass antibody. The immunostimulating agent of the present invention may be the compound of the present invention per se, or may be obtained by formulating the compound of the present invention by using a pharmacologically acceptable carrier and the like.

As a pharmacologically acceptable carrier that the immunostimulating agent of the present invention may contain, various conventional organic or inorganic carrier substances are used as preparation materials, which are added as excipient, lubricant, binder or disintegrant in solid preparations; solvent, solubilizing agent, suspending agent, isotonicity agent, buffering agent or soothing agent in liquid preparations, and the like. Where necessary, preparation additives such as preservative, antioxidant, colorant, sweetening agent and the like can also be used.

The immunostimulating agent of the present invention may further contain α-cyclodextrin in addition to the compound of the present invention.

In the present invention, α-cyclodextrin refers to cyclic oligosaccharide wherein six D-glucoses form a cyclic structure with α 1→4 bond.

The α-cyclodextrin used in the present invention may be in the form of a derivative. While such derivative is not particularly limited as long as it has the skeleton of α-cyclodextrin, examples thereof include derivatives wherein α-cyclodextrin is chemically modified by methylation and the like or enzymatically modified by maltosylation and the like, and the like.

While α-cyclodextrin used in the present invention can be produced by, for example, enzymatically converting starch by cyclodextrin glucanotransferase, and the like, the production method is not limited thereto and it may be produced by a method known per se. In addition, a commercially available product may be used, and it is convenient and preferable.

While the weight ratio of compound (I) or a salt thereof and α-cyclodextrin (compound (I) or a salt thereof: α-cyclodextrin) in the immunostimulating agent of the present invention is not particularly limited, 1:0.0002 - 2.0000 is preferable, and 1:0.002 - 0.2 is more preferable.

While the weight ratio of compound (II) or a salt thereof and α-cyclodextrin (compound (II) or a salt thereof: α-cyclodextrin) in the immunostimulating agent of the present invention is not particularly limited, 1:0.0002 - 2.0000 is preferable, and 1:0.002 - 0.2 is more preferable.

The immunostimulating agent of the present invention may further contain hydroxypropyl-β-cyclodextrin in addition to the compound of the present invention (preferably, compound (II) or a salt thereof).

In the present invention, hydroxypropyl-β-cyclodextrin refers to β-cyclodextrin, which is a cyclic oligosaccharide wherein 7 seven D-glucoses form a cyclic structure by α1→4 bond, wherein at least one hydroxyl group is substituted by a hydroxypropyl group, and particularly, 2-hydroxypropyl-β-cyclodextrin wherein the above-mentioned hydroxypropyl group is a 2-hydroxypropyl group is preferable.

Hydroxypropyl-β-cyclodextrin to be used in the present invention is not particularly limited as long as it has the skeleton of β-cyclodextrin, and has at least one hydroxypropyl group in the side chain, and may be subjected to, for example, chemical modification such as methylation and the like, enzyme modification such as maltosylation and the like, and the like.

Hydroxypropyl-β-cyclodextrin to be used in the present invention can also be produced by, for example, reacting β-cyclodextrin with propylene oxide under alkali conditions and the like, though the method is not limited thereto, and can be produced by a method known per se. In addition, a commercially available product may be used, since it is convenient and preferable.

While the weight ratio of compound (II) or a salt thereof and hydroxypropyl-β-cyclodextrin (compound (II) or a salt thereof:hydroxypropyl-β-cyclodextrin) in the immunostimulating agent of the present invention is not particularly limited as long as compound (II) or a salt thereof can be in a dissolution state, 1:0.0002 - 2.0000 is preferable, and 1:0.004 - 0.4 is more preferable.

The immunostimulating agent of the present invention may further contain at least one kind selected from the group consisting of carboxymethyl cellulose, polysorbate (e.g., Tween 80 etc.) and polyethylene glycol (e.g., Macrogol etc.) in addition to the compound of the present invention.

While the weight ratio of compound (I) or a salt thereof and carboxymethyl cellulose (compound (I) or a salt thereof: carboxymethyl cellulose) in the immunostimulating agent of the present invention is not particularly limited, 1:0.0005 - 5.0000 is preferable, and 1:0.005 - 0.5 is more preferable.

While the weight ratio of compound (II) or a salt thereof and carboxymethyl cellulose (compound (II) or a salt thereof: carboxymethyl cellulose) in the immunostimulating agent of the present invention is not particularly limited, 1:0.0005 - 5.0000 is preferable, and 1:0.005 - 0.5 is more preferable.

While the weight ratio of compound (I) or a salt thereof and polysorbate (compound (I) or a salt thereof: polysorbate) in the immunostimulating agent of the present invention is not particularly limited, 1:0.0005 - 5.0000 is preferable, and 1:0.005 - 0.5 is more preferable.

While the weight ratio of compound (II) or a salt thereof and polysorbate (compound (II) or a salt thereof: polysorbate) in the immunostimulating agent of the present invention is not particularly limited, 1:0.0005 - 5.0000 is preferable, and 1:0.005 - 0.5 is more preferable.

While the weight ratio of compound (I) or a salt thereof and polyethylene glycol (compound (I) or a salt thereof: polyethylene glycol) in the immunostimulating agent of the present invention is not particularly limited, 1:0.0005 - 5.0000 is preferable, and 1:0.005 - 0.5 is more preferable.

While the weight ratio of compound (II) or a salt thereof and polyethylene glycol (compound (II) or a salt thereof: polyethylene glycol) in the immunostimulating agent of the present invention is not particularly limited, 1:0.0005 - 5.0000 is preferable, and 1:0.005 - 0.5 is more preferable.

Examples of the dosage form of the immunostimulating agent of the present invention include oral preparations such as tablet (including sugar-coated tablet, film-coated tablet, sublingual tablet, orally disintegrating tablet), capsule (including soft capsule, microcapsule), granule, powder, troche, syrup, emulsion, suspension and the like; and parenteral agents such as injection (e.g., subcutaneous injection, intravenous injection, intramuscular injection, intraperitoneal injection, drip infusion), external preparation (e.g., dermal preparation, ointment), suppository (e.g., rectal suppository, vaginal suppository), pellet, drip infusion, eye drop, pulmonary preparation (inhalant) and the like. These preparations may be controlled-release preparations (e.g., sustained-release microcapsule) such as immediate-release preparation, sustained-release preparation and the like.

When the immunostimulating agent of the present invention is an oral preparation, coating may be performed where necessary, aiming at masking taste, enteric property or sustainability. Examples of the coating base to be used for coating include various known coating bases.

The immunostimulating agent of the present invention can be produced by a method used conventionally in the technical field of preparation formulation, for example, the method described in the Japanese Pharmacopoeia, 16th Edition and the like.

The immunostimulating agent of the present invention can be processed into a preparation for children, in addition to that for adults.

The subject of administration of the immunostimulating agent of the present invention is not particularly limited as long as it is an animal having an immune system. Examples thereof include mammals (e.g., human, mouse, rat, rabbit, dog, cat, bovine, horse, swine, monkey etc.), birds (e.g., chicken, duck, goose etc.) and the like. The immunostimulating agent of the present invention can be safely administered orally or parenterally (e.g., topical, rectal, intravenous administration) to them.

Since the compound of the present invention has a superior adjuvant activity as shown in the below-mentioned Examples, the immunostimulating agent of the present invention is useful as an adjuvant (particularly vaccine adjuvant).

The "adjuvant" in the present invention is a generic term for substances that increase antibody production and enhance immune response when combined with an antigen.

When the immunostimulating agent of the present invention is used as an adjuvant, the dosage form thereof may be, for example, an aqueous or a non-aqueous (e.g., oily etc.) solution, suspension, emulsion and the like. These can be prepared by mixing the compound of the present invention with a pharmacologically acceptable carrier (e.g., solvent, suspending agent etc.) and performing a method such as manual shaking, mechanical shaking, ultrasonic dispersing, dispersing by a homomixer, self emulsification, membrane emulsification, D-phase emulsification method, vacuum emulsification method, ultra-high pressure emulsification method and the like.

The immunostimulating agent of the present invention may be used in combination with other adjuvant. Examples of other adjuvant include Freund's Incomplete Adjuvant, Freund's Complete Adjuvant, Montanide ISA, particulates (e.g., urate crystals, silica, aluminum hydroxide gel (e.g., Alum etc.), polystyrene, asbestos, titanium oxide, black nickel oxide, hydroxyapatite etc.), TLR (Toll-like receptor) agonist (e.g., TLR 1/TLR 2 agonist (e.g., Pam3CSK4 etc.), TLR 2/TLR 6 agonist (e.g., MALP-2 etc.), TLR 3 agonist (e.g., polyinosinic polycytidylic acid (Poly I:C) etc.), TLR 4 agonist (e.g., lipopolysaccharide (LPS), monophosphoryl lipid (MPL) etc.), TLR 5 agonist (e.g., flagellin etc.), TLR 7/TLR 8 agonist (e.g., Imiquimod(R-837), Resquimod(R-848) etc.), TLR 9 agonist (e.g., sizofiran-CpG complex, CpG-ODNs etc.), TLR 11 agonist (e.g., profilin etc.), TLR agonist other than the above (e.g., BCG-CWS, OK-432, IC31, 1018ISS etc.)), inulin (e.g. Advax™), cholera toxin B subunit (CTB), ricin, chitosan, saponin (e.g., QS-21 etc.), squalene (e.g., MF59(AddaVax™) etc.), α-GalCer, lipopeptide (e.g., Pam2CSK4, Macrophage-activating lipopeptide 2 etc.), long-chain peptide (e.g., NY-ESO-1 etc.), deoxycholic acid, liposome (e.g., deoxycholic acid-contained liposome, phospholipid-contained liposome etc.), nanoparticle (e.g., γ-PGA nanoparticle, polylactic acid nanoparticle, polystyrene nanoparticle etc.), carbomer homopolymer, ISCOM, biopolymer, β-cyclodextrin, γ-cyclodextrin, surfactant (e.g., benzalkonium-type cationic surfactant, sulfonic acid-type anionic surfactant, saccharide-type non-ionic surfactant, sulfobetaine-type amphoteric surfactant, lung surfactant, surfactin etc.), lipid (e.g., saturated fatty acid, unsaturated fatty acid, cationic lipid, anionic lipid, phospholipid, sphingolipid, lecithin etc.), citrulline, nucleic acid (e.g., ssDNA, dsDNA, ssRNA, dsRNA etc.), c-GMP-AMP, VLP (virus-like particle) (e.g., alphavirus etc.), Mycobacterium tuberculosis adjuvant, acid-fast bacteria-secreted antigen (e.g., Ag85B etc.), probiotic lactic acid bacterium (e.g., lactobacillus plantarum, lactobacillus casei, lactobacillus lactis etc.), cytokine (e.g., interleukin-1, interleukin-2, interleukin-7, interleukin-12, interleukin-15, interleukin-18, TNF-α, GM-CSF, INF-α etc.), CLR (C-type lectin receptor) agonist (e.g., β-glucan, Man9GlcNAc2, TDM, Filamentous actin etc.), cGAS/STING agonist (e.g., cGAMP, cdiGMP, cdiAMP, DMXAA etc.), RIG-1 receptor agonist (e.g., 5-PPP ssRNA etc.), NLR (Nucleotide binding oligomerization domain)-like receptor agonist (e.g., peptidoglycan, KF156, FK565, Murabutide etc.), IRF3 agonist, CD22 agonist and the like.

The immunostimulating agent of the present invention is preferably used in combination with at least one kind selected from the group consisting of aluminum hydroxide gel (e.g., Alum etc.), TLR (Toll-like receptor) agonist (e.g., TLR 1/TLR 2 agonist (e.g., Pam3CSK4 etc.), TLR 2/TLR 6 agonist (e.g., MALP-2 etc.), TLR 3 agonist (e.g., polyinosinic polycytidylic acid (Poly I:C) etc.), TLR 4 agonist (e.g., lipopolysaccharide (LPS), monophosphoryl lipid (MPL) etc.), TLR 5 agonist (e.g., flagellin etc.), TLR 7/TLR 8 agonist (e.g., Imiquimod(R-837), Resquimod(R-848) etc.), TLR 9 agonist (e.g., sizofiran-CpG complex, CpG-ODNs etc.), TLR 11 agonist (e.g., profilin etc.), TLR agonist other than the above (e.g., BCG-CWS, OK-432, IC31, 1018ISS etc.)), saponin (e.g., QS-21 etc.), squalene (e.g., MF59(AddaVax™) etc.), α-GalCer, lipopeptide (e.g., Pam2CSK4, Macrophage-activating lipopeptide 2 etc.), liposome, nucleic acid (e.g., ssDNA, dsDNA, ssRNA, dsRNA etc.), cGAS/STING agonist (e.g., cGAMP, cdiGMP, cdiAMP, DMXAA etc.), RIG-1 receptor agonist (e.g., 5-PPP ssRNA etc.) and NLR (Nucleotide binding oligomerization domain)-like receptor agonist (e.g., peptidoglycan, KF156, FK565, Murabutide etc.). Of these, a combined use with at least one kind selected from the group consisting of polyinosinic polycytidylic acid (PolyI:C), TLR (Toll-like receptor) agonist, monophosphoryl lipid (MPL), CpG-ODNs, nucleic acid (e.g., ssDNA, dsDNA, ssRNA, dsRNA etc.), cGAS/STING agonist, RIG-1 receptor agonist and NLR-like receptor agonist is more preferable.

The present invention also provides a vaccine containing the compound of the present invention and an antigen. A vaccine containing the compound of the present invention and an antigen is one embodiment of a pharmaceutical composition containing the compound of the present invention and an antigen.

The compound of the present invention to be contained in the vaccine of the present invention (That is, compound (I) or a salt thereof, compound (II) or a salt thereof) may be one similar to the compound of the present invention contained in the immunostimulating agent of the present invention.

The antigen to be used in the present invention is not particularly limited as long as it is a substance capable of inducing an immune reaction, and examples thereof include allergen, pathogen antigen, self antigen in the living body, tumor antigen and the like.

The allergen to be used in the present invention can be pollen allergen, food allergen or house dust allergen. The pollen allergen is not particularly limited, and examples thereof include cedar pollen allergen, Japanese cypress pollen allergen, ragweed allergen, Dactylis glomerata allergen and the like. The food allergen is not particularly limited, and examples thereof include casein, lactalbumin, lactoglobulin, ovomucoid, ovalbumin, conalbumin and the like. The house dust allergen is not particularly limited, and examples thereof include mites allergen, cat allergen and the like.

The pathogen antigen to be used in the present invention can be pathogenic virus antigen, pathogenic microorganism antigen or pathogenic protozoan antigen. The pathogenic virus antigen is not particularly limited, and examples thereof include antigen of virus such as human immunodeficiency virus (HIV), hepatitis virus (e.g., type A, type B, type C, type D and type E hepatitis virus), influenza virus (e.g., type A, type B and type C, influenza virus, for example, antigen described in "Surveillance Report Influenza virus characterisation, Summary Europe, September 2015" (http://ecdc.europa.eu/en/publications/surveillance_reports/inf luenza/pages/influenza_virus_characterisation.aspx) etc.), simple herpes virus, West Nile fever virus, human papilloma virus, horse encephalitis virus, human T cell leukemia virus (e.g., HTLV-I), polio virus, varicella-zoster virus, mumps virus, rotavirus, norovirus, RS virus, measles virus, ebola virus and the like, and the antigen of influenza virus is particularly preferably used. The pathogenic microorganism antigen is not particularly limited, and examples thereof include antigens expressed in pathogenic bacterium (e.g., Haemophilus influenzae type B (Hib), pneumococcus, clostridium tetani, corynebacterium diphtheriae, bordetella pertussis, cholera, salmonella, bacillus typhosus, chlamydiae, mycobacteria, legionella), pathogenic yeast (e.g., Aspergillus, Candida) or the like. The pathogenic protozoan antigen is not particularly limited, and examples thereof include antigens expressed in malaria, schistosome or the like.

The self antigen in the living body, which is to be used in the present invention, is not particularly limited, and examples thereof include amyloid β, prion in neurological diseases such as Alzheimer's disease, Creutzfeldt-Jakob disease and the like; ApoB100, angiotensin I, angiotensin II in circulatory diseases such as arteriosclerosis, hypertension and the like; insulin, IL-5 in autoimmune/allergic diseases such as Type I diabetes mellitus, bronchial asthma and the like; IL-6, TNF-α in rheumatoid arthritis, and the like.

The tumor antigen to be used in the present invention can be an antigen of a solid tumor such as epithelial and non-epithelial tumors or an antigen of a tumor in hematopoietic tissue. The solid tumor antigen is not particularly limited, and examples thereof include MART-1/Melan-A, Mage-1, Mage-3, gp100, tyrosinase, tyrosinase-related protein 2 (trp2), CEA, PSA, CA-125, erb-2, Muc-1, Muc-2, TAG-72, AES, FBP, C-lectin, NY-ESO-1, galectin-4/NY-CO-27, Pec60, HER-2/erbB-2/neu, telomerase, G250, Hsp105, point mutated ras oncogene, point mutated p53 oncogene, carcinoembryonic antigen and the like. The antigen of a tumor (e.g., leukemia) in hematopoietic tissue is not particularly limited, and examples thereof include proteinase 3, WT-1, hTERT, PRAME, PML/RAR-a, DEK/CAN, cyclophilin B, TEL-MAL1, BCR-ABL, OFA-iLRP, Survivin, idiotype, Sperm protein 17, SPAN-Xb, CT-27, MUC1 and the like.

The content of the antigen in the vaccine of the present invention may be an effective amount that functions as a vaccine, and the amount can be determined by those of ordinary skill in the art based on, for example, tests using an experiment animal and the like, without requiring undue experiments. Specifically, the content of the antigen in the vaccine of the present invention is generally 1 - 100 µg, based on the total weight of the vaccine.

While the content of the compound of the present invention in the vaccine of the present invention is not particularly limited and may be appropriately adjusted according to, for example, the kind of antigen, subject of administration, administration form, administration route and the like, it is generally 2 µg - 20 mg, preferably 20 µg - 200 µg, based on the total weight of the vaccine, for oral, intramuscular, transdermal, interdermal, subcutaneous or intraperitoneal administration and generally 0.01 µg - 1 mg, preferably 0.1 µg - 100 µg, based on the total weight of the vaccine, for intratracheal, intranasal(transnasal), intraocular, vaginal, rectal, intravenous, intraintestinal or inhalation administration.

The vaccine of the present invention may contain a pharmacologically acceptable carrier in addition to the compound of the present invention and antigen. Examples of the pharmacologically acceptable carrier that the vaccine of the present invention may contain include those recited as examples of the pharmacologically acceptable carrier that the immunostimulating agent of the present invention may contain.

The vaccine of the present invention may further contain other adjuvant. Examples of other adjuvant include those recited as examples of the adjuvant that can be used in combination with the immunostimulating agent of the present invention.

The vaccine of the present invention may contain α-cyclodextrin in addition to the compound of the present invention and an antigen. The α-cyclodextrin that may be contained in the vaccine of the present invention may be similar to α-cyclodextrin that may be contained in the immunostimulating agent of the present invention.

The content of α-cyclodextrin in the vaccine of the present invention is not particularly limited, and 0.005 - 20 wt% is preferable, and 0.05 - 5 wt% is more preferable.

While the weight ratio of the content of compound (I) or a salt thereof and the content of α-cyclodextrin (compound (I) or a salt thereof:α-cyclodextrin) in the vaccine of the present invention is not particularly limited, 1:0.0002 - 2.0000 is preferable, and 1:0.002 - 0.2 is more preferable.

While the weight ratio of the content of compound (II) or a salt thereof and the content of α-cyclodextrin (compound (II) or a salt thereof:α-cyclodextrin) in the vaccine of the present invention is not particularly limited, 1:0.0002 - 2.0000 is preferable, and 1:0.002 - 0.2 is more preferable.

The vaccine of the present invention may contain hydroxypropyl-β-cyclodextrin in addition to the compound of the present invention (preferably, compound (II) or a salt thereof) and an antigen. The hydroxypropyl-β-cyclodextrin that may be contained in the vaccine of the present invention may be similar to hydroxypropyl-β-cyclodextrin that may be contained in the immunostimulating agent of the present invention.

The content of hydroxypropyl-β-cyclodextrin in the vaccine of the present invention is not particularly limited, and 0.005 - 20 wt% is preferable, and 0.05 - 5 wt% is more preferable.

While the weight ratio of the content of compound (II) or a salt thereof and the content of hydroxypropyl-β-cyclodextrin (compound (II) or a salt thereof: hydroxypropyl-β-cyclodextrin) in the vaccine of the present invention is not particularly limited as long as compound (II) or a salt thereof can be in a dissolution state, 1:0.005 -5.0000 is preferable, and 1:0.004 - 0.4 is more preferable.

The vaccine of the present invention may contain at least one kind selected from the group consisting of carboxymethyl cellulose, polysorbate (e.g., Tween 80 etc.) and polyethylene glycol (e.g., Macrogol etc.) in addition to the compound of the present invention and an antigen. The carboxymethyl cellulose, polysorbate (e.g., Tween 80 etc.) and polyethylene glycol (e.g., Macrogol etc.) that may be contained in the vaccine of the present invention may be similar to carboxymethyl cellulose, polysorbate (e.g., Tween 80 etc.) and polyethylene glycol (e.g., Macrogol etc.) that may be contained in the immunostimulating agent of the present invention.

The each content of carboxymethyl cellulose, polysorbate (e.g., Tween 80 etc.) or polyethylene glycol (e.g., Macrogol etc.) in the vaccine of the present invention is not particularly limited, and 0.005 - 20 wt% is preferable, and 0.05 - 5 wt% is more preferable.

While the weight ratio of the content of compound (I) or a salt thereof and the content of carboxymethyl cellulose (compound (I) or a salt thereof: carboxymethyl cellulose) in the vaccine of the present invention is not particularly limited, 1:0.0005 - 5.0000 is preferable, and 1:0.005 - 0.5 is more preferable.

While the weight ratio of the content of compound (II) or a salt thereof and the content of carboxymethyl cellulose (compound (II) or a salt thereof: carboxymethyl cellulose) in the vaccine of the present invention is not particularly limited, 1:0.0005 - 5.0000 is preferable, and 1:0.005 - 0.5 is more preferable.

While the weight ratio of the content of compound (I) or a salt thereof and the content of polysorbate (compound (I) or a salt thereof: polysorbate) in the vaccine of the present invention is not particularly limited, 1:0.0005 - 5.0000 is preferable, and 1:0.005 - 0.5 is more preferable.

While the weight ratio of the content of compound (II) or a salt thereof and the content of polysorbate (compound (II) or a salt thereof: polysorbate) in the vaccine of the present invention is not particularly limited, 1:0.0005 - 5.0000 is preferable, and 1:0.005 - 0.5 is more preferable.

While the weight ratio of the content of compound (I) or a salt thereof and the content of polyethylene glycol (compound (I) or a salt thereof: polyethylene glycol) in the vaccine of the present invention is not particularly limited, 1:0.0005 - 5.0000 is preferable, and 1:0.005 - 0.5 is more preferable.

While the weight ratio of the content of compound (II) or a salt thereof and the content of polyethylene glycol (compound (II) or a salt thereof: polyethylene glycol) in the vaccine of the present invention is not particularly limited, 1:0.0005 - 5.0000 is preferable, and 1:0.005 - 0.5 is more preferable.

The vaccine of the present invention may contain the immunostimulating agent of the present invention and an antigen.

Examples of the dosage form of the vaccine of the present invention include those recited as examples of the dosage form of the immunostimulating agent of the present invention.

The vaccine of the present invention can be produced by a method used conventionally in the technical field of preparation formulation, for example, the method described in the Japanese Pharmacopoeia, 16th Edition and the like. For example, it can be prepared by mixing the compound of the present invention and a desired antigen and, where necessary, emulsifying or dispersing the mixture, or adding the compound of the present invention to a vaccine containing a desired antigen and, where necessary, emulsifying or dispersing the mixture and the like.

The subject of administration of the vaccine of the present invention is not particularly limited as long as it is an animal having an immune system, and examples thereof include those recited as examples of the administration subject of the immunostimulating agent of the present invention.

The vaccine of the present invention may be administered by single administration or multiple successive administrations. When the vaccine of the present invention is successively administered, the dosing period is not particularly limited and can be appropriately set according to, for example, the kind of antigen, subject of administration, administration form, administration route and the like. It is generally within the range of 1 - 90 days, preferably 1 - 30 days.

While the administration route of the vaccine of the present invention is not particularly limited, the vaccine of the present invention is preferably administered by a route selected from oral administration, intramuscular administration, transdermal administration, intradermal administration, subcutaneous administration, intraperitoneal administration, intratracheal administration, intranasal administration (transnasal administration), intraocular administration, vaginal administration, rectal administration, intravenous administration, intraintestinal administration, and inhalation administration, and particularly preferably administered by subcutaneous administration or intranasal administration (transnasal administration). The vaccine of the present invention is particularly preferably administered by intradermal administration.

By administering the vaccine of the present invention to a target, allergy, infection, tumor and the like can be prevented or treated.

The present invention also provides a pharmaceutical composition containing the compound of the present invention and α-cyclodextrin (hereinafter sometimes to be also conveniently indicated as the pharmaceutical composition A of the present invention).

The compound of the present invention (i.e., compound (I) or a salt thereof, compound (II) or a salt thereof) to be contained in the pharmaceutical composition A of the present invention may be one similar to the compound of the present invention which is contained in the immunostimulating agent of the present invention.

As α-cyclodextrin to be contained in the pharmaceutical composition A of the present invention, those similar to α-cyclodextrin that may be contained in the immunostimulating agent of the present invention can be used.

While the content of the compound of the present invention in the pharmaceutical composition A of the present invention is not particularly limited, it is preferably 0.1 - 99.9 wt%, more preferably 1 - 99 wt%.

While the content of α-cyclodextrin in the pharmaceutical composition A of the present invention is not particularly limited, it is preferably 0.005 - 20 wt%, more preferably 0.05 - 5 wt%.

While the weight ratio of the content of the compound (I) or a salt thereof and that of α-cyclodextrin (the compound (I) or a salt thereof: α-cyclodextrin) in the pharmaceutical composition A of the present invention is not particularly limited, it is preferably 1:0.0002 - 2.0000, more preferably 1:0.002 - 0.2.

While the weight ratio of the content of the compound (II) or a salt thereof and that of α-cyclodextrin (the compound (II) or a salt thereof: α-cyclodextrin) in the pharmaceutical composition A of the present invention is not particularly limited, it is preferably 1:0.0002 - 2.0000, more preferably 1:0.002 - 0.2.

The pharmaceutical composition A of the present invention may contain a pharmacologically acceptable carrier in addition to the compound of the present invention and α-cyclodextrin. Examples of the pharmacologically acceptable carrier that the pharmaceutical composition A of the present invention may contain include those similar to those exemplified as the pharmacologically acceptable carrier that the immunostimulating agent of the present invention may contain.

The present invention also provides a pharmaceutical composition containing the compound of the present invention and hydroxypropyl-β-cyclodextrin (hereinafter sometimes conveniently referred to as the pharmaceutical composition B of the present invention).

The compound of the present invention (preferably, compound (II) or a salt thereof) to be contained in the pharmaceutical composition B of the present invention may be one similar to the compound of the present invention which is contained in the immunostimulating agent of the present invention.

The hydroxypropyl-β-cyclodextrin to be contained in the pharmaceutical composition B of the present invention may be one similar to the hydroxypropyl-p-cyclodextrin which is contained in the immunostimulating agent of the present invention.

While the content of the compound of the present invention in the pharmaceutical composition B of the present invention is not particularly limited, it is preferably 0.1 - 99.9 wt%, more preferably 1 - 99 wt%.

While the content of hydroxypropyl-p-cyclodextrin in the pharmaceutical composition B of the present invention is not particularly limited, it is preferably 0.005 - 20 wt%, more preferably 0.05 - 5 wt%.

While the weight ratio of the content of compound (II) or a salt thereof and the content of hydroxypropyl-β-cyclodextrin (compound (II) or a salt thereof: hydroxypropyl-β-cyclodextrin) in the pharmaceutical composition B of the present invention is not particularly limited as long as compound (II) or a salt thereof can be in a dissolution state, 1:0.0002 - 2.0000 is preferable, and 1:0.004 - 0.4 is more preferable.

The pharmaceutical composition B of the present invention may contain a pharmacologically acceptable carrier in addition to the compound of the present invention and hydroxypropyl-β-cyclodextrin. Examples of the pharmacologically acceptable carrier that the pharmaceutical composition B of the present invention may contain include those similar to those exemplified as the pharmacologically acceptable carrier that the immunostimulating agent of the present invention may contain.

The present invention also provides a pharmaceutical composition containing the compound of the present invention and at least one kind selected from the group consisting of carboxymethyl cellulose, polysorbate (e.g., Tween 80 etc.) and polyethylene glycol (e.g., Macrogol etc.) (hereinafter sometimes to be also conveniently indicated as the pharmaceutical composition C of the present invention).

The compound of the present invention (i.e., compound (I) or a salt thereof, compound (II) or a salt thereof) to be contained in the pharmaceutical composition C of the present invention may be one similar to the compound of the present invention which is contained in the immunostimulating agent of the present invention.

As carboxymethyl cellulose, polysorbate (e.g., Tween 80 etc.) and polyethylene glycol (e.g., Macrogol etc.) to be contained in the pharmaceutical composition C of the present invention, those similar to carboxymethyl cellulose, polysorbate (e.g., Tween 80 etc.) and polyethylene glycol (e.g., Macrogol etc.) that may be contained in the immunostimulating agent of the present invention can be used.

While the content of the compound of the present invention in the pharmaceutical composition C of the present invention is not particularly limited, it is preferably 0.1 - 99.9 wt%, more preferably 1 - 99 wt%.

While the each content of carboxymethyl cellulose, polysorbate (e.g., Tween 80 etc.) or polyethylene glycol (e.g., Macrogol etc.) in the pharmaceutical composition C of the present invention is not particularly limited, it is preferably 0.005 - 20 wt%, more preferably 0.05 - 5 wt%.

While the weight ratio of the content of the compound (I) or a salt thereof of the present invention and that of carboxymethyl cellulose (the compound (I) or a salt thereof of the present invention: carboxymethyl cellulose) in the pharmaceutical composition C of the present invention is not particularly limited, it is preferably 1:0.0005 - 5.0000, more preferably 1:0.005 - 0.5.

While the weight ratio of the content of the compound (II) or a salt thereof of the present invention and that of carboxymethyl cellulose (the compound (II) or a salt thereof of the present invention: carboxymethyl cellulose) in the pharmaceutical composition C of the present invention is not particularly limited, it is preferably 1:0.0005 - 5.0000, more preferably 1:0.005 - 0.5.

While the weight ratio of the content of the compound (I) or a salt thereof of the present invention and that of polysorbate (the compound (I) or a salt thereof of the present invention: polysorbate) in the pharmaceutical composition C of the present invention is not particularly limited, it is preferably 1:0.0005 - 5.0000, more preferably 1:0.005 - 0.5.

While the weight ratio of the content of the compound (II) or a salt thereof of the present invention and that of polysorbate (the compound (II) or a salt thereof of the present invention: polysorbate) in the pharmaceutical composition C of the present invention is not particularly limited, it is preferably 1:0.0005 - 5.0000, more preferably 1:0.005 - 0.5.

While the weight ratio of the content of the compound (I) or a salt thereof of the present invention and that of polyethylene glycol (the compound (I) or a salt thereof of the present invention: polyethylene glycol) in the pharmaceutical composition C of the present invention is not particularly limited, it is preferably 1:0.0005 - 5.0000, more preferably 1:0.005 - 0.5.

While the weight ratio of the content of the compound (II) or a salt thereof of the present invention and that of polyethylene glycol (the compound (II) or a salt thereof of the present invention: polyethylene glycol) in the pharmaceutical composition C of the present invention is not particularly limited, it is preferably 1:0.0005 - 5.0000, more preferably 1:0.005 - 0.5.

The pharmaceutical composition C of the present invention may contain a pharmacologically acceptable carrier in addition to the compound of the present invention and at least one kind selected from the group consisting of carboxymethyl cellulose, polysorbate (e.g., Tween 80 etc.) and polyethylene glycol (e.g., Macrogol etc.). Examples of the pharmacologically acceptable carrier that the pharmaceutical composition C of the present invention may contain include those similar to those exemplified as the pharmacologically acceptable carrier that the immunostimulating agent of the present invention may contain.

Examples of the dosage form of the pharmaceutical compositions A, B and C of the present invention include those similar to those exemplified as the dosage form of the immunostimulating agent of the present invention.

The pharmaceutical compositions A, B and C of the present invention can be produced by a method used conventionally in the technical field of preparation formulation, for example, the method described in the Japanese Pharmacopoeia, 16th Edition and the like.

Examples of the administration subject of the pharmaceutical compositions A, B and C of the present invention include those recited as examples of the administration subject of the immunostimulating agent of the present invention.

The pharmaceutical composition A of the present invention may also be provided in the form of a kit wherein the compound of the present invention and α-cyclodextrin are separately packaged.

The pharmaceutical composition B of the present invention may also be provided in the form of a kit wherein the compound of the present invention and hydroxypropyl-p-cyclodextrin are separately packaged.

The pharmaceutical composition C of the present invention may also be provided in the form of a kit wherein the compound of the present invention and at least one kind selected from the group consisting of carboxymethyl cellulose, polysorbate (e.g., Tween 80 etc.) and polyethylene glycol (e.g., Macrogol etc.) are separately packaged.

Since the pharmaceutical compositions A, B and C of the present invention have an antigen-specific IgG1 or IgG2a subclass antibody production-enhancing effect (immunostimulatory effect), respectively, they may be used, for example, as immunostimulating agents, adjuvants (e.g., vaccine adjuvant etc.) and the like.

### Examples

The present invention is explained in more detail in the following by referring to Synthesis Examples and Examples, which do not limit the present invention. The present invention may be modified without departing from the scope of the invention. The reagents, apparatuses and materials used in the present invention are commercially available unless particularly indicated.

### (1) Synthesis of the compound of the present invention

### [Synthetic Example 1]

### Synthesis of N²-hexadecanoyl-L-arginine (SZ61)

To L-arginine (5.00 g, 28.7 mmol) were added water (17.9 ml) and isopropyl alcohol (53.8 ml), and the mixture was adjusted to pH 11 with 27% aqueous sodium hydroxide solution. While maintaining the liquid temperature at 27.8 - 28.3°C and pH at 10.5 - 11.5, palmitoyl chloride (9.58 ml, 31.6 mmol) and 27% aqueous sodium hydroxide solution (4.68 ml, 31.6 mmol) were added dropwise over 90 min, and the mixture was stirred at 28°C for 1 hr. Thereafter, the pH was adjusted to 12 with 27% aqueous sodium hydroxide solution, and the mixture was heated to 52°C. The pH was adjusted to 6 with concentrated hydrochloric acid, and the mixture was cooled to 10°C over 1 hr. The obtained suspension was filtered, and the solid collected by filtration was washed with water (150 ml) and isopropyl alcohol (100 ml) to give N²-hexadecanoyl-L-arginine (10.7 g, 26.0 mmol, yield 91%).
^{1l}H-NMR (400 MHz, CD₃COOD)δ: 0.91(t, 3H, J=6.8 Hz), 1.28-1.42(m, 24H), 1.65(m, 2H), 1.73(m, 2H), 1.80-2.04(m, 2H), 2.37(t, 2H, J=7.6 Hz), 3.30(m, 2H), 4.64(m, 1H).
ESIMS (m/z) : 413.4([M+2H]²⁺), 825.7([M+H]⁺), 823.9([M-H]⁻).

### [Synthetic Example 2]

### Synthesis of N-hexadecanoylagmatine (SZ62)

To agmatine sulfate (5.00 g, 21.9 mmol) were added water (34.5 ml) and isopropyl alcohol (20.3 ml), and the mixture was adjusted to pH 11 with 27% aqueous sodium hydroxide solution. Using a cooling apparatus, the mixture was cooled to 10°C. While maintaining the liquid temperature at 10.5 - 11.5°C and pH at 10.8 - 11.3, palmitoyl chloride (6.64 ml, 21.9 mmol) and 27% aqueous sodium hydroxide solution (3.24 ml, 21.9 mmol) were added dropwise over 40 min, and the mixture was stirred at 10°C for 30 min and at room temperature for 1 hr. Thereafter, the mixture was heated to 60°C, isopropyl alcohol (10 ml) was added, and pH was adjusted to 12 with 27% aqueous sodium hydroxide solution, and the mixture was cooled to room temperature with stirring. The obtained suspension was filtered, and the solid collected by filtration was washed with water to give a crude product (6.2 g). This was dissolved in methanol, treated with an anion exchange resin (Amberlite IRA400 OH) and the eluate was concentrated under reduced pressure to give N-hexadecanoylagmatine (4.30 g, 11.7 mmol, yield 53%) as a white solid.
¹H-NMR (400 MHz, CD₃OD)δ: 0.92(t, 3H, J=6.8 Hz), 1.25-1.38(m, 24H), 1.55-1.65(m, 6H), 2.19(t, 2H, J=7.6 Hz), 3.16-3.23(m, 4H). ESIMS (m/z) : 369.3([M+H]⁺), 737.7([2 M+H]⁺), 413.2([M+HCOO]⁻).

### [Synthetic Example 3]

### Synthesis of N²-hexadecanoyl-L-arginine methyl ester hydrochloride (SZ63)

N²-hexadecanoyl-L-arginine (300 mg, 0.73 mmol) synthesized in the same manner as in Synthetic Example 1 was dissolved in methanol, 3 drops of thionyl chloride was added with a 2 mL Komagome pipette, and the mixture was stirred at 50°C for 23 hr. After cooling to room temperature, the mixture was concentrated under reduced pressure to give N²-hexadecanoyl-L-arginine methyl ester hydrochloride (200 mg, 0.43 mmol, yield 79%) as a white solid.
¹H-NMR (400 MHz, CDCl₃)δ: 0.90(t, 3H, J=6.8 Hz), 1.24-1.32(m, 24H), 1.62(m, 2H), 1.71(m, 2H), 1.78-2.02(m, 2H), 2.31(t, 2H, J=7.6 Hz), 3.23(m, 1H), 3.41(m, 1H), 3.77(s, 3H), 4.50(m, 1H), 7.02-7.12(m, 2H), 7.92(brs, 1H).
ESIMS (m/z) : 427.4([M+H]⁺), 853.7([2 M+H]⁺), 471.2([M+HCOO]⁻).

### [Synthetic Example 4]

### Synthesis of N²-octadecanoyl-L-glutamine tert-butyl ester (SZ64)

To L-glutamine tert-butyl ester hydrochloride (300 mg, 1.27 mmol) were added N,N-dimethylformamide (12.7 ml) and stearic acid (536 mg, 1.89 mmol) at room temperature, and the mixture was cooled in an ice bath. Then, 1-ethyl-3-(3-dimethylaminopropyl)carbodiimide hydrochloride (362 mg, 1.89 mmol) and 1-hydroxybenzotriazole (255 mg, 1.89 mmol), triethylamine (0.53 ml, 3.81 mmol) were added, and the mixture was removed from the ice bath and stirred at room temperature for 21.5 hr. 10% Aqueous citric acid solution (30 ml) was added to discontinue the reaction and the mixture was extracted with ethyl acetate (60 ml, 50 ml). The combined organic layers were washed twice with saturated aqueous sodium hydrogen carbonate solution (50 ml), once with 15% brine (50 ml), dried over anhydrous magnesium sulfate, filtered and the filtrate was concentrated under reduced pressure. The obtained solid was slurry washed with ethyl acetate/hexane (1/1, v/v) (20 ml) to give N²-octadecanoyl-L-glutamine tert-butyl ester (387.3 mg, 0.83 mmol, yield 83%) as a white solid.
¹H-NMR (400 MHz, CDCl₃)δ: 0.88(t, 3H, J=7.0 Hz), 1.20-1.30(m, 28H), 1.47(s, 9H), 1.63(m, 2H), 1.87(m, 1H), 2.14-2.27(m, 3H), 2.31(m, 2H), 4.48(m, 1H), 5.33(brs, 1H), 6.30(d, 1H, J=7.6 Hz), 6.57(brs, 1H).
ESIMS( m/z) :469.35([M+H]⁺).

### [Synthetic Example 5]

### Synthesis of N²-octadecanoyl-L-glutamine (SZ65)

To N²-octadecanoyl-L-glutamine tert-butyl ester (200 mg, 0.43 mmol) synthesized in the same manner as in Synthetic Example 4 were added dichloromethane (2.15 ml) and triisopropylsilane (0.44 ml, 2.13 mmol) at room temperature and the mixture was cooled in an ice bath. Then, trifluoroacetic acid (2.15 ml) was added, and the mixture was removed from the ice bath and stirred at room temperature for 2 hr. Thereafter, the mixture was concentrated under reduced pressure together with toluene, and slurry washed with ethyl acetate/hexane (1/1) to give N²-octadecanoyl-L-glutamine (161 mg, 0.31 mmol, yield 71%) as a white solid.
¹H-NMR (400 MHz, DMSO)δ: 0.85(t, 3H, J=6.8 Hz), 1.18-1.28(m, 28H), 1.47(m, 2H), 1.72(m, 1H), 1.91(m, 1H), 2.07-2.14(m, 4H), 4.13(m, 1H), 6.75(brs, 1H), 7.27(brs, 1H), 8.03(d, 1H, J=7.6 Hz) .
ESIMS (m/z) : 411.20([M-H]⁻), 823.55([2 M-H]⁻).

### [Synthetic Example 6]

### Synthesis of N-docosanoyl glycine methyl ester (SZ69)

To glycine methyl ester hydrochloride (500 mg, 3.98 mmol) were added N,N-dimethylformamide (39.8 ml) and behenic acid (2.03 g, 5.97 mmol) at room temperature, and the mixture was cooled in an ice bath. Then, 1-ethyl-3-(3-dimethylaminopropyl)carbodiimide hydrochloride (1.14 g, 5.97 mmol), 1-hydroxybenzotriazole (807 mg, 5.97 mmol), and triethylamine (1.67 ml, 12.0 mmol) were added, and the mixture was removed from the ice bath and stirred at room temperature for 19.5 hr. Thereafter, to the reaction mixture were added 10% aqueous citric acid solution (25 ml) and ethyl acetate (70 ml) and the mixture was filtered. The solid collected by filtration was slurry washed with hexane/ethyl acetate(2/3, v/v) (30 ml), diethyl ether (30 ml), and water (30 ml). Of the obtained solid (1.31 g), 643 mg was taken and purified by silica gel column chromatography (hexane/ethyl acetate) to give N-docosanoyl glycine methyl ester (308 mg, 0.75 mmol, yield 38%) as a white solid.
¹H-NMR (400 MHz, CDCl₃)δ: 0.88(t, 3H, J=7.0 Hz), 1.25-1.30(m, 36H), 1.64(m, 2H), 2.24(t, 2H, J=7.6 Hz), 3.77(s, 3H), 4.05(d, 2H, J=5.2 Hz), 5.91(s, 1H).
ESIMS (m/z) : 412.4 ([M+H]⁺), 823.8 ([2 M+H]⁺).

### [Synthetic Example 7]

### Synthesis of N-docosanoyl-L-leucine methyl ester (SZ70)

To L-leucine methyl ester hydrochloride (500 mg, 2.75 mmol) were added N,N-dimethylformamide (28 ml), and behenic acid (1.41 g, 4.13 mmol) at room temperature, and the mixture was cooled in an ice bath. Then, 1-ethyl-3-(3-dimethylaminopropyl)carbodiimide hydrochloride (791 mg, 4.13 mmol), 1-hydroxybenzotriazole (558 mg, 4.13 mmol), and triethylamine (1.15 ml, 8.26 mmol) were added, and the mixture was removed from the ice bath and stirred at room temperature for 20 hr. To the reaction mixture were added 10% aqueous citric acid solution (25 ml), and ethyl acetate (70 ml) and the mixture was filtered. The solid collected by filtration was purified by silica gel column chromatography (hexane/ethyl acetate). Finally, the obtained solid was slurry washed with ethyl acetate (10 ml), and methanol (10 ml) to give N-docosanoyl-L-leucine methyl ester (176 mg, 0.38 mmol, yield 14%) as a white solid.
¹H-NMR (400 MHz, CDCl₃)δ: 0.88 (t, 3H, J=6.8 Hz), 0.94(m, 6H), 1.25-1.29(m, 36H), 1.48-1.68(m, 5H), 2.21(t, 2H, J=7.6 Hz), 3.73(s, 3H), 4.66(dt, 1H, J=4.8 Hz, 8.8 Hz), 5.76(d, 1H, J=8.0 Hz) .
ESIMS (m/z) : 468.4 ([M+H]⁺), 935. 9 ([2 M+H]⁺).

### [Synthetic Example 8]

### Synthesis of N-docosanoyl-L-phenylalanine methyl ester (SZ71)

To L-phenylalanine methyl ester hydrochloride (500 mg, 2.32 mmol) were added N,N-dimethylformamide (23 ml), and behenic acid (1.18 g, 3.48 mmol) at room temperature, and the mixture was cooled in an ice bath. Then, 1-ethyl-3-(3-dimethylaminopropyl)carbodiimide hydrochloride (667 mg, 3.48 mmol), 1-hydroxybenzotriazole (470 mg, 3.48 mmol), and triethylamine (0.97 ml, 6.95 mmol) were added, and the mixture was removed from the ice bath and stirred at room temperature for 20 hr. Thereafter, 10% aqueous citric acid solution (30 ml) and ethyl acetate (70 ml) were added and the mixture was filtered. The solid collected by filtration was purified by silica gel column chromatography (hexane/ethyl acetate) to give N-docosanoyl-L-phenylalanine methyl ester (300 mg, 0.60 mmol, yield 26%) as a white solid.
¹H-NMR (400 MHz, CDCl₃)δ: 0.88(t, 3H, J=6.8 Hz), 1.20-1.32(m, 36H), 1.58(m, 2H), 2.16(t, 2H, J=7.2 Hz), 3.10(dd, 1H, J=8.4 Hz, 14.0 Hz), 3.16(dd, 1H, J=6.0 Hz, 14.0 Hz), 3.73(s, 3H), 4.91(dt, 1H, J=7.6 Hz, 6.0 Hz), 5.83(d, 1H, J=7.6 Hz), 7.08-7.10(m, 2H), 7.22-7.31(m, 3H).
ESIMS (m/z): 502.5([M+H]⁺).

### [Synthetic Example 9-1]

### Synthesis of N-docosanoyl-L-glutamic acid 5-tert-butyl-1-methyl ester

To L-glutamic acid 5-tert-butyl-1-methyl ester hydrochloride (543 mg, 2.14 mmol) were added N,N-dimethylformamide (14.3 ml), and behenic acid (875 mg, 2.57 mmol) at room temperature, and the mixture was cooled in an ice bath. Then, 1-ethyl-3-(3-dimethylaminopropyl)carbodiimide hydrochloride (493 mg, 2.57 mmol), 1-hydroxybenzotriazole (347 mg, 2.57 mmol), and triethylamine (0.60 ml, 4.3 mmol) were added, and the mixture was removed from the ice bath and stirred at room temperature for 22 hr. Ethyl acetate (25 ml) was added and the mixture was filtered. The solid collected by filtration was washed three times with ethyl acetate (10 ml) and three times with methanol (10 ml) to give N-docosanoyl-L-glutamic acid 5-tert-butyl-1-methyl ester (281 mg, 0.52 mmol, yield 24%) as a white solid.
¹H-NMR (400 MHz, CDCl₃)δ: 0.88(t, 3H, J=7.0 Hz), 1.25-1.28(m, 36H), 1.44(s, 9H), 1.62(m, 2H), 1.96(m, 1H), 2.12(m, 1H), 2.20(t, 2H, J=7.6 Hz), 2.30(m, 2H), 3.74(s, 3H), 4.59(dt, 1H, J=5.2 Hz, 8.0 Hz), 6.19(d, 1H, J=7.6 Hz).
ESIMS (m/z) : 540.5([M+H]⁺), 1079.9([2 M+H]⁺).

### [Synthetic Example 9-2]

### Synthesis of N-docosanoyl-L-glutamic acid 1-methyl ester (SZ72)

To N-docosanoyl-L-glutamic acid 5-tert-butyl-1-methyl ester (200 mg, 0.37 mmol) synthesized in Synthetic Example 9-1 were added dichloromethane (3.7 ml) and triisopropylsilane (0.38 ml, 1.85 mmol) at room temperature, and the mixture was cooled in an ice bath. Then, trifluoroacetic acid (3.7 ml) was added, and the mixture was removed from the ice bath and stirred at room temperature for 45 min. The mixture was concentrated under reduced pressure together with toluene to give N-docosanoyl-L-glutamic acid 1-methyl ester (178 mg, 0.37 mmol, yield 99%) as a white solid.
¹H-NMR (400 MHz, CDCl₃)δ: 0.88(t, 3H, J=6.8 Hz), 1.20-1.30(m, 36H), 1.62(m, 2H), 2.02(m, 1H), 2.28(m, 1H), 2.36(t, 2H, J=7.6 Hz), 2.56(m, 2H), 3.83(s, 3H), 4.76(dt, 1H, J=5.3 Hz, 8.0 Hz), 6.83(d, 1H, J=7.6 Hz).
ESIMS (m/z) : 484.4([M+H]⁺), 482.2([M-H]⁻).

### [Synthetic Example 10-1]

### Synthesis of N⁶-(tert-butoxycarbonyl)-N²-docosanoyl-L-lysine methyl ester

To N⁶-(tert-butoxycarbonyl)-L-lysine methyl ester hydrochloride (300 mg, 1.0 mmol) were added N,N-dimethylformamide (6.7 ml) and behenic acid (412 mg, 1.2 mmol) at room temperature, and the mixture was cooled in an ice bath. Then, 1-ethyl-3-(3-dimethylaminopropyl)carbodiimide hydrochloride (232 mg, 1.2 mmol), 1-hydroxybenzotriazole (164 mg, 1.2 mmol), and triethylamine (0.28 ml, 2.0 mmol) were added, and the mixture was removed from the ice bath and stirred at room temperature for 22 hr. Thereafter, ethyl acetate (10 ml) was added and the mixture was filtered. The solid collected by filtration was washed three times with ethyl acetate (10 ml) to give N⁶-(tert-butoxycarbonyl)-N²-docosanoyl-L-lysine methyl ester (75.8 mg, 0.13 mmol, yield 13%) as a white solid.
¹H-NMR (400 MHz, CDCl₃)δ: 0.88(t, 3H, J=6.8 Hz), 1.25-1.30(m, 36H), 1.44(s, 9H), 1.49(m, 2H), 1.59-1.72(m, 3H), 1.84(m, 1H), 2.22(t, 2H, J=7.6 Hz), 3.10(m, 2H), 3.74(s, 3H), 4.56-4.63(m, 2H), 6.01(d, 1H, J=6.4 Hz).
ESIMS (m/z): 583.5([M+H]⁺).

### [Synthetic Example 10-2]

### Synthesis of N²-docosanoyl-L-lysine methyl ester trifluoroacetic acid salt (SZ73)

To N⁶-(tert-butoxycarbonyl)-N²-docosanoyl-L-lysine methyl ester (73.8 mg, 0.13 mmol) synthesized in Synthetic Example 10-1 were added dichloromethane (1.3 ml) and triisopropylsilane (0.13 ml, 0.63 mmol) at room temperature, and the mixture was cooled in an ice bath. Then, trifluoroacetic acid (1.3 ml) was added, and the mixture was removed from the ice bath and stirred at room temperature for 50 min. Thereafter, the mixture was concentrated under reduced pressure together with toluene to give N²⁻docosanoyl-L-lysine methyl ester trifluoroacetic acid salt (78.8 mg, 0.13 mmol, yield 100%) as a white solid.
¹H-NMR (400 MHz, CDCl₃)δ: 0.88(t, 3H, J=6.8 Hz), 1.25-1.30(m, 36H), 1.60(m, 2H), 1.73(m, 2H), 1.94(m, 2H), 2.35(dd, 2H, J=6.4 Hz, 8.4 Hz), 3.13-3.23(m, 2H), 3.84(s, 3H), 4.73(dt, 1H, J=4.4 Hz, 8.8 Hz), 6.87-7.00(m, 3H).
ESIMS(m/z) : 483.4([M+H]⁺), 482.2([M-H]⁻).

### [Synthetic Example 11]

### Synthesis of N-docosanoyl-L-isoleucine methyl ester (SZ76)

To L-isoleucine methyl ester hydrochloride (500 mg, 2.75 mmol) were added N,N-dimethylformamide (28 ml) and behenic acid (1.41 g, 4.13 mmol) at room temperature, and the mixture was cooled in an ice bath. Then, 1-ethyl-3-(3-dimethylaminopropyl)carbodiimide hydrochloride (792 mg, 4.13 mmol), 1-hydroxybenzotriazole (558 mg, 4.13 mmol), and triethylamine (1.15 ml, 8.25 mmol) were added, and the mixture was removed from the ice bath and stirred at room temperature for 18 hr. Thereafter, ethyl acetate (70 ml) was added and the mixture was filtered. The filtrate was concentrated under reduced pressure, and the residue was purified by silica gel column chromatography (hexane/ethyl acetate) to give resultant product containing the object product as a main component.
This was dissolved in dichloromethane (70 ml), washed three times with saturated aqueous sodium hydrogen carbonate solution (20 ml), and once with water (30 ml) and 15% brine (30 ml), and the organic layer was concentrated under reduced pressure. Of the residue (886 mg), 200 mg was taken and purified by PTLC (hexane/ethyl acetate) to give N-docosanoyl-L-isoleucine methyl ester (112 mg, 0.24 mmol, yield 39%) as a white solid.
¹H-NMR (400 MHz, CDCl₃)δ: 0.86-0.94(m, 9H), 1.11-1.33(m, 37H), 1.43(m, 1H), 1.63(m, 2H), 1.87(m, 1H), 2.21(t, 2H, J=7.6 Hz), 3.73(s, 3H), 4.62(dd, 1H, J=4.8 Hz, 8.8 Hz), 5.91(d, 1H, J=8.4 Hz) .
ESIMS (m/z) : 468.4([M+H]⁺), 935.9([2 M+H]⁺).

### [Synthetic Example 12]

### Synthesis of N-docosanoyl-L-valine methyl ester (SZ77)

To L-valine methyl ester hydrochloride (500 mg, 2.98 mmol) were added N,N-dimethylformamide (29.8 ml) and behenic acid (1.52 g, 4.47 mmol) at room temperature, and the mixture was cooled in an ice bath. Then, 1-ethyl-3-(3-dimethylaminopropyl)carbodiimide hydrochloride (857 mg, 4.47 mmol), 1-hydroxybenzotriazole (604 mg, 4.47 mmol), and triethylamine (1.25 ml, 8.94 mmol) were added, and the mixture was removed from the ice bath and stirred at room temperature for 18 hr. Thereafter, ethyl acetate (70 ml) was added and the mixture was filtered. The filtrate was concentrated under reduced pressure, and the residue was purified by silica gel column chromatography (hexane/ethyl acetate). The obtained solid was slurry washed with diethyl ether (30 ml) to give N-docosanoyl-L-valine methyl ester (331 mg, 0.73 mmol, yield 25%) as a white solid.
¹H-NMR (400 MHz, CDCl₃)δ: 0.86-0.95(m, 9H), 1.25-1.33(m, 36H), 1.64(m, 2H), 2.15(dsep, 1H, J=4.8 Hz, 6.8 Hz), 2.23(t, 2H, J=7.6 Hz), 3.74(s, 3H), 4.59(dd, 1H, J=4.8 Hz, 8.8 Hz), 5.88(d, 1H, J=8.4 Hz).
ESIMS (m/z) : 454.4([M+H]⁺), 907.8([2 M+H]⁺).

### [Synthetic Example 13]

### Synthesis of N-hexadecanoyl glycine methyl ester (SZ78)

To glycine methyl ester hydrochloride (65.3 mg, 0.52 mmol) were added N,N-dimethylformamide (5.2 ml) and palmitic acid (200 mg, 0.78 mmol) at room temperature, and the mixture was cooled in an ice bath. Then, 1-ethyl-3-(3-dimethylaminopropyl)carbodiimide hydrochloride (150 mg, 0.78 mmol), 1-hydroxybenzotriazole (105 mg, 0.78 mmol), and triethylamine (0.22 ml, 1.56 mmol) were added, and the mixture was removed from the ice bath and stirred at room temperature for 19 hr. Thereafter, silica gel was added to the reaction solution to evaporate the solvent, and the residue was purified by silica gel column chromatography (hexane/ethyl acetate) to give N-hexadecanoyl glycine methyl ester (99.3 mg, 0.30 mmol, yield 58%) as a white solid.
¹H-NMR (400 MHz, CDCl₃)δ: 0.88(t, 3H, J=6.8 Hz), 1.25-1.30(m, 24H), 1.64(m, 2H), 2.23(t, 2H, J=7.6 Hz), 3.77(s, 3H), 4.06(d, 2H, J=4.8 Hz), 5.91(s, 1H).
ESIMS (m/z) : 328.2([M+H]⁺), 655.6([2 M+H]⁺).

### [Synthetic Example 14]

### Synthesis of N-hexadecanoyl-L-leucine methyl ester (SZ79)

To L-leucine methyl ester hydrochloride (94.5 mg, 0.52 mmol) were added N,N-dimethylformamide (5.2 ml) and palmitic acid (200.0 mg, 0.78 mmol) at room temperature, and the mixture was cooled in an ice bath. Then, 1-ethyl-3-(3-dimethylaminopropyl)carbodiimide hydrochloride (150 mg, 0.78 mmol), 1-hydroxybenzotriazole (105 mg, 0.78 mmol), and triethylamine (0.22 ml, 1.56 mmol) were added, and the mixture was removed from the ice bath and stirred at room temperature for 22 hr. Silica gel was added to the reaction solution to evaporate the solvent, and the residue was purified by silica gel column chromatography (hexane/ethyl acetate) and PTLC (hexane/ethyl acetate) to give N-hexadecanoyl-L-leucine methyl ester (184 mg, 0.48 mmol, yield 92%) as a white solid.
¹H-NMR (400 MHz, CDCl₃)δ: 0.88(t, 3H, J=6.8 Hz), 0.94(m, 6H), 1.25-1.30(m, 24H), 1.50-1.68(m, 5H), 2.21(t, 2H, J=7.6 Hz), 3.73(s, 3H), 4.66(dt, 1H, J=5.2 Hz, 8.4 Hz), 5.78(d, 1H, J=8.0 Hz) .
ESIMS (m/z) : 384.3([M+H]⁺), 767.7([2 M+H]⁺).

### [Synthetic Example 14B]

### N-hexadecanoyl-L-leucine methyl ester (SZ79)

To a solution of the hexadecanoic acid (5.0 g, 20 mmol), 1-ethyl-3-(3-dimethylaminopropyl)carbodiimide hydrochloride (5.6 g, 29 mmol) and 1-hydroxybenzotriazole (3.9 g, 29 mmol) in N,N-dimethylformamide (150 mL) was added N,N-diisopropylethylamine (10 g, 78 mmol) dropwise. After addition, the mixture was stirred at room temperature for 15 minutes. The L-leucine methyl ester hydrochloride (5.3 g, 29 mmol) was added to the above mixture, and the mixture was stirred at 25°C overnight. The mixture was poured into water, and the precipitate was stirred for 15 minutes. The white solid was afforded by filtration, washed with water, ethyl acetate, dried under reduced pressure to provide N-hexadecanoyl-L-leucine methyl ester (SZ79, 5.5 g, yield 74%) as a white solid.
¹HNMR (CDCl₃, 400 MHz) δ: 5.85 (d, 1H, J=8.0 Hz), 4.63-4.68 (m, 1H), 3.73 (s, 3H), 2.21 (t, 2H, J=7.6 Hz), 1.50-1.68 (m, 5H), 1.20-1.30 (m, 24H), 0.86-0.95 (m, 9H). MS: 384.3 [M+H]⁺.

### [Synthetic Example 15]

### Synthesis of N-hexadecanoyl-L-phenylalanine methyl ester (SZ80)

To L-phenylalanine methyl ester hydrochloride (112 mg, 0.52 mmol) were added N,N-dimethylformamide (5.2 ml), and palmitic acid (200 mg, 0.78 mmol) at room temperature, and the mixture was cooled in an ice bath. Then, 1-ethyl-3-(3-dimethylaminopropyl)carbodiimide hydrochloride (150 mg, 0.78 mmol), 1-hydroxybenzotriazole (105 mg, 0.78 mmol), and triethylamine (0.22 ml, 1.56 mmol) were added, and the mixture was removed from the ice bath and stirred at room temperature for 19 hr. Silica gel was added to the reaction solution to evaporate the solvent, and the residue was purified by silica gel column chromatography (hexane/ethyl acetate) and PTLC (hexane/ethyl acetate) to give N-hexadecanoyl-L-phenylalanine methyl ester (164 mg, 0.39 mmol, yield 76%) as a white solid.
¹H-NMR (400 MHz, CDCl₃)δ: 0.88(t, 3H, J=6.8 Hz), 1.25-1.32(m, 24H), 1.56-1.60(m, 2H), 2.16(t, 2H, J=8.0 Hz), 3.10(dd, 1H, J=5.6 Hz, 13.6 Hz), 3.15(dd, 1H, J=5.6 Hz, 13.6 Hz), 3.73(s, 3H), 4.91(dt, 1H, J=7.6 Hz, 6.0 Hz), 5.83(d, 1H, J=7.2 Hz), 7.07-7.10(m, 2H), 7.24-7.31(m, 3H).
ESIMS (m/z) : 418.3([M+H]⁺), 835.7([2 M+H]⁺).

### [Synthetic Example 16-1]

### Synthesis of N⁶-(tert-butoxycarbonyl)-N²-hexadecanoyl-L-lysine methyl ester

To N⁶-(tert-butoxycarbonyl)-L-lysine methyl ester hydrochloride (154 mg, 0.52 mmol) were added N,N-dimethylformamide (5.2 ml), and palmitic acid (200 mg, 0.78 mmol) at room temperature, and the mixture was cooled in an ice bath. Then, 1-ethyl-3-(3-dimethylaminopropyl)carbodiimide hydrochloride (150 mg, 0.78 mmol), 1-hydroxybenzotriazole (105 mg, 0.78 mmol), and triethylamine (0.22 ml, 1.56 mmol) were added, and the mixture was removed from the ice bath and stirred at room temperature for 22 hr. Silica gel was added to the reaction solution to evaporate the solvent, and the residue was purified by silica gel column chromatography (hexane/ethyl acetate) to give N⁶-(tert-butoxycarbonyl)-N²-hexadecanoyl-L-lysine methyl ester (254 mg, 0.51 mmol, yield 98%) as a white solid.
¹H-NMR (400 MHz, CDCl₃)δ: 0.86(t, 3H, J=6.8 Hz), 1.23-1.35(m, 26H), 1.42(s, 9H), 1.47(m, 2H), 1.57-1.67(m, 3H), 1.81(m, 1H), 2.20(t, 2H, J=7.6 Hz), 3.08(m, 2H), 3.72(s, 3H), 4.57(m, 1H), 4.64(brs, 1H), 6.16(d, 1H, J=7.2 Hz).
ESIMS (m/z): 499.40([M+H]⁺).

### [Synthetic Example 16-2]

### Synthesis of N²-hexadecanoyl-L-lysine methyl ester trifluoroacetic acid salt (SZ81)

To N⁶-(tert-butoxycarbonyl)-N²-hexadecanoyl-L-lysine methyl ester (250 mg, 0.50 mmol) synthesized in Synthetic Example 16-1 were added dichloromethane (5 ml), and triisopropylsilane (0.52 ml, 2.5 mmol) at room temperature and the mixture was cooled in an ice bath. Then, trifluoroacetic acid (5 ml) was added, and the mixture was removed from the ice bath and stirred at room temperature for 1.5 hr. The mixture was concentrated under reduced pressure, and the residue was purified by PTLC (methanol/ethyl acetate) to give N²-hexadecanoyl-L-lysine methyl ester trifluoroacetic acid salt (182 mg, 0.35 mmol, yield 71%) as a white solid.
¹H-NMR (400 MHz, CD₃OD)δ: 0.90(t, 3H, J=7.0 Hz), 1.29-1.33(m, 24H), 1.45(m, 2H), 1.60-1.76(m, 5H), 1.88(m, 1H), 2.24(t, 2H, J=7.6 Hz), 2.92(t, 2H, J=7.6 Hz), 3.72(s, 3H), 4.42(dd, 1H, J=5.2 Hz, 9.2 Hz).
ESIMS (m/z) : 399.3([M+H]⁺), 797.7([2 M+H]⁺).

### [Synthetic Example 17-1]

### Synthesis of N-hexadecanoyl-L-glutamic acid 5-tert-butyl-1-methyl ester

To L-glutamic acid 5-tert-butyl-1-methyl ester hydrochloride (132 mg, 0.52 mmol) were added N,N-dimethylformamide (5.2 ml), and palmitic acid (200 mg, 0.78 mmol) at room temperature, and the mixture was cooled in an ice bath. Then, 1-ethyl-3-(3-dimethylaminopropyl)carbodiimide hydrochloride (150 mg, 0.78 mmol), 1-hydroxybenzotriazole (105 mg, 0.78 mmol), and triethylamine (0.22 ml, 1.56 mmol) were added, and the mixture was removed from the ice bath and stirred at room temperature for 22 hr. Silica gel was added to the reaction solution to evaporate the solvent, and the residue was purified by silica gel column chromatography (hexane/ethyl acetate) to give N-hexadecanoyl-L-glutamic acid 5-tert-butyl-1-methyl ester (214 mg, 0.47 mmol, yield 90%) as a white solid.
¹H-NMR (400 MHz, CDCl₃)δ: 0.88(t, 3H, J=6.8 Hz), 1.25-1.29(m, 24H), 1.44(s, 9H), 1.63(m, 2H), 1.95(m, 1H), 2.13(m, 1H), 2.21(t, 2H, J=8.0 Hz), 2.31(m, 2H), 3.75(s, 3H), 4.60(dt, 1H, J=5.2 Hz, 8.0 Hz), 6.17(d, 1H, J=7.6 Hz).
ESIMS (m/z) : 456.4([M+H]⁺), 911.8([2 M+H]⁺).

### [Synthetic Example 17-2]

### Synthesis of N-hexadecanoyl-L-glutamic acid 1-methyl ester (SZ82)

To N-hexadecanoyl-L-glutamic acid 5-tert-butyl-1-methyl ester (210 mg, 0.46 mmol) synthesized in Synthetic Example 17-1 were added dichloromethane (4.6 ml), and triisopropylsilane (0.47 ml, 2.3 mmol) at room temperature, and the mixture was cooled in an ice bath. Then, trifluoroacetic acid (4.6 ml) was added, and the mixture was removed from the ice bath and stirred at room temperature for 1.5 hr. The mixture was concentrated under reduced pressure, and the residue was purified by PTLC (hexane/ethyl acetate) to give N-hexadecanoyl-L-glutamic acid 1-methyl ester (117 mg, 0.29 mmol, yield 63%) as a white solid.
¹H-NMR (400 MHz, CD₃OD)δ: 0.90(t, 3H, J=7.0 Hz), 1.29-1.32(m, 24H), 1.61(m, 2H), 1.93(m, 1H), 2.14(m, 1H), 2.24(t, 2H, J=7.4 Hz), 2.39(t, 2H, J=7.4 Hz), 3.72(s, 3H), 4.44(dd, 1H, J=5.2 Hz, 9.2 Hz).
ESIMS(m/z): 400.2([M+H]⁺), 799.6([2 M+H]⁺), 398.1([M-H]⁻), 797.5([2 M-H]⁻).

### [Synthetic Example 18]

### Synthesis of N-acetyl-L-leucine methyl ester (SZ83)

The title compound was obtained in the same manner as in Synthetic Example 14B, except that acetic acid was used instead of hexadecanoic acid.
¹H-NMR (CDCl₃, 400 MHz): δ 6.14 (d, 1H, J=10.0 Hz), 4.67-4.58 (m, 1H), 3.72 (s, 3H), 2.12 (s, 3H), 1.67-1.40 (m, 3H), 0.94-0.91 (m, 6H).
MS: 188.1 [M+H]⁺.

### [Synthetic Example 19]

### Synthesis of N-hexanoyl-L-leucine methyl ester (SZ84)

The title compound was obtained in the same manner as in Synthetic Example 14B, except that hexanoic acid was used instead of hexadecanoic acid.
¹H-NMR (CDCl₃, 300 MHz): δ 5.87 (d, 1H, J=7.8 Hz), 4.63-4.69 (m, 1H), 3.73 (s, 3H), 2.22 (t, 2H, J=5.4 Hz), 1.50-1.69 (m, 5H), 1.25-1.36 (m, 4H), 0.87-0.97 (m, 9H).
MS: 244.2[M+H]⁺.

### [Synthetic Example 20]

### Synthesis of N-(2-octadecyleicosanoyl)-L-leucine methyl ester (SZ86)

To a solution of the L-leucine methyl ester hydrochloride (205 mg, 1.13 mmol), 2-octadecyleicosanoic acid (492 mg, 0.87 mmol), 1-ethyl-3-(3-dimethylaminopropyl) carbodiimide hydrochloride (217 mg, 1.13 mmol) and 1-hydroxybenzotriazole (153 mg, 1.13 mmol), in dichloromethane (20 ml) was added trimethylamine (364 µl, 1.13 mmol). After addition, the mixture was stirred at room temperature overnight. The mixture was poured into dichloromethane (120 mL), washed with water (20 mL), saturated sodium hydrogen carbonate (50 mL), dried over magnesium sulfate and concentrated. The residue was purified by silica gel chromatography column (hexane/ethyl acetate) to N-(2-Octadecyleicosanoyl)-L-leucine methyl ester (SZ86, 4.3 g, yield 90%) as a yellow solid.
¹H-NMR (400 MHz, CDCl₃) : δ 5.77 (d, 1H, J=8.4 Hz), 4.70 (m, 1H), 3.75 (s, 3H), 2.06 (m, 1H), 1.69-1.56 (m, 7H), 1.43-1.28 (m, 64H), 0.97 (dd, 6H, J=1.9 Hz, 6.0 Hz), 0.90 (d, 6H, J=6.6 Hz).

### [Synthetic Example 21]

### Synthesis of N-hexadecanoyl-L-leucine tert-butyl ester (SZ89)

The title compound was obtained in the same manner as in Synthetic Example 14B, except that L-leucine tert-butyl ester was used instead of L-leucine methyl ester hydrochloride.
¹HNMR (400 MHz, CDCl₃): δ 5.82 (d, 1H, J=8.4 Hz), 4.55 (m, 1H), 2.24 (t, 2H, J=8.4 Hz), 1.71-1.59 (m, 4H), 1.53-1.46 (m, 10H), 1.32-1.27 (m, 24H), 0.97 (dd, 6H, J=0.8 Hz, 6.0 Hz), 0.90 (d, 3H, J=7.2 Hz).

### [Synthetic Example 22]

### Synthesis of N-hexadecanoyl-L-leucinamide (SZ90)

The title compound was obtained in the same manner as in Synthetic Example 14B, except that L-leucinamide was used instead of L-leucine methyl ester hydrochloride.
¹H-NMR (CDCl₃, 400 MHz) δ: 6.32(br s, 1H), 5.97 (d, 1H, J=7.8 Hz), 5.48 (br s, 1H), 4.50-4.52 (m, 1H), 2.21 (t, 2H, J=7.6 Hz), 1.52-1.70 (m, 5H), 1.21-1.31 (m, 24H), 0.86-0.96 (m, 9H).
MS: 369.3 [M+H]⁺.

### [Synthetic Example 23]

### Synthesis of N²-hexadecanoyl-N¹,N¹-diethyl-L-leucinamide (SZ92)

To a solution of compound N-hexadecanoyl-L-leucine (Synthetic Example 27-1, 1.5 g, 4.1 mmol), 1-ethyl-3-(3-dimethylaminopropyl) carbodiimide hydrochloride (1.2 g, 6.9 mmol) and 1-hydroxybenzotriazole (820 mg, 6.9 mmol) in N,N-dimethylformamide (50 mL) was added N,N-diisopropylethylamine (2.1 g, 16 mmol). After addition, the mixture was stirred for 15 minutes, diethylamine hydrochloride (660 mg, 6.9 mmol) was added to the mixture, then the mixture was stirred overnight at 25°C. The mixture was poured into water, and the mixture was stirred for 15 minutes. The white solid was afforded by filtration, washed with water, ethyl acetate, dried *in vivo* to give N²-hexadecanoyl-N¹,N¹-diethyl-L-leucinamide (SZ92, 1.7 g, yield 68%) as a white solid.
¹H-NMR (CDCl₃, 300 MHz): δ 6.23 (d, 1H, J=8.7 Hz), 4.96-5.03 (m, 1H), 3.22-3.54 (m, 4 H), 2.22 (t, 2H, J=7.8 Hz), 1.53-1.73 (m, 5H), 1.21-1.31 (m, 27H), 1.12 (t, 3H, J=6.9 Hz) 0.86-0.99 (m, 9H) .
MS: 425.4 [M+H]⁺.

### [Synthetic Example 24]

### Synthesis of N-hexadecanoyl-L-histidine methyl ester (SZ94)

The title compound was obtained in the same manner as in Synthetic Example 14B, except that L-histidine methyl ester was used instead of L-leucine methyl ester hydrochloride.
¹H-NMR (CDCl₃, 400 MHz): δ 7.56 (s, 1H), 7.09 (d, 1H, J=7.2 Hz), 6.80 (s, 1H), 4.78-4.83 (m, 1H), 3.68 (s, 3H), 3.04-3.16 (m, 2H), 2.23 (t, 2H, J=7.6 Hz), 1.58-1.63 (m, 2H), 1.24 (m, 24H), 0.87 (t, 3H, J=6.8 Hz).
MS: 408.3 [M+H]⁺.

### [Synthetic Example 25]

### Synthesis of N-hexadecanoyl-L-proline methyl ester (SZ95)

The title compound was obtained in the same manner as in Synthetic Example 14B, except that L- proline methyl ester was used instead of L-leucine methyl ester hydrochloride.
¹H-NMR (CDCl₃, 300 MHz) : δ 4.38-4.50 (m, 1H), 3.71 (s, 3H), 3.45-3.67 (m, 2H), 2.23-2.36 (m, 2H), 2.07-2.20 (m, 2H), 1.90-2.06 (m, 2H), 1.59-1.68 (m, 2H), 1.24 (m, 24H), 0.87 (t, 3H, J=6.9 Hz).
MS: 368.3 [M+H]⁺.

### [Synthetic Example 26]

### Synthesis of N-hexadecanoyl-L-serine methyl ester (SZ96)

The title compound was obtained in the same manner as in Synthetic Example 14B, except that L- serine methyl ester was used instead of L-leucine methyl ester hydrochloride.
¹H-NMR (CDCl₃, 400 MHz): *δ* 6.39 (d, 1H, J=6.0 Hz), 4.67-4.71 (m, 1H), 3.91-4.00 (m, 2H), 3.80 (s, 3H), 2.54 (br s, 1H), 2.27 (t, 2H, J=7.6 Hz), 1.61-1.68(m, 2H), 1.24 (s, 24H), 0.88 (t, 3H, J=6.4 Hz).
MS: 358.3 [M+H]⁺.

### [Synthetic Example 27-1]

### Synthesis of N-hexadecanoyl-L-leucine

To a solution of N-hexadecanoyl-L-leucine methyl ester (Synthetic Example 14B, 4.5 g, 12 mmol) in methanol (50 mL)/dichloromethane (5 mL) was added aqueous 1M lithium hydroxide solution (15 ml, 15 mmol) dropwise. After addition, the mixture was stirred for 3 hours at room temperature. The mixture was concentrated, the residue was dissolved in water, extracted with ethyl acetate twice. The aqueous phase was acidified with concentrated hydrochloric acid into pH 1-2, the precipitate was stirred for 15 minutes, the white solid was afforded by filtration, washed with water, dried *in vacuo* to give compound N-hexadecanoyl-L-leucine (3.6 g, yield 84%) as a white solid,
¹H-NMR (CDCl₃, 400 MHz) : δ 10.44 (br s, 1H), 6.09 (d, 1H, J=7.6 Hz), 4.61-4.63 (m, 1H), 2.24 (t, 2H, J=6.8 Hz), 1.56-1.73 (m, 5H), 1.20-1.30 (m, 24H), 0.86-0.96 (m, 9H). MS: 370.3 [M+H]⁺.

### [Synthetic Example 27-2]

### Synthesis of N-hexadecanoyl-L-leucine hexyl ester (SZ97)

To a solution of N-hexadecanoyl-L-leucine (3.0 g, 8.1 mmol), 1-ethyl-3-(3-dimethylaminopropyl) carbodiimide hydrochloride (2.3 g, 12 mmol) in N,N-dimethylformamide (50 mL) was added (98 mg, 0.80 mmol). After addition, the mixture was stirred for 15 minutes. Hexanol (1.2 g, 12 mmol) was added to the mixture, then the mixture was stirred overnight at 25°C. The mixture was poured into water, extracted with dichloromethane, and the organic phase was washed with water, brine, dried over sodium sulfate, concentrated. The residue was purified by silica gel column chromatography (petroleum ether:ethyl acetate=4:1-2:1, *v:v*) to provide N-hexadecanoyl-L-leucine hexyl ester (SZ97, 350 mg, yield 10%) as a colorless oil.
¹H-NMR (CDCl₃, 300 MHz): *δ* 5.87 (d, 1H, J=8.7 Hz), 4.63-4.66 (m, 1H), 4.12 (t, 2H, J=6.6 Hz), 2.21 (t, 2H, J=7.8 Hz), 1.52-1.71 (m, 7H), 1.22-1.36 (m, 30H), 0.84-0.97 (m, 12H).
MS: 454.4 [M+H]⁺.

### [Synthetic Example 28-1]

### Synthesis of Nω-nitro-L-arginine hexyl ester

Nω-nitro-L-arginine (4.0 g, 18 mmol) was dissolved in saturated hydrogen chloride/hexanol (60 mL). The mixture was heated at 100°C for 1 hour. The mixture was concentrated, and the residue was sonicated and scratched with spatula for 15 minutes in methanol (20 mL)/diethyl ether (60 mL). The white solid was afforded by filtration, washed with diethyl ether, dried by reduced pressure to provide Nω-nitro-L-arginine hexyl ester (4.5 g, yield 89%) as a white solid.
MS: 304.1 [M+H]⁺.

### [Synthetic Example 28-2]

### Synthesis of Nω-nitro-N²-hexadecanoyl-L-arginine hexyl ester

To a solution of hexadecanoic acid (2.0 g, 7.9 mmol), 1-ethyl-3-(3-dimethylaminopropyl)carbodiimide hydrochloride (2.3 g, 12 mmol) and 1-hydroxybenzotriazole (1.6 g, 12 mmol) in N,N-dimethylformamide (60 mL) was added N,N-diisopropylethylamine (4.1 g, 32 mmol). After addition, the mixture was stirred for 15 minutes, Nω-nitro-L-arginine hexyl ester (3.6 g, 12 mmol) was added to the mixture. The mixture was stirred overnight at 25°C. The mixture was poured into water. The white solid was afforded by filtration, washed with water, ethyl acetate, dried *in vivo* to give Nω-nitro-N²-hexadecanoyl-L-arginine hexyl ester (2.4 g, yield 61%) as a white solid.
¹H-NMR (CDCl₃, 400 MHz): *δ* 8.79 (br s, 1H), 7.81 (br s, 2H), 6.37 (d, 1H, J=3.2 Hz), 4.62 (m, 1H), 4.13-4.19 (m, 2H), 3.68 (br s, 1H), 3.30 (br s, 1H), 2.27(t, 2H, J=6.4 Hz), 1.55-1.95 (m, 10H), 1.18-1.31 (m, 30H), 0.86-0.92 (m, 6H).

### [Synthetic Example 28-3]

### Synthesis of N²-hexadecanoyl-L-arginine hexyl ester hydrochloride salt (SZ99)

To a solution of Nω-nitro-N²-hexadecanoyl-L-arginine hexyl ester (2.4 g, 4.7 mmol) in methanol (50 mL) were added 4-5 drops concentrated hydrochloric acid and Palladium/carbon (240 mg). After addition, the mixture was stirred under hydrogene (50 psi) at 50°C overnight. Palladium/carbon was removed by filtration, and the filtrate was concentrated to give N²-Hexadecanoyl-L-arginine hexyl ester hydrochloride salt (SZ99, 2.1 g, yield 91%) as a white solid.
¹H-NMR (CD₃OD, 300 MHz) : *δ* 4.37-4.42 (m, 1H), 4.13 (t, 2H, J=6.3 Hz), 3.22 (t, 2H, J=7.2 Hz), 2.27 (t, 2H, J=7.2 Hz), 1.58-1.92(m, 8H), 1.21-1.41 (m, 30H), 0.89-0.95 (m, 6 H).
MS: 497.4 [M+H]⁺.

### [Synthetic Example 29]

### Synthesis of N²-docosanoyl-N¹,N¹-diethyl-L-glutamic acid 1-amide (SZ106)

The title compound was obtained in the same manner as in Synthetic Examples 39-1 to 39-5, except that diethylamine was used instead of hexanol.
¹H-NMR (CD₃OD, 400 MHz): *δ* 4.87(m, 1H), 3.56-3.63 (m, 1H), 3.43-3.51 (m, 2H), 3.24-3.30 (m, 1H, J=7.2 Hz), 2.38 (dt, 2H, J=1.6 Hz, 6.0 Hz), 2.21-2.25 (dt, 2H, J=2.0 Hz, 6.0 Hz), 1.93-2.00 (m, 1H), 1.79-1.86 (m, 1H), 1.60 (t, 2 H, J=6.8 Hz), 1.24-1.28 (m, 39H), 1.11 (t, 3H, J=6.8 Hz), 1.09 (t, 3H, J=6.4 Hz).
MS: 525.4 [M+H]⁺.

### [Synthetic Example 30]

### Synthesis of N-docosanoylagmatine hydrochloride salt (SZ108)

To a solution of docosanoic acid (2.7 g, 8.0 mmol) in dichloromethane (15 mL) was added N,N-dimethylformamide (5 drops) followed by oxalyl chloride (1.2 mL). After stirring at room temperature for 20 minutes, the reaction mixture was concentrated and dissolved in tetrahydrofuran (5 mL). A mixture of agmatine sulfate salt (2.7 g, 12 mmol), 2-propanol (15 mL) and water (20 mL) was added 27% aqueous sodium hydroxide to adjust pH 11. Then the above tetrahydrofuran solution was added to the solution of agmatine sulfate salt dropwise with adding 27% aqueous sodium hydroxide to keep pH=11. The mixture was stirred at room temperature for 2 hours. The formed solid was filtered and dissolved in 30 mL of tetrahydrofuran. To the solution was added hydrogen chloride/tetrahydrofuran (1 mol/L, 30 mL) dropwise. After stirring for 30 minutes at room temperature, the mixture was concentrated to give N-docosanoylagmatine hydrochloride salt (SZ108, 2.94 g, yield 66%) as white solid.
¹H-NMR (CD₃OD, 400 MHz): *δ* 3.31-3.30 (m, 2H), 3.20 (t, 2H, J=6.4 Hz), 2.29-2.25 (m, 1H), 2.18-2.20 (m, 1H), 1.61-1.58 (m, 4H), 1.29 (s, 40H), 0.89 (t, 3H, J=6.4 Hz).
MS: 453.3 [M+H]⁺

### [Synthetic Example 31-1]

### Synthesis of Nω-nitro-N²-tert-butoxycarbonyl-N¹,N¹-diethyl-L-arginine amide

A solution of Nω-nitro-N²-*tert*-butoxycarbonyl-L-arginine (10.0 g, 31.4 mmol) and triethylamine (3.48 g, 34.5 mmol) in anhydrous tetrahydrofuran (130 mL) was cooled to -25 °C and ethylchloroformate (3.76 g, 34.5 mmol) was added. After stirring for 20 minutes, a solution of diethylamine (2.75 g, 37.6 mmol) in anhydrous tetrahydrofuran (20 mL) was added. The mixture was maintained at -25°C for 20 minutes and warmed to room temperature for 18 hours. The mixture was concentrated and diluted with ethyl acetate (300 mL), washed with 10% aqueous citric acid (150 mL), saturated sodium hydrogen carbonate (150 mL) and brine (150 mL). The organic layer was dried over sodium sulfate, filtered and concentrated to give Nω-nitro-N²-*tert-*butoxycarbonyl-N¹,N¹-diethyl-L-arginine amide (9.12 g, yield 78%) as yellow oil.
¹H-NMR (CDCl₃, 300 MHz) *δ*: 4.55-4.52 (m, 1H), 3.61-3.51 (m, 2H), 3.35-3.20 (m, 4H), 1.78-1.69 (m, 4H), 1.44 (s, 9H), 1.23-1.27 (m, 3H), 1.10-1.15 (m, 3H).

### [Synthetic Example 31-2]

### Synthesis of Nω-nitro-N²-hexadecanoyl-N¹,N¹-diethyl-L-arginine amide

A solution of Nω-nitro-N²-*tert*-butoxycarbony-N¹,N¹-diethyl-L-arginine amide (9.12 g, 24.4 mmol) in hydrogen chloride/methanol (120 mL) was stirred at room temperature for 1 hour. The mixture was concentrated to give the Nω-nitro-N¹,N¹-diethyl-L-arginine amide hydrochloride salt (10.5 g, yield 100%) as a yellow solid.
¹H-NMR (CD₃OD, 400 MHz) : *δ* 4.41 (t, 1H, J=6.0 Hz), 3.61-3.47 (m, 2H), 3.41-3.26 (m, 4H), 1.91 (br s, 2H), 1.75 (br s, 2H), 1.25 (t, 3H, J=7.2 Hz), 1.15 (t, 3H, J=7.2 Hz).

### [Synthetic Example 31-3]

### Synthesis of Nω-nitro-N²-hexadecanoyl-N¹,N¹-diethyl-L-arginine amide

To a solution of the compound hexadecanoic acid (2.8 g, 11 mmol), 1-ethyl-3-(3-dimethylaminopropyl)carbodiimide hydrochloride (4.5 g, 24 mmol), 1-hydroxybenzotriazole (3.2 g, 24 mmol) in N,N-dimethylformamide (100 mL) was added triethylamine (4.8 g, 47 mmol). After addition, the mixture was stirred at room temperature for 15 minutes. Nω-nitro-N¹,N¹-diethyl-L-arginine amide hydrochloride salt (5.0 g, 16 mmol) was added to the mixture. Then the mixture was stirred at room temperature for 6 hours. The mixture was diluted with water (200 mL), extracted with ethyl acetate (200 mL×3). The organic layers were combined and washed with brine (200 mL), dried over sodium sulfate, filtered and concentrated. The residue was purified by column chromatography on silica gel (dichloromethane/methanol=50/1) to give Nω-nitro-N²-hexadecanoyl-N¹,N¹-diethyl-L-arginine amide (3.6 g, yield 44%) as colorless oil.
¹H-NMR (CDCl₃, 300 MHz) : *δ* 7.94 (br s, 1H), 6.84-6.82 (m, 1H), 4.90-4.85 (m, 1H), 3.79-3.49 (m, 2H), 3.36-3.21 (m, 4H), 2.27 (t, 2H, J=7.5 Hz), 1.66-1.60 (m, 4H), 1.58-1.20 (m, 29H), 1.13 (t, 3H, J=6.9 Hz), 0.89 (t, 3H, J=6.9 Hz).

### [Synthetic Example 31-4]

### Synthesis of N²-hexadecanoyl-N¹,N¹-diethyl-L-arginine amide hydrochloride salt (SZ110)

To a solution of Nω-nitro-N²-hexadecanoyl-N¹,N¹-diethyl-L-arginine amide (3.6 g, 6.9 mmol) in methanol (50 mL) were added concentrated hydrochloric acid (0.5 mL) and Palladium/carbon (10%, 1.0 g). The mixture was stirred at 50 °C overnight under hydrogen (50 psi). The mixture was filtered and the filtrate was concentrated to give N²-Hexadecanoyl-N¹,N¹-diethyl-L-arginine amide hydrochloride salt (SZ110, 3.5 g, yield 100%) as a yellow solid.
¹H-NMR (CD₃OD, 400 MHz): *δ* 4.80 (dd, 1H, J=4.8 Hz, 8.0 Hz), 3.50-3.43 (m, 4H), 3.23-3.19 (m, 2H), 2.25 (d, 2H, J=7.6 Hz), 1.75-1.60 (m, 6H), 1.35-1.31 (m, 27H), 1.11 (t, 3H, J=7.2 Hz), 0.90 (t, 3H, J=7.2 Hz).
MS: 468.2 [M+H]⁺.

### [Synthetic Example 32]

### Synthesis of N²-docosanoyl-L-arginine hexyl ester hydrochloride salt (SZ121)

The title compound was obtained in the same manner as in Synthetic Examples 28-1 to 28-3, except that docosanoic acid was used instead of hexadecanoic acid.
¹H-NMR (CD₃OD, 400 MHz): *δ* 4.42-4.39 (m, 1H), 4.13 (t, 2H, J=6.8 Hz), 3.23-3.19 (m, 2H), 2.26-2.22 (m, 2H), 1.93-1.86 (m, 1H), 1.77-1.61 (m, 7H), 1.39-1.32 (m, 42H), 0.92-0.87 (m, 6H). MS: 581.3 [M+H]⁺.

### [Synthetic Example 33]

### Synthesis of N-docosanoyl-L-leucine hexyl ester (SZ124)

The title compound was obtained in the same manner as in Synthetic Examples 27-1 to 27-2, except that docosanoic acid was used instead of hexadecanoic acid.
¹H-NMR (400 MHz, CDCl₃) : *δ* 5.82 (d, J=6.8 Hz, 1H), 4.66-4.61 (m, 1H), 4.13 (t, 2H, J=6.8 Hz), 2.22 (t, 2H, J=7.6 Hz), 1.68-1.60 (m, 7H), 1.56-1.49 (m, 1H), 1.38-1.28 (m, 41H), 0.95-0.86 (m, 12H) .
MS: 538.3 [M+H]⁺.

### [Synthetic Example 34]

### Synthesis of N²-docosanoyl-N¹,N¹-diethyl-L-leucinamide (SZ125)

The title compound was obtained in the same manner as in Synthetic Example 23, except that docosanoic acid was used instead of hexadecanoic acid.
¹H-NMR (400 MHz, CDCl₃): *δ* 6.20 (d, 1H, J=8.8 Hz), 5.01-4.95 (m, 1H), 3.54-3.47 (m, 1H), 3.42-3.36 (m, 2H), 3.30-3.20 (m, 1H), 2.19 (t, 2H, J=7.6 Hz), 1.68-1.54 (m, 4H), 1.40-1.34 (m, 1H), 1.26 (s, 38H), 1.11 (t, 3H, J=7.2 Hz), 0.99 (d, 3H, J=6.0 Hz), 0.93-0.86 (m, 6H).
MS: 509.3 [M+H]⁺.

### [Synthetic Example 35]

### Synthesis of N²-docosanoyl-L-arginine amide hydrochloride salt (SZ128)

The title compound was obtained in the same manner as in Synthetic Examples 31-1 to 31-4, except that diethylamine and docosanoic acid were used instead of ammonium chloride and hexadecanoic acid respectively.
¹H-NMR (CD₃OD, 400 MHz): *δ* 4.37 (dd, 1H, J=5.2 Hz, 8.0 Hz), 3.2-3.19 (m, 2H), 2.26 (t, 2H, J=7.2 Hz), 1.90-1.83 (m, 1H), 1.72-1.59 (m, 5H), 1.32-1.20 (m, 36H), 0.90 (t, 3H, J=7.2 Hz).
MS: 496.3 [M+H]⁺.

### [Synthetic Example 36]

### Synthesis of N²-docosanoyl-N¹,N¹-diethyl-L-arginine amide hydrochloride salt (SZ129)

The title compound was obtained in the same manner as in Synthetic Examples 31-1 to 31-4, except that docosanoic acid was used instead of hexadecanoic acid.
¹H-NMR (DMSO-*d₆*, 400 MHz): *δ* 8.05 (d, 1H, J=8.2 Hz), 7.78-7.76 (m, 1H), 7.31-7.26 (m, 4H), 4.64 (dd, 1H, J=5.2 Hz, 8.0 Hz), 3.41-3.32 (m, 3H), 3.20-3.06 (m, 3H), 2.10 (t, 2H, J=7.2 Hz), 1.63-1.38 (m, 6H), 1.32-1.20 (m, 36H), 1.13 (t, 3H, J=7.2 Hz), 1.00 (t, 3H, J=7.2 Hz), 0.85 (t, 3H, J=6.8 Hz).
MS: 552.3 [M+H]⁺.

### [Synthetic Example 37]

### Synthesis of N²-hexanoyl-L-arginine hexyl ester hydrochloride salt (SZ130)

The title compound was obtained in the same manner as in Synthetic Examples 28-1 to 28-3, except that hexanoic acid was used instead of hexadecanoic acid.
¹H-NMR (CD₃OD, 400 MHz): *δ* 4.41-4.38 (m, 1H), 4.13 (t, 2H, J=6.0 Hz), 3.23-3.19 (m, 2H), 2.26 (t, 2H, J=6.8 Hz), 1.95-1.86 (m, 1H), 1.78-1.60 (m, 7H), 1.38-1.30 (m, 10H), 0.93-0.89 (m, 6H) .
MS: 357.1 [M+H]⁺.

### [Synthetic Example 38]

### Synthesis of N-hexadecanoyl-L-glutamic acid hexyl ester (SZ134)

The title compound was obtained in the same manner as in Synthetic Examples 39-1 to 39-5, except that hexadecanoic acid was used instead of docosanoic acid.
¹H-NMR (CDCl₃, 400MHz): *δ* 6.22 (d, J=7.6 Hz, 1H), 4.66-4.63 (m, 1H), 4.14 (d, J=6.0 Hz, 2H), 2.46-2.41 (m, 2H), 2.25-2.21 (m, 3H), 1.97-1.91 (m, 1H), 1.68-1.61 (m, 4H), 1.38-1.29 (m, 31H), 0.91-0.86 (m, 6H).
MS: 470.2 [M+H]⁺.

### [Synthetic Example 39-1]

### Synthesis of N-benzyloxycarbonyl-5-tert-butyl-L-glutamate

To a solution of 5-*tert*-butyl-L-glutamate (15 g, 76 mmol) in 110 mL of water and 50 mL of dioxane was added sodium carbonate (8.0 g, 76 mmol) followed by benzyl chloroformate (13 g, 76 mmol) in 60 mL of dioxane at 0°C for 3 hours. After stirring at room temperature overnight, the mixture was extracted with ethyl acetate (50 mL×2). The aqueous layer was acidified to pH 2 with 6N hydrochloric acid and extracted with ethyl acetate (50 mL×3). The organic layer was washed with brine and dried over sodium sulfate, filtered and concentrated to give N-benzyloxycarbonyl-5-tert-butyl-L-glutamate (12 g, yield 47%) as yellow oil.
¹H-NMR (CDCl₃, 300MHz): *δ* 7.35 (br s, 5H), 5.76 (d, 1H, J=7.2 Hz), 5.12 (s, 2H), 4.41-4.38 (m, 1H), 2.43-2.35 (m, 2H), 2.24-2.11 (m, 1H), 2.03-1.96 (m, 1H) 1.44 (s, 9H).

### [Synthetic Example 39-2]

### Synthesis of N-benzyloxycarbonyl-5-tert-butyl-L-glutamate hexyl ester

To a solution of compound N-benzyloxycarbonyl-5-*tert-*butyl-L-glutamate (2.0 g, 6 mmol), triethylamine (0.67 g, 6.6 mmol) and 30 mL of dry tetrahydrofuran was added ethyl chloroformate (0.72 g, 6.6 mmol) at -25°C. After stirring for 30 minutes at this temperature, hexanol (0.92 g, 9.0 mmol) was added to the reaction. The mixture was stirred for 18 hours at room temperature. The mixture was diluted with ethyl acetate (100 mL) washed with water, dried over sodium sulfate, filtered and concentrated. The residue was purified by silica gel chromatography (Petroleum ether/ethyl acetate=5/1) to afford N-benzyloxycarbonyl-5-tert-butyl-L-glutamate hexyl ester (1.4 g, 56%) as yellow oil.
¹H-NMR (CDCl₃, 400 MHz): *δ* 7.35-7.30 (m, 5H), 5.44 (d, 1H, J=3.6 Hz), 5.10 (s, 2H), 4.41-4.37 (m, 1H), 4.14-4.11 (m, 2H), 2.43-2.27 (m, 2H), 2.21-2.13 (m, 1H), 1.86-1.80 (m, 1H), 1.64-1.50 (m, 2H), 1.42 (s, 9H), 1.30-1.25 (m, 6H), 0.90-0.83 (m, 3H) .

### [Synthetic Example 39-3]

### Synthesis of 5-tert-butyl-L-glutamate hexyl ester

To a mixture of compound N-benzyloxycarbonyl-5-tert-butyl-L-glutamate hexyl ester (12 g, 27 mmol) was added Palladium/carbon (1.2 g) in 150 mL of ethyl acetate. The mixture was stirred for 4 hours at room temperature under hydrogen atomsphere (50 psi). The suspension was filtered and filtrated was concentrated to afford 5-tert-butyl-L-glutamate hexyl ester (6.8 g, yield 86%) as yellow oil.
¹H-NMR (CDCl₃, 400 MHz): *δ* 4.14-4.03 (m, 2H), 3.47-3.43 (m, 1H), 2.36-2.34 (m, 2H), 2.07-1.97 (m, 1H), 1.84-1.75 (m, 1H), 1.67-1.60 (m, 4H), 1.44 (s, 9H), 1.39-1.23 (m, 6H), 0.89 (t, 3H, J=7.2 Hz).

### [Synthetic Example 39-4]

### Synthesis of N-docosanoyl-5-tert-butyl-L-glutamate hexyl ester

To a mixture of docosanoic acid in 30 mL of dichloromethane were added 5 drops of N,N-dimethylformamide followed by oxalyl chlororide (0.89 g, 7.0 mmol). The mixture was stirred at room temperature for 1 hour. The mixture was concentrated and the residue was dissolved in 10 mL of tetrahydrofuran. The obtained solution was added to a solution 5-tert-butyl-L-glutamate hexyl ester (2.0 g, 5.9 mmol) and triethylamine (2.6 g, 2.6 mmol) in 30 mL of tetrahydrofuran at 0°C. The resultant mixture was stirred at room temperature for 2 hours. The mixture was diluted with ethyl acetate (100 mL), washed with 50 mL of water, 50 mL of brine, dried over sodium sulfate, filtered and concentrated. The residue was purified by silica gel chromatography (petroleum ether/ethyl acetate=5/1) to afford N-docosanoyl-5-tert-butyl-L-glutamate hexyl ester (3.0 g, yield 86%) as a white solid.
¹H-NMR (CDCl₃, 400MHz): *δ* 6.18 (d, 1H, J=7.6 Hz), 4.60-4.57 (m, 1H), 4.12 (t, 2H, J=6.8 Hz), 2.30-2.10 (m, 6H), 2.04-1.91 (m, 1H), 1.65-1.60 (m, 4H), 1.44 (s, 9H), 1.36-1.27 (m, 41H), 0.90-0.86 (m, 6H).

### [Synthetic Example 39-5]

### Synthesis of N-docosanoyl-L-glutamic acid hexyl ester (SZ135)

To a solution of N-docosanoyl-5-*tert*-butyl-L-glutamate hexyl ester (1.5 g, 2.5 mmol) in 15 mL of dichloromethane, was added 15 mL of trifluoroacetic acid under ice-water bath. The mixture was stirred for 2 hours at room temperature. The reaction mixture was diluted with 100 mL of dichloromethane, washed with water (30 mL×2), brine (30 mL), dried over sodium sulfate, filtered and concentrated. The residue was triturated in 10 mL of methanol and filtered. The solid was dried to afford N-docosanoyl-L-glutamic acid hexyl ester (SZ135 1.0 g, yield 81%) as a white solid.
¹H-NMR (CDCl₃, 400MHz): *δ* 6.24 (d, 1H, J=7.6 Hz), 4.70-4.58 (m, 1H), 4.14 (t, 2H, J=6.8 Hz), 2.54-2.38 (m, 2H), 2.28-2.23 (m, 3H), 2.01-1.84 (m, 1H), 1.62-1.40 (m, 4H), 1.38-1.36 (m, 43H), 0.90-0.82 (m, 6H).

### [Synthetic Example 40]

### Synthesis of N²-hexadecanoyl-L-glutamine methyl ester (SZ136)

The title compound was obtained in the same manner as in Synthetic Example 4, except that L-glutamine methyl ester hydrochloride and hexadecanoic acid were used instead of L-glutamine tert-butyl ester hydrochloride and stearic acid respectively.
^{1l}H-NMR (CDCl₃, 400 MHz) : *δ* 6.44 (d, 1H, J = 7.6 Hz), 6.31 (brs, 1H), 5.39 (brs, 1H), 4.63 (m, 1H), 3.78 (s, 3H), 2.38-2.20 (m, 5H), 2.01-1.95 (m, 1H), 1.62 (m, 2H), 1.28 (m, 24H), 0.90 (t, 3H, J = 6.8 Hz).
ESIMS: 366.3 [M+H]⁺.

### (2) Adjuvant activity test

### [Example 1] Evaluation test of adjuvant activity (IgG1 production amount) of N-hexadecanoylagmatine (SZ62)

8-Week-old female BALB/c mice were divided into groups (N=6), and each group was primarily immunized by dorsal-subcutaneous administration of (1) ovalbumin (OVA) (10 µg) dissolved in saline as an antigen (OVA single administration group), (2) OVA 10 µg and aluminum hydroxide gel adjuvant (2.56 µmol, 0.2 mg) dissolved in saline (Alum administration group, positive control) (3) N-hexadecanoylagmatine (SZ62) (0.256 µmol) dissolved in saline added with OVA 10 µg and 5% α-cyclodextrin (hereinafter to be simply referred to as αCD) (SZ62 administration group), each per mouse. One week later, the secondary immunization was carried out by dorsal-subcutaneous administration of solutions similar to those of the above-mentioned (1) - (3) again. Two weeks from the secondary immunization, the whole blood was extracted under anesthesia, and the obtained serum sample was measured for anti-OVA-specific IgG1 subclass antibody.

### [Significance of IgG1 subclass antibody, IgG2a subclass antibody measurements]

Helper T cell, which is one of the immunocompetent cells, includes several types and induces different immunofunction depending on the type thereof. In general, a cell called Th1 cell is considered to be involved in the induction of cellular immunity, finds, for example, virus infected cells and the like, and induces a function of killer T cells to directly attack them and the like. On the other hand, Th2 cell is considered to be involved in the induction of humoral immunity and induces, for example, IgE production from B cells. Th1 cell and Th2 cell are considered to be in a relationship where an increase in one of them suppresses the other. For example, it is considered that the onset of allergy can be suppressed by setting the Th1/Th2 balance to be Th1 dominant. IgG antibody includes some subclasses and, in the case of mouse, IgG1 subclass antibody is considered to be involved in the immunofunction of Th2 type, and IgG2a subclass antibody is considered to be involved in the immunofunction of Th1 type.
It is said that whether the induced immunity is Th1 type or Th2 type can be known to a certain extent by measuring production of these subclass antibodies.

A method for measurement of the anti-OVA-specific IgG1 subclass antibody in Example 1, and the following Examples 5, 7 and 8 is as follows.

### [Measurement method of anti-OVA-specific IgG1 subclass antibody]

To a 96 well plate was added 5 µg/ml OVA at 100 µl/well, and the mixture was incubated at 4°C overnight. After incubation, the mixture was washed three times with 200 µl of PBS-T (PBS+0.05%(v/v) Tween 20) per well, and blocked with 100 µl of 5% FCS/PBS solution per well at room temperature for 1 - 2 hr. Thereafter, the mixture was washed three times with PBS-T (200 µl/well), a diluted serum sample (100 µl/well) or the same amount of 5% FCS/PBS solution as a control was added, and the mixture was incubated at 37°C for 1 hr. Thereafter, the mixture was washed five times with PBS-T (200 µl/well), a diluted biotinylated anti-mouse IgG1 antibody (100 µl/well) was added, and the mixture was incubated at 37°C for 45 min. Thereafter, the mixture was washed five times with PBS-T (200 µl/well), a diluted anti-biotin-HRP antibody was added (100 µl/well), and the mixture was incubated at 37°C for another 30 min. Thereafter, the mixture was washed five times with PBS-T (200 µl/well), TMB substrate solution was added (100 µl/well), and the mixture was incubated at room temperature for 10 - 15 min. Thereafter, a reaction quenching liquid (2N sulfuric acid solution) was added (50 µl/well) to discontinue a color developing reaction, and the absorbance (OD Value=450 nm) was measured by a microplate reader.

The results are shown in Fig. 1.

As is clear from the results shown in Fig. 1, anti-OVA-specific IgG1 subclass antibody production significantly increased in the Alum administration group (Alum in Figure) and the SZ62 administration group (SZ62 in Figure) as compared to the OVA single administration group (Saline in Figure). The result has confirmed that N-hexadecanoylagmatine (SZ62) has an adjuvant activity equivalent to that of Alum.

### [Example 2] Evaluation test of adjuvant activity (IgG2a production amount) of N-hexadecanoylagmatine (SZ62)

The serum sample obtained in Example 1 was measured for anti-OVA-specific IgG2a subclass antibody.

### [Measurement method of anti-OVA-specific IgG2a subclass antibody]

To a 96 well plate was added 5 µg/ml OVA at 100 µl/well, and the mixture was incubated at 4°C overnight. After incubation, the mixture was washed three times with 200 µl of PBS-T (PBS+0.05%(v/v) Tween 20) per well, and blocked with 100 µl of 5% FCS/PBS solution per well at room temperature for 1 - 2 hr. Thereafter, the mixture was washed three times with PBS-T (200 µl/well), a diluted serum sample (100 µl/well) or the same amount of 5% FCS/PBS solution as a control was added, and the mixture was incubated at 37°C for 1 hr. Thereafter, the mixture was washed five times with PBS-T (200 µl/well), a diluted biotinylated anti-mouse IgG2a antibody (100 µl/well) was added, and the mixture was incubated at 37°C for 45 min. Thereafter, the mixture was washed five times with PBS-T (200 µl/well), a diluted anti-biotin-HRP antibody was added (100 µl/well), and the mixture was incubated at 37°C for another 30 min. Thereafter, the mixture was washed five times with PBS-T (200 µl/well), TMB substrate solution was added (100 µl/well), and the mixture was incubated at room temperature for 10 - 15 min. Thereafter, a reaction quenching liquid (2N sulfuric acid solution) was added (50 µl/well) to discontinue a color developing reaction, and the absorbance (OD Value=450 nm) was measured by a microplate reader.

The results are shown in Fig. 2.

As is clear from the results shown in Fig. 2, anti-OVA-specific IgG2a subclass antibody production significantly increased in the Alum administration group (Alum in Figure) and the SZ62 administration group (SZ62 in Figure) as compared to the OVA single administration group (Saline in Figure). The result has confirmed that N-hexadecanoylagmatine (SZ62) has an adjuvant activity equivalent to that of Alum.

### [Example 3] Evaluation test of allergy inducing activity of N-hexadecanoylagmatine (SZ62)

The serum sample obtained in Example 1 was measured for an anti-OVA-specific IgE antibody. The measurement method of the anti-OVA-specific IgE antibody in Example 3 and Examples 6, 9 is as follows.

### [Measurement method of anti-OVA-specific IgE antibody]

DS mouse IgE ELISA (OVA) kit manufactured by DS Pharma Biomedical Co., Ltd. was used. The protocol is briefly shown below.

The serum sample and a standard reagent for drawing an analytical curve are diluted with a buffer and, after stirring, left standing at room temperature for 10 min. The sample and the standard solution are added to a plate bound with an IgE capture antibody in advance and, after stirring, the mixture is left standing at room temperature for 60 min. The mixture is washed three times with wash, HRP-labeled OVA is added, and the mixture is left standing at room temperature for 30 min. The mixture is washed three times with wash, a substrate solution is added, and the mixture is left standing in dark at room temperature for 30 min. A reaction quenching liquid is added, the mixture is stirred, and the absorbance (OD Value=450 nm) is immediately measured.

The results are shown in Fig. 3.

As is clear from the results shown in Fig. 3, production of anti-OVA-specific IgE antibody significantly increased in the ALUM administration group (Alum in Figure) as compared to that of the OVA single administration group (Saline in Figure) as previously reported. In contrast, an increase in the production of anti-OVA-specific IgE antibody was not observed in the SZ62 administration group (SZ62 in Figure). The results have clarified that N-hexadecanoylagmatine (SZ62) shows a lower allergy inducing activity as compared to aluminum hydroxide gel adjuvant that problematically induces allergy by inoculation.

### (3) Evaluation test as transnasal influenza vaccine adjuvant

### [Example 4] Evaluation test of transnasal influenza vaccine adjuvant activity of N-hexadecanoylagmatine (SZ62)

8-Week-old female BALB/c mice were divided into groups (N=6), and each group was primarily immunized by transnasal administration of (1) influenza vaccine (Influenza HA Vaccine "SEIKEN", manufactured by Denka Seiken Co. Ltd.) (20 µl) diluted with saline (influenza vaccine single administration group) as an antigen, (2) influenza vaccine (20 µl) and Poly(I:C) (5 µg) as positive control adjuvant, diluted with saline (Poly(I:C) administration group), (3) influenza vaccine (20 µl) and N-hexadecanoylagmatine (SZ62) (0.2 µg) as an adjuvant diluted with saline (SZ62 (0.2 µg) administration group), (4) influenza vaccine (20 µl) and N-hexadecanoylagmatine (SZ62) (1.00 µg) as an adjuvant diluted with saline (SZ62 (1 µg) administration group), each per mouse. Two weeks later, the secondary immunization was carried out by transnasal administration of solutions similar to those of the above-mentioned (1) - (4) again. Two weeks from the secondary immunization, nasal cavity washing was performed. As a nasal cavity wash, thorax of mouse was opened to expose trachea, the trachea was incised, an Atom venous catheter with clause was inserted and 1 mL of saline was injected. The liquid discharged from the nose was centrifuged at 10000 g for 3 min, and the supernatant was recovered and used as a nasal cavity wash sample. The nasal cavity wash sample was subjected to the measurement of anti-influenza IgA antibody. The measurement method was as follows.

### [Measurement method of anti-influenza IgA antibody]

0.5 µg/ml influenza vaccine (Influenza HA Vaccine "SEIKEN", manufactured by Denka Seiken Co. Ltd.) was added to a 96 well plate by 100 µl per well, and the mixture was incubated at 4°C overnight. After incubation, the mixture was washed three times with 200 µl of PBS-T (PBS+0.05% (v/v) Tween 20) per well, and blocked with 100 µl of 5% FCS/PBS solution per well at room temperature for 1 - 2 hr. Thereafter, the mixture was washed three times with PBS-T (200 µl/well), a diluted serum sample (100 µl/well), nasal cavity wash sample, and the same amount of 5% FCS/PBS solution as a control was added, and the mixture was incubated at 37°C for 1 hr. Then, the mixture was washed five times with PBS-T (200 µl/well), a diluted biotinylated anti-mouse IgA antibody was added, and the mixture was incubated at 37°C for 45 min. Then, the mixture was washed five times with PBS-T (200 µl/well), a diluted anti-biotin-HRP antibody was added (100 µl/well), and the mixture was incubated at 37°C for another 30 min. Thereafter, the mixture was washed five times with PBS-T (200 µl/well), TMB substrate solution was added (100 µl/well), and the mixture was reacted at room temperature for 10 - 15 min. Thereafter, a reaction quenching liquid (2N sulfuric acid solution) was added (50 µl/well) to discontinue a color developing reaction, and the absorbance (OD Value=450 nm) was measured by a microplate reader.

The evaluation results of IgA antibody production in nasal cavity wash are shown in Fig. 4.

As is clear from the results shown in Fig. 4, production of anti-influenza IgA antibody in the nasal cavity wash of the SZ62 (1 µg) administration group (1 µg in Figure) significantly increased as compared to that of the influenza vaccine single administration group (Saline in Figure). On the other hand, IgA antibody production of Poly(I:C) administration group (Poly(I:C) in Figure) used as a positive control adjuvant increased as compared to that of the influenza vaccine single administration group; however, a significant increase was not found. The results confirm that N-hexadecanoylagmatine (SZ62) is an adjuvant that induces IgA to influenza vaccine on the nasal mucosa of the administration site.

### [Example 5] Evaluation test of adjuvant activity of N²-hexadecanoyl-L-arginine (SZ61), N-hexadecanoylagmatine (SZ62), N²-hexadecanoyl-L-arginine methyl ester hydrochloride (SZ63), N²-octadecanoyl-L-glutamine tert-butyl ester (SZ64), N²-octadecanoyl-L-glutamine (SZ65)

This test was performed in the same manner as in Example 1 except that N²-hexadecanoyl-L-arginine (SZ61), N-hexadecanoylagmatine (SZ62), N²-hexadecanoyl-L-arginine methyl ester hydrochloride (SZ63), N²-octadecanoyl-L-glutamine tert-butyl ester (SZ64), and N²⁻octadecanoyl-L-glutamine (SZ65) were used. The results are shown in Fig. 5.

As is clear from the results shown in Fig. 5, anti-OVA-specific IgG1 subclass antibody production significantly increased in the Alum administration group (Alum in Figure), SZ61 administration group (SZ61 in Figure), SZ62 administration group (SZ62 in Figure), SZ63 administration group (SZ63 in Figure), SZ64 administration group (SZ64 in Figure), and SZ65 administration group (SZ65 in Figure) as compared to the OVA single administration group (Saline in Figure). The result has clarified that N²-hexadecanoyl-L-arginine (SZ61), N²-hexadecanoyl-L-arginine methyl ester hydrochloride (SZ63), N²-octadecanoyl-L-glutamine tert-butyl ester (SZ64) and N²-octadecanoyl-L-glutamine (SZ65) have, like N-hexadecanoylagmatine (SZ62), an adjuvant activity equivalent to or not less than that of Alum.

### [Example 6] Evaluation test of allergy inducing activity of N²-hexadecanoyl-L-arginine (SZ61), N-hexadecanoylagmatine (SZ62), N²-hexadecanoyl-L-arginine methyl ester hydrochloride (SZ63), N²-octadecanoyl-L-glutamine tert-butyl ester (SZ64), N²-octadecanoyl-L-glutamine (SZ65)

This test was performed in the same manner as in Example 3 except that N²-hexadecanoyl-L-arginine (SZ61), N-hexadecanoylagmatine (SZ62), N²-hexadecanoyl-L-arginine methyl ester hydrochloride (SZ63), N²-octadecanoyl-L-glutamine tert-butyl ester (SZ64), and N²-octadecanoyl-L-glutamine (SZ65) were used. The results are shown in Fig. 6.

As is clear from the results shown in Fig. 6, production of anti-OVA-specific IgE antibody significantly increased in the ALUM administration group (in Figure: Alum) as compared to that of the OVA single administration group (Saline in Figure) as previously reported. In contrast, a significant increase in the anti-OVA-specific IgE antibody production was not observed in the SZ61 administration group (SZ61 in Figure), SZ62 administration group (SZ62 in Figure), SZ63 administration group (SZ63 in Figure), SZ64 administration group (SZ64 in Figure), and SZ65 administration group (SZ65 in Figure). The results have clarified that not only N-hexadecanoylagmatine (SZ62) but also N²-hexadecanoyl-L-arginine (SZ61), N²-hexadecanoyl-L-arginine methyl ester hydrochloride (SZ63), N²-octadecanoyl-L-glutamine tert-butyl ester (SZ64) and N²-octadecanoyl-L-glutamine (SZ65) show a lower allergy inducing activity as compared to aluminum hydroxide gel adjuvant that problematically induces allergy by inoculation.

### [Example 7] Evaluation test of adjuvant activity of N-docosanoyl glycine methyl ester (SZ69), N-docosanoyl-L-leucine methyl ester (SZ70), N-docosanoyl-L-phenylalanine methyl ester (SZ71), N-docosanoyl-L-glutamic acid 1-methyl ester (SZ72), N²-docosanoyl-L-lysine methyl ester trifluoroacetic acid salt (SZ73), N-docosanoyl-L-isoleucine methyl ester (SZ76), N-docosanoyl-L-valine methyl ester (SZ77)

This test was performed in the same manner as in Example 1 except that N-docosanoyl glycine methyl ester (SZ69), N-docosanoyl-L-leucine methyl ester (SZ70), N-docosanoyl-L-phenylalanine methyl ester (SZ71), N-docosanoyl-L-glutamic acid 1-methyl ester (SZ72), N²-docosanoyl-L-lysine methyl ester trifluoroacetic acid salt (SZ73), N-docosanoyl-L-isoleucine methyl ester (SZ76), and N-docosanoyl-L-valine methyl ester (SZ77) were used and a positive control adjuvant Addavax™ (InvivoGen) administration group was formed. The results are shown in Fig. 7.

As is clear from the results shown in Fig. 7, anti-OVA-specific IgG1 subclass antibody production significantly increased in the Addavax™ administration group (Addavax in Figure), SZ69 administration group (SZ69 in Figure), SZ70 administration group (SZ70 in Figure), SZ72 administration group (SZ72 in Figure), SZ73 administration group (SZ73 in Figure), SZ76 administration group (SZ76 in Figure), and SZ77 administration group (SZ77 in Figure) as compared to the OVA single administration group (saline in Figure). Although a significant difference was not found, it was clarified that anti-OVA-specific IgG1 subclass antibody production of the SZ71 administration group is equivalent to or not less than that of Alum. These results have clarified that N-docosanoyl glycine methyl ester (SZ69), N-docosanoyl-L-leucine methyl ester (SZ70), N-docosanoyl-L-phenylalanine methyl ester (SZ71), N-docosanoyl-L-glutamic acid 1-methyl ester (SZ72), N²-docosanoyl-L-lysine methyl ester trifluoroacetic acid salt (SZ73), N-docosanoyl-L-isoleucine methyl ester (SZ76), and N-docosanoyl-L-valine methyl ester (SZ77) have an adjuvant activity equivalent to or not less than that of Alum.

### [Example 8] Evaluation test of adjuvant activity of N-hexadecanoylagmatine (SZ62), N²-hexadecanoyl-L-arginine methyl ester hydrochloride (SZ63), N²-octadecanoyl-L-glutamine tert-butyl ester (SZ64), N-hexadecanoyl glycine methyl ester (SZ78), N-hexadecanoyl-L-leucine methyl ester (SZ79), N-hexadecanoyl-L-phenylalanine methyl ester (SZ80), N²-hexadecanoyl-L-lysine methyl ester trifluoroacetic acid salt (SZ81), N-hexadecanoyl-L-glutamic acid 1-methyl ester (SZ82)

This test was performed in the same manner as in Example 7 except that N-hexadecanoylagmatine (SZ62), N²-hexadecanoyl-L-arginine methyl ester hydrochloride (SZ63), N²-octadecanoyl-L-glutamine tert-butyl ester (SZ64), N-hexadecanoyl glycine methyl ester (SZ78), N-hexadecanoyl-L-leucine methyl ester (SZ79), N-hexadecanoyl-L-phenylalanine methyl ester (SZ80), N²-hexadecanoyl-L-lysine methyl ester trifluoroacetic acid salt (SZ81), and N-hexadecanoyl-L-glutamic acid 1-methyl ester (SZ82) were used and a positive control adjuvant Addavax™ (InvivoGen) administration group was formed. The results are shown in Fig. 8.

As is clear from the results shown in Fig. 8, anti-OVA-specific IgG1 subclass antibody production significantly increased in the Addavax™ administration group (Addavax in Figure), SZ62 administration group (SZ62 in Figure), SZ63 administration group (SZ63 in Figure), SZ64 administration group (SZ64 in Figure), SZ80 administration group (SZ80 in Figure), and SZ82 administration group (SZ82 in Figure) as compared to the OVA single administration group (saline in Figure). Although a significant difference was not found, it was clarified that anti-OVA-specific IgG1 subclass antibody production of the SZ78 administration group (SZ78 in Figure), SZ79 administration group (SZ79 in Figure), and SZ81 administration group (SZ81 in Figure) is equivalent to that of Alum. These results have clarified that N²-hexadecanoyl-L-arginine methyl ester hydrochloride (SZ63), N²-octadecanoyl-L-glutamine tert-butyl ester (SZ64), N-hexadecanoyl glycine methyl ester (SZ78), N-hexadecanoyl-L-leucine methyl ester (SZ79), N-hexadecanoyl-L-phenylalanine methyl ester (SZ80), N²-hexadecanoyl-L-lysine methyl ester trifluoroacetic acid salt (SZ81), and N-hexadecanoyl-L-glutamic acid 1-methyl ester (SZ82) have, like N-hexadecanoylagmatine (SZ62), an adjuvant activity equivalent to or not less than that of Alum.

### [Example 9] Evaluation test of allergy inducing activity of N-hexadecanoylagmatine (SZ62), N²-octadecanoyl-L-glutamine tert-butyl ester (SZ64), N-docosanoyl-L-leucine methyl ester (SZ70), N-docosanoyl-L-phenylalanine methyl ester (SZ71)

This test was performed in the same manner as in Example 6 except that N-hexadecanoylagmatine (SZ62), N²-octadecanoyl-L-glutamine tert-butyl ester (SZ64), N-docosanoyl-L-leucine methyl ester (SZ70), and N-docosanoyl-L-phenylalanine methyl ester (SZ71) were used and a positive control adjuvant Addavax™ (InvivoGen) administration group was formed. The results are shown in Fig. 9.

As is clear from the results shown in Fig. 9, anti-OVA-specific IgE antibody production significantly increased in the ALUM administration group (Alum in Figure) as compared to that of the OVA single administration group (saline in Figure) as previously reported. In addition, anti-OVA-specific IgE antibody production of the Addavax™ administration group (Addavax in Figure) also significantly increased as compared to the OVA single administration group (saline in Figure). In contrast, a significant increase in the anti-OVA-specific IgE antibody production was not observed in the SZ62 administration group (SZ62 in Figure), SZ64 administration group (SZ64 in Figure), SZ70 administration group (SZ70 in Figure), and SZ71 administration group (SZ71 in Figure). The results have clarified that N-hexadecanoylagmatine (SZ62), N²-octadecanoyl-L-glutamine tert-butyl ester (SZ64), N-docosanoyl-L-leucine methyl ester (SZ70), and N-docosanoyl-L-phenylalanine methyl ester (SZ71) show a lower allergy inducing activity as compared to aluminum hydroxide gel adjuvant and Addavax™ containing an existing adjuvant component that problematically induce allergy by inoculation.

### (4) Th1/Th2 response evaluation test

### [Example 10] Evaluation test of Th1/Th2 response of N-hexadecanoylagmatine (SZ62), N-docosanoyl-L-leucine methyl ester (SZ70)

8-Week-old female BALB/c mice were divided into groups (N=6), and each group was primarily immunized by dorsal-subcutaneous administration of (1) ovalbumin (OVA) (10 µg) dissolved in saline as an antigen (OVA single administration group), (2) OVA 10 µg and Addavax™ (mixed with saline at 1:1) dissolved in saline (Addavax™ administration group), (3) N-hexadecanoylagmatine (SZ62) (0.256 µmol) dissolved in saline added with OVA 10 µg and 5% αCD (SZ62 administration group), (4) N-docosanoyl-L-leucine methyl ester (SZ70) (0.256 µmol) dissolved in saline added with OVA 10 µg and 5% αCD (SZ70 administration group), each per mouse. Ten days later, groin lymph node was collected from all mice of the above-mentioned (1) - (4), lymph node cells were isolated and cultured in 10% FBS-containing RPMI1640 medium. Thereto was added 0.1 µg, 1 µg, 10 µg, 100 µg of OVA, respectively, 40 hr later, cell supernatant was recovered, and IFN-γ and IL-4 in the supernatant were measured by ELISA.

The measurement results of IFN-γ and IL-4 are shown in Figs. 10, 11, respectively.

As is clear from the results shown in Fig. 10, it was found that IFN-γ production from lymph node cells in the Addavax™ administration group (Addavax in Figure), SZ62 administration group (SZ62 in Figure), and SZ70 administration group (SZ70 in Figure) increased in an OVA volume dependent manner.

As is clear from the results shown in Fig. 11, IL-4 production from lymph node cells in the Addavax™ administration group (Addavax in Figure) increased in an OVA volume dependent manner. On the other hand, the SZ62 administration group (SZ62 in Figure) and SZ70 administration group (SZ70 in Figure) did not show an increase in the IL-4 production from lymph node cells. These results reveal that N-hexadecanoylagmatine (SZ62) and N-docosanoyl-L-leucine methyl ester (SZ70) may be adjuvants that induce Th1 type immunity.

### (5) Dispersibility study test

### [Example 11] Dispersibility study test of N-hexadecanoylagmatine (SZ62: compound wherein group corresponding to R⁷ in formula (II) is C₁₅ alkyl group)

N-hexadecanoylagmatine (SZ62: compound wherein group corresponding to R⁷ in formula (II) is C₁₅ alkyl group) (0.256 µmol) was separated in a 2 mL tube containing zilconia beads, 1 mL of 5% αCD-added saline or 10% 2-hydroxypropyl-β-cyclodextrin (hereinafter to be also simply referred to as HP-β-CD)-added saline was added and the mixture was stirred. Thereafter, the absorbance (OD Value=650 nm) was measured by a microplate reader, and the turbidity was calculated as an index of dispersibility. The measurement method of the turbidity was as follows.

### [Measurement method of turbidity]

SZ62 (0.256 µmol) was dispensed to a 2 mL tube containing zirconia beads, 5% αCD-added saline or 10% HP-β-CD-added saline (1 mL) was added, and the mixture was vigorously stirred three times under conditions of one time for 3.15 seconds at 6000 rpm. The mixture was dispensed by 200 µL to a 96 well plate, and the absorbance (OD Value=650 nm) was measured by a microplate reader. As a control, kaolinite (1 mg) was dissolved in distilled water (1 L), and the absorbance (OD Value=650 nm) was measured by a microplate reader. The absorbance thereof (OD Value=650 nm) was defined to be turbidity: 1, and the turbidity of each sample was calculated from the ratio with the measured value of kaolinite.

The measurement results of turbidity are shown in Fig. 12.

As is clear from the results shown in Fig. 12, the turbidity of SZ62 after addition of 10% HP-β-CD-added saline and stirring the mixture was significantly lower than the turbidity of SZ62 after addition of 5% αCD-added saline and stirring the mixture.

### (6) Adjuvant activity test

### [Example 12] Evaluation test of adjuvant activity of N-hexadecanoylagmatine (SZ62: compound wherein group corresponding to R⁷ in formula (II) is C₁₅ alkyl group)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (5 µg) added to 5% αCD-added saline, (3) OVA (5 µg) added to 10% HP-β-CD-added saline, (4) OVA (5 µg) and SZ62 (0.256 (µmol) dissolved or dispersed in 5% αCD-added saline, and (5) OVA (5 µg) and SZ62 (0.256 µmol) dissolved in 10% HP-β-CD-added saline, each per mouse. One week later, the secondary immunization was carried out by dorsal-subcutaneous administration of solutions or dispersions similar to those of the above-mentioned (1) - (5) again. Two weeks from the secondary immunization, the blood was extracted under anesthesia, and the obtained serum samples were measured for anti-OVA-specific IgG1 subclass antibody. The results are shown in Fig. 13.

As is clear from the results shown in Fig. 13, anti-OVA-specific IgG1 subclass antibody production significantly increased in the group administered with SZ62 dispersed in 5% aCD-added saline and the group administered with SZ62 dissolved in 10% HP-β-CD-added saline, as compared to the OVA single administration group (saline in Figure). The results show that SZ62 dissolved in 10% HP-β-CD-added saline also has an adjuvant activity. In general, when a medicament is formulated using a liquid carrier, solubilization of the medicament in the liquid carrier is considered to be important. To use SZ62 as a vaccine adjuvant preparation, dissolution in 10% HP-β-CD-added saline is considered to be more preferable than dispersing in 5% αCD-added saline.

### (7) Adjuvant activity test

### [Example 13] Evaluation test of adjuvant activity of N-acetyl-l-leucine methyl ester (SZ83), N-hexanoyl-L-leucine methyl ester (SZ84), N-(2-octadecyleicosanoyl)-L-leucine methyl ester (SZ86), N-hexadecanoyl-L-leucine tert-butyl ester (SZ89), N-hexadecanoyl-L-leucinamide (SZ90)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (5 µg) and SZ83 (0.256 µmol) dissolved or dispersed in 5% αCD-added saline (SZ83 administration group), (3) OVA (5 µg) and SZ84 (0.256 µmol) dissolved or dispersed in 5% αCD-added saline (SZ84 administration group), (4) OVA (5 µg) and SZ86 (0.256 µmol) dissolved or dispersed in 5% αCD-added saline (SZ86 administration group), (5) OVA (5 µg) and SZ89 (0.256 µmol) dissolved or dispersed in 5% αCD-added saline (SZ89 administration group), (6) OVA (5 µg) and SZ90 (0.256 µmol) dissolved or dispersed in 5% αCD-added saline (SZ90 administration group), each per mouse. After 12 days, blood samples were collected under anesthesia, and the obtained serum samples were subjected to the measurement of an anti-OVA-specific IgG1 subclass antibody. The results are shown in Fig. 14.

### [Measurement method of concentration of anti-OVA-specific IgG1 subclass antibody and anti-OVA-specific IgG2a subclass antibody]

To a 96 well plate was added 5 µg/ml OVA at 100 µl/well, and the mixture was incubated at 4°C overnight. After incubation, the mixture was washed three times with 200 µl of PBS-T (PBS+0.05% (v/v) Tween 20) per well, and blocked with 100 µl of 5% FCS/PBS solution per well at room temperature for 1 - for 2 hr. Thereafter, the mixture was washed three times with PBS-T (200 µl/well), a diluted serum sample (100 µl/well) or the same amount of 5% FCS/PBS solution as a control was added, and the mixture was incubated at 37°C for 1 hr. To calculate concentration of the antibody, the standard of an anti-OVA-specific IgG1 subclass antibody diluted with 2% FCS/PBS solution was added at 100 ng/ml, 50 ng/ml, 25 ng/ml, 12.5 ng/ml, 6.25 ng/ml, 3.125 ng/ml, 1.56 ng/ml, 0.781 ng/ml, 0.391 ng/ml, 0.195 ng/ml, 0.098 ng/ml, 0 ng/ml, per 1 well. When an anti-OVA-specific IgG2a subclass antibody was measured, the standard of an anti-OVA-specific IgG2a subclass antibody was added at 50 ng/ml, 25 ng/ml, 12,5 ng/ml, 6.25 ng/ml, 3.125 ng/ml, 1.56 ng/ml, 0.781 ng/ml, 0.391 ng/ml, 0.195 ng/ml, 0.098 ng/ml, 0.049 ng/ml, 0 ng/ml, per 1 well. Thereafter, similar to the samples, the mixture was washed five times with PBS-T (200 µl/well), a diluted biotinylated anti-mouse IgG1 antibody was added (100 µl/well), and the mixture was incubated at 37°C for 45 min. Thereafter, the mixture was washed five times with PBS-T (200 µl/well), a diluted anti-biotin-HRP antibody was added (100 µl/well), and the mixture was incubated at 37°C for another 30 min. Thereafter, the mixture was washed five times with PBS-T (200 µl/well), TMB substrate solution was added (100 µl/well), and the mixture was incubated at room temperature for 10 - for 15 min. Thereafter, a reaction quenching liquid (2N sulfuric acid solution) was added (50 µl/well) to discontinue a color developing reaction, and the absorbance (OD Value=450 nm) was measured by a microplate reader. A standard curve was drawn from the measured OD values, and the concentration was calculated.

As is clear from the results shown in Fig. 14, production of anti-OVA-specific IgG1 subclass antibody in the SZ83 administration group (SZ83 in Figure), SZ84 administration group (SZ84 in Figure), SZ86 administration group (SZ86 in Figure), SZ89 administration group (SZ89 in Figure), SZ90 administration group (SZ90 in Figure) was found to show a tendency to increase. These results suggest a possibility that N-acetyl-l-leucine methyl ester (SZ83), N-hexanoyl-L-leucine methyl ester (SZ84), N-(2-octadecyleicosanoyl)-L-leucine methyl ester (SZ86), N-hexadecanoyl-L-leucine tert-butyl ester (SZ89), N-hexadecanoyl-L-leucinamide (SZ90) have an adjuvant activity.

### [Example 14] Evaluation test of adjuvant activity of N²-hexadecanoyl-N¹,N¹-diethyl-L-leucinamide (SZ92), N-hexadecanoyl-L-histidine methyl ester (SZ94), N-hexadecanoyl-L-proline methyl ester (SZ95), N-hexadecanoyl-L-serine methyl ester (SZ96)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (5 µg) and SZ92 (0.256 µmol) dissolved or dispersed in 5% αCD-added saline (SZ92 administration group), (3) OVA (5 µg) and SZ94 (0.256 µmol) dissolved or dispersed in 5% αCD-added saline (SZ94 administration group), (4) OVA (5 µg) and SZ95 (0.256 µmol) dissolved or dispersed in 5% αCD-added saline (SZ95 administration group), (5) OVA (5 µg) and SZ96 (0.256 µmol) dissolved or dispersed in 5% αCD-added saline (SZ96 administration group), each per mouse. After 12 days, blood samples were collected under anesthesia, and the obtained serum samples were subjected to the measurement of anti-OVA-specific IgG1 subclass antibody. The results are shown in Fig. 15.

As is clear from the results shown in Fig. 15, the production of an anti-OVA-specific IgG1 subclass antibody significantly increased in the SZ92 administration group (SZ92 in Figure), as compared to the OVA single administration group (saline in Figure). While a significant difference was not observed, it was found that the production of an anti-OVA-specific IgG1 subclass antibody tended to increase in the SZ94 administration group (SZ94 in Figure), SZ95 administration group (SZ95 in Figure), and SZ96 administration group (SZ96 in Figure). These results suggest a possibility that N²-hexadecanoyl-N¹,N¹-diethyl-L-leucinamide (SZ92), N-hexadecanoyl-L-histidine methyl ester (SZ94), N-hexadecanoyl-L-proline methyl ester (SZ95), and N-hexadecanoyl-L-serine methyl ester (SZ96) have an adjuvant activity.

### [Example 15] Evaluation test of adjuvant activity of N-hexadecanoyl-L-leucine hexyl ester (SZ97), N²-hexadecanoyl-L-arginine hexyl ester hydrochloride (SZ99), N²-docosanoyl-N¹,N¹-diethyl-L-glutamic acid 1-amide (SZ106)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (5 µg) and SZ97 (0.256 µmol) dissolved or dispersed in 5% αCD-added saline (SZ97 administration group), (3) OVA (5 µg) and SZ99 (0.256 µmol) dissolved or dispersed in 5% αCD-added saline (SZ99 administration group), (4) OVA (5 µg) and SZ106 (0.256 µmol) dissolved or dispersed in 5% αCD-added saline (SZ106 administration group), each per mouse. After 12 days, blood samples were collected under anesthesia, and the obtained serum samples were subjected to the measurement of anti-OVA-specific IgG1 subclass antibody. The results are shown in Fig. 16.

As is clear from the results shown in Fig. 16, production of anti-OVA-specific IgG1 subclass antibody by SZ99 administration group (SZ99 in Figure) significantly increased as compared to OVA single administration group (saline in Figure). While a significant difference was not found, production of an anti-OVA-specific IgG1 subclass antibody in the SZ97 administration group (SZ97 in Figure) and SZ106 administration group (SZ106 in Figure) was found to show a tendency to increase. These results suggest a possibility that N-hexadecanoyl-L-leucine hexyl ester (SZ97), N²-hexadecanoyl-L-arginine hexyl ester hydrochloride (SZ99), N²-docosanoyl-N¹,N¹-diethyl-L-glutamic acid 1-amide (SZ106) have an adjuvant activity.

### [Example 16] Evaluation test of adjuvant activity of N-docosanoylagmatine hydrochloride (SZ108), N²-hexadecanoyl-N¹,N¹-diethyl-L-arginine amide hydrochloride (SZ110)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (5 µg) and SZ108 (0.256 µmol) dissolved or dispersed in 5% αCD-added saline (SZ108 administration group), (3) OVA (5 µg) and SZ110 (0.256 µmol) dissolved or dispersed in 5% αCD-added saline (SZ110 administration group), each per mouse. After 12 days, blood samples were collected under anesthesia, and the obtained serum samples were subjected to the measurement of anti-OVA-specific IgG1 subclass antibody. The results are shown in Fig. 17.

As is clear from the results shown in Fig. 17, the anti-OVA-specific IgG1 subclass antibody production in the SZ108 administration group (SZ108 in Figure) significantly increased as compared to the OVA single administration group (Saline in Figure). While a significant difference was not observed, it was found that the production of an anti-OVA-specific IgG1 subclass antibody tended to increase in the SZ110 administration group (SZ110 in Figure). These results suggest a possibility that the N-docosanoylagmatine hydrochloride (SZ108) and N²-hexadecanoyl-N¹,N¹-diethyl-L-arginine amide hydrochloride (SZ110) have an adjuvant activity.

### [Example 17] Evaluation test of adjuvant activity of N²-docosanoyl-L-arginine hexyl ester hydrochloride (SZ121), N-docosanoyl-L-leucine hexyl ester (SZ124), N²-docosanoyl-N¹,N¹-diethyl-L-leucinamide (SZ125)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (5 µg) and SZ121 (0.256 µmol) dissolved or dispersed in 5% αCD-added saline (SZ121 administration group), (3) OVA (5 µg) and SZ124 (0.256 µmol) dissolved or dispersed in 5% αCD-added saline (SZ124 administration group), (4) OVA (5 µg) and SZ125 (0.256 µmol) dissolved or dispersed in 5% αCD-added saline (SZ125 administration group), each per mouse. After 12 days, blood samples were collected under anesthesia, and the obtained serum samples were subjected to the measurement of anti-OVA-specific IgG1 subclass antibody. The results are shown in Fig. 18.

As is clear from the results shown in Fig. 18, the anti-OVA-specific IgG1 subclass antibody production in the SZ121 administration group (SZ121 in Figure) significantly increased as compared to the OVA single administration group (Saline in Figure). While a significant difference was not observed, it was found that the production of an anti-OVA-specific IgG1 subclass antibody tended to increase in the SZ124 administration group (SZ124 in Figure) and SZ125 administration group (SZ125 in Figure). These results suggest a possibility that the N²-docosanoyl-L-arginine hexyl ester hydrochloride (SZ121), N-docosanoyl-L-leucine hexyl ester (SZ124) and N²-docosanoyl-N¹,N¹-diethyl-L-leucinamide (SZ125) have an adjuvant activity.

### [Example 18] Evaluation test of adjuvant activity after primary immunization with N²-docosanoyl-L-arginine amide hydrochloride (SZ128), N²-docosanoyl-N¹,N¹-diethyl-L-arginine amide hydrochloride (SZ129), N²-hexanoyl-L-arginine hexyl ester hydrochloride (SZ130), N-hexadecanoyl-L-glutamic acid hexyl ester (SZ134), N-docosanoyl-L-glutamic acid hexyl ester (SZ135), N²-hexadecanoyl-L-glutamine methyl ester (SZ136)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (5 µg) and SZ128 (0.256 µmol) dissolved or dispersed in 5% aCD-added saline (SZ128 administration group), (3) OVA (5 µg) and SZ129 (0.256 µmol) dissolved or dispersed in 5% αCD-added saline (SZ129 administration group), (4) OVA (5 µg) and SZ130 (0.256 µmol) dissolved or dispersed in 5% αCD-added saline (SZ130 administration group), (5) OVA (5 µg) and SZ134 (0.256 µmol) dissolved or dispersed in 5% αCD-added saline (SZ134 administration group), (6) OVA (5 µg) and SZ135 (0.256 µmol) dissolved or dispersed in 5% αCD-added saline (SZ135 administration group), (7) OVA (5 µg) and SZ136 (0.256 µmol) dissolved or dispersed in 5% αCD-added saline (SZ136 administration group), each per mouse. After 12 days, blood samples were collected under anesthesia, and the obtained serum samples were subjected to the measurement of anti-OVA-specific IgG1 subclass antibody. The results are shown in Fig. 19.

As is clear from the results shown in Fig. 19, the anti-OVA-specific IgG1 subclass antibody production of the SZ128 administration group (SZ128 in Figure) and SZ129 administration group (SZ129 in Figure) significantly increased as compared to the OVA single administration group (saline in Figure). While a significant difference was not observed, it was found that the production of an anti-OVA-specific IgG1 subclass antibody tended to increase in the SZ130 administration group (SZ130 in Figure), SZ134 administration group (SZ134 in Figure), SZ135 administration group (SZ135 in Figure), SZ136 administration group (SZ136 in Figure). These results suggest a possibility that N²-docosanoyl-L-arginine amide hydrochloride (SZ128), N²-docosanoyl-N¹,N¹-diethyl-L-arginine amide hydrochloride (SZ129), N²-hexanoyl-L-arginine hexyl ester hydrochloride (SZ130), N-hexadecanoyl-L-glutamic acid hexyl ester (SZ134), N-docosanoyl-L-glutamic acid hexyl ester (SZ135) and N²-hexadecanoyl-L-glutamine methyl ester (SZ136) have an adjuvant activity.

### [Example 19] Evaluation test of adjuvant activity and allergy inducing activity of N-(2-octadecyleicosanoyl)-L-leucine methyl ester (SZ86), N-hexadecanoyl-L-leucinamide (SZ90), N²-hexadecanoyl-N¹,N¹-diethyl-L-leucinamide (SZ92), N²-hexadecanoyl-L-arginine hexyl ester hydrochloride (SZ99), N²-docosanoyl-N¹,N¹-diethyl-L-glutamic acid 1-amide (SZ106)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 10 µg and Addavax™ (mixed with saline at 1:1) dissolved in saline (Addavax™ administration group), (3) OVA (5 ug) and SZ86 (0.256 µmol) dissolved or dispersed in 5% αCD-added saline (SZ86 administration group), (4) OVA (5 µg) and SZ90 (0.256 µmol) dissolved or dispersed in 5% αCD-added saline (SZ90 administration group), (5) OVA (5 µg) and SZ92 (0.256 µmol) dissolved or dispersed in 5% αCD-added saline (SZ92 administration group), (6) OVA (5 pg) and SZ99 (0.256 µmol) dissolved or dispersed in 5% αCD-added saline (SZ99 administration group), (7) OVA (5 µg) and SZ106 (0.256 µmol) dissolved or dispersed in 5% αCD-added saline (SZ106 administration group), each per mouse. One week later, the secondary immunization was carried out by dorsal-subcutaneous administration of solutions or dispersions similar to those of the above-mentioned (1) - (7) again. Two weeks from the secondary immunization, the blood was extracted under anesthesia, and the obtained serum samples were measured for anti-OVA-specific IgG1 subclass antibody. The results are shown in Fig. 20.

As is clear from the results shown in Fig. 20, the anti-OVA-specific IgG1 subclass antibody production of the Addavax™ administration group (Addavax in Figure) and SZ92 administration group (SZ92 in Figure) significantly increased as compared to the OVA single administration group (Saline in Figure). While a significant difference was not observed, it was found that the production of an anti-OVA-specific IgG1 subclass antibody tended to increase in the SZ86 administration group (SZ86 in Figure), SZ90 administration group (SZ90 in Figure), SZ99 administration group (SZ99 in Figure) and SZ106 administration group (SZ106 in Figure). These results suggest a possibility that N-(2-octadecyleicosanoyl)-L-leucine methyl ester (SZ86), N-hexadecanoyl-L-leucinamide (SZ90), N²-hexadecanoyl-N¹,N¹-diethyl-L-leucinamide (SZ92), N²-hexadecanoyl-L-arginine hexyl ester hydrochloride (SZ99) and N²-docosanoyl-N¹,N¹-diethyl-L-glutamic acid 1-amide (SZ106) have an adjuvant activity.

The allergy inducing activity test was performed in the same manner as in Example 3 except that Addavax™, N-(2-octadecyleicosanoyl)-L-leucine methyl ester (SZ86), N-hexadecanoyl-L-leucinamide (SZ90), N²-hexadecanoyl-N¹,N¹-diethyl-L-leucinamide (SZ92), N²-hexadecanoyl-L-arginine hexyl ester hydrochloride (SZ99) and N²-docosanoyl-N¹,N¹-diethyl-L-glutamic acid 1-amide (SZ106) were used. The results are shown in Fig. 21.

As is clear from the results shown in Fig. 21, the anti-OVA-specific IgE antibody production of the Addavax™ administration group (Addavax in Figure) significantly increased as compared to the OVA single administration group (saline in Figure). In contrast, a significant increase in the anti-OVA-specific IgE antibody production was not observed in the SZ86 administration group (SZ86 in Figure), SZ90 administration group (SZ90 in Figure), SZ92 administration group (SZ92 in Figure), SZ99 administration group (SZ99 in Figure) and SZ106 administration group (SZ106 in Figure). From these results, it was clarified that N-(2-octadecyleicosanoyl)-L-leucine methyl ester (SZ86), N-hexadecanoyl-L-leucinamide (SZ90), N²-hexadecanoyl-N¹,N¹-diethyl-L-leucinamide (SZ92), N²-hexadecanoyl-L-arginine hexyl ester hydrochloride (SZ99) and N²-docosanoyl-N¹,N¹-diethyl-L-glutamic acid 1-amide (SZ106) have a low allergy inducing activity as compared to Addavax™known to induce allergy due to inoculation.

### (8) Evaluation of adjuvant not inducing allergy

### [Definition of index for evaluation of adjuvant not inducing allergy]

It is a problem that aluminum hydroxide gel adjuvant and Addavax™ potentiate antigen-specific antibody production but induce allergy. Therefore, the index for adjuvant not inducing allergy (Effective Index) was defined as follows, and an index of each administration group in Example 19 was calculated. That is, a value obtained by dividing the mean of the anti-OVA-specific IgG1 subclass antibody concentration of each group by the mean of the anti-OVA-specific IgE antibody concentration of each group was relatively compared with a value obtained by dividing the mean of the anti-OVA-specific IgG1 subclass antibody concentration of the Addavax™ administration group by the mean of the anti-OVA-specific IgE antibody concentration of the Addavax™ administration group as 1. The results are shown in Fig. 22.

As is clear from the results shown in Fig. 22, it was clarified that the Effective Index of the SZ86 administration group (SZ86 in Figure), SZ90 administration group (SZ90 in Figure) and SZ92 administration group (SZ92 in Figure) is greater than 1 of the Addavax™ administration group. These results suggest that the N-(2-octadecyleicosanoyl)-L-leucine methyl ester (SZ86), N-hexadecanoyl-L-leucinamide (SZ90) and N²-hexadecanoyl-N¹,N¹-diethyl-L-leucinamide (SZ92) are superior adjuvants that do not induce allergy as compared to Addavax™.

### [Example 20] Evaluation test of adjuvant activity and allergy inducing activity after secondary immunization with N-(2-octadecyleicosanoyl)-L-leucine methyl ester (SZ86), N²-hexadecanoyl-L-leucinamide (SZ90), N²-hexadecanoyl-N¹,N¹-diethyl-L-leucinamide (SZ92), N-hexadecanoyl-L-leucine hexyl ester (SZ97), N²-docosanoyl-N¹,N¹-diethyl-L-glutamic acid 1-amide (SZ106), N-docosanoylagmatine hydrochloride (SZ108)

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 10 µg and Addavax™ (mixed with saline at 1:1) dissolved in saline (Addavax™ administration group) (3) OVA (5 µg) and SZ86 (0.256 µmol) dissolved or dispersed in 5% αCD-added saline (SZ86 administration group), (4) OVA (5 µg) and SZ90 (0.256 µmol) dissolved or dispersed in 5% αCD-added saline (SZ90 administration group), (5) OVA (5 µg) and SZ92 (0.256 µmol) dissolved or dispersed in 5% αCD-added saline (SZ92 administration group), (6) OVA (5 µg) and SZ97 (0.256 µmol) dissolved or dispersed in 5% αCD-added saline (SZ97 administration group), (7) OVA (5 µg) and SZ106 (0.256 µmol) dissolved or dispersed in 5% αCD-added saline (SZ106 administration group), (8) OVA (5 µg) and SZ108 (0.256 µmol) dissolved or dispersed in 5% αCD-added saline (SZ108 administration group), each per mouse. One week later, the secondary immunization was carried out by dorsal-subcutaneous administration of solutions or dispersions similar to those of the above-mentioned (1) - (8) again. Two weeks from the secondary immunization, the blood was extracted under anesthesia, and the obtained serum samples were measured for anti-OVA-specific IgG1 subclass antibody. The results are shown in Fig. 23.

As is clear from the results shown in Fig. 23, the anti-OVA-specific IgG1 subclass antibody production of the Addavax™ administration group (Addavax in Figure), SZ92 administration group (SZ92 in Figure) and SZ97 administration group (SZ97 in Figure) is significantly increased as compared to the OVA single administration group (saline in Figure). While a significant difference was not observed, it was found that the production of an anti-OVA-specific IgG1 subclass antibody tended to increase in the SZ86 administration group (SZ86 in Figure), SZ90 administration group (SZ90 in Figure), SZ106 administration group (SZ106 in Figure), SZ108 administration group (SZ108 in Figure). These results suggest that the N-(2-octadecyleicosanoyl)-L-leucine methyl ester (SZ86), N²-hexadecanoyl-L-leucinamide (SZ90), N²-hexadecanoyl-N¹,N¹-diethyl-L-leucinamide (SZ92), N-hexadecanoyl-L-leucine hexyl ester (SZ97), N²-docosanoyl-N¹,N¹-diethyl-L-glutamic acid 1-amide (SZ106) and N-docosanoylagmatine hydrochloride (SZ108) have an adjuvant activity.

The allergy inducing activity test was performed in the same manner as in Example 3 except that Addavax™, N-(2-octadecyleicosanoyl)-L-leucine methyl ester (SZ86), N-hexadecanoyl-L-leucinamide (SZ90), N²-hexadecanoyl-N¹,N¹-diethyl-L-leucinamide (SZ92), N-hexadecanoyl-L-leucine hexyl ester (SZ97), N²-docosanoyl-N¹,N¹-diethyl-L-glutamic acid 1-amide (SZ106) and N-docosanoylagmatine hydrochloride (SZ108) were used. The results are shown in Fig. 24.

As is clear from the results shown in Fig. 24, the anti-OVA-specific IgE antibody production in the Addavax™ administration group (Addavax in Figure) significantly increased as compared to the OVA single administration group (saline in Figure). In contrast, a significant increase in the anti-OVA-specific IgE antibody production was not observed in the SZ86 administration group (SZ86 in Figure), SZ90 administration group (SZ90 in Figure), SZ92 administration group (SZ92 in Figure), SZ97 administration group (SZ97 in Figure), SZ106 administration group (SZ106 in Figure), SZ108 administration group (SZ108 in Figure). From these results, it was clarified that N-(2-octadecyleicosanoyl)-L-leucine methyl ester (SZ86), N-hexadecanoyl-L-leucinamide (SZ90), N²-hexadecanoyl-N¹,N¹-diethyl-L-leucinamide (SZ92), N-hexadecanoyl-L-leucine hexyl ester(SZ97), N²-docosanoyl-N¹,N¹-diethyl-L-glutamic acid 1-amide (SZ106) and N-docosanoylagmatine hydrochloride (SZ108) have a low allergy inducing activity as compared to Addavax™ known to induce allergy due to inoculation.

The evaluation of an adjuvant that does not induce allergy was performed in the same manner as in Example 19 except that N-(2-octadecyleicosanoyl)-L-leucine methyl ester (SZ86), N-hexadecanoyl-L-leucinamide (SZ90), N²-hexadecanoyl-N¹,N¹-diethyl-L-leucinamide (SZ92), N-hexadecanoyl-L-leucine hexyl ester (SZ97), N²-docosanoyl-N¹,N¹-diethyl-L-glutamic acid 1-amide (SZ106) and N-docosanoylagmatine hydrochloride (SZ108) were used. The results are shown in Fig. 25.

As is clear from the results shown in Fig. 25, it was clarified that the Effective Index of the SZ90 administration group (SZ90 in Figure), SZ92 administration group (SZ92 in Figure) and SZ97 administration group (SZ97 in Figure) is higher than 1 of the Addavax™ administration group. These results suggest that N-hexadecanoyl-L-leucinamide (SZ90), N²-hexadecanoyl-N¹,N¹-diethyl-L-leucinamide (SZ92) and N-hexadecanoyl-L-leucine hexyl ester (SZ97) are superior adjuvants that do not induce allergy as compared to Addavax™.

### (9) Evaluation as combination adjuvant

### [Combination adjuvant]

From the 1990s, combination adjuvants of a mixture of plural adjuvants have been developed. For example, it is known that adjuvants such as aluminum hydroxide salt, oil-in-water emulsion, liposome and the like can afford stronger and effective immune responses when combined with molecules such as immunostimulating agents (e.g., monophosphoryl lipid (MPL), QS-21, vitamin E and the like), and the like. MPL and aluminum hydroxide salt have been introduced as an adjuvant (AS04) for a cervical cancer vaccine, and a combination of Addavax™ and CpG is being studied as a cancer vaccine adjuvant. As an important aspect in considering the combination of adjuvants, it is known that a combination of plural adjuvants does not always afford a good effect, and some combination causes a competitive failure.

In the following Examples, whether a good effect is obtained by combining MPL or CpG generally studied as a combination adjuvant with the compound of the present invention was studied.

### [Example 21] Evaluation test of adjuvant activity and allergy inducing activity after secondary immunization when a mixture of N-hexadecanoylagmatine (SZ62: compound wherein the group corresponding to R⁷ in the formula (II) is C₁₅ alkyl group) and MPL or CpG is administered

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA (10 µg) and aluminum hydroxide salt dissolved in saline (Alum administration group), (3) OVA 10 µg and Addavax™ (mixed with saline at 1:1) dissolved in saline (Addavax™ administration group), (4) OVA 10 µg and CpG 5 µg dissolved in saline (CpG administration group), (5) OVA 10 µg and MPL 5 µg dissolved in saline (MPL administration group), (6) OVA (5 µg) and SZ62 (0.256 µmol) dissolved or dispersed in 5% αCD-added saline (SZ62 administration group), (7) OVA (5 µg), SZ62 (0.256 µmol) and CpG 5 µg dissolved or dispersed in 5% αCD-added saline (SZ62+CpG administration group), (8) OVA 10 µg, Addavax™(mixed with saline at 1:1) and CpG 5 µg dissolved in saline (Addavax™+CpG administration group), (9) OVA (5 µg), SZ62 (0.256 µmol) and MPL 5 µg dissolved or dispersed in 5% αCD-added saline (SZ62+MPL administration group), (10) OVA (10 µg), aluminum hydroxide salt (Alum) and MPL 5 µg dissolved in saline (Alum+MPL administration group), each per mouse. One week later, the secondary immunization was carried out by dorsal-subcutaneous administration of solutions or dispersions similar to those of the above-mentioned (1) - (10) again. Two weeks from the secondary immunization, the blood was extracted under anesthesia, and the obtained serum samples were measured for anti-OVA-specific IgG1 subclass antibody, anti-OVA-specific IgG2a subclass antibody and anti-OVA-specific IgE antibody. The results thereof are respectively shown in Fig. 26, Fig. 27 and Fig. 28. The measurement methods were respectively similar to Example 18 and Example 3.

As is clear from the results shown in Fig. 26, the anti-OVA-specific IgG1 subclass antibody production in the Addavax™ administration group (Addavax in Figure), MPL administration group (MPL in Figure), SZ62+CpG administration group (SZ62+CpG in Figure), Addavax™+CpG administration group (Addavax+CpG in Figure), SZ62+MPL administration group (SZ62+MPL in Figure), Alum+MPL administration group (Alum+MPL in Figure) significantly increased as compared to the OVA single administration group (saline in Figure). While a significant difference was not observed, it was found that the production of an anti-OVA-specific IgG1 subclass antibody tended to increase in the Alum administration group (Alum in Figure) and SZ62 administration group (SZ62 in Figure). These results show that SZ62 provides a good effect by combining with CpG and MPL.

As is clear from the results shown in Fig. 27, the anti-OVA-specific IgG2a subclass antibody production in the CpG administration group (CpG in Figure), SZ62+CpG administration group (SZ62+CpG in Figure), Addavax™+CpG administration group (Addavax+CpG in Figure), Alum+MPL administration group (Alum+MPL in Figure) significantly increased as compared to the OVA single administration group (saline in Figure). While a significant difference was not observed, it was found that the production of an anti-OVA-specific IgG2a subclass antibody tended to increase in the Alum administration group (Alum in Figure), Addavax™ administration group (Addavax in Figure), MPL administration group (MPL in Figure), SZ62 administration group (SZ62 in Figure) and SZ62+MPL administration group (SZ62+MPL in Figure). These results show that SZ62 provides a good effect by combining with CpG or MPL.

As is clear from the results shown in Fig. 28, the anti-OVA-specific IgE antibody production in the Addavax™ administration group (Addavax in Figure) and Addavax™+CpG administration group (Addavax+CpG in Figure) significantly increased as compared to the OVA single administration group (saline in Figure). While a significant difference was not observed, it was found that the production of an anti-OVA-specific IgE antibody tended to increase in the SZ62+CpG administration group (SZ62+CpG in Figure) and SZ62+MPL administration group (SZ62+MPL in Figure). However, these results suggest that, since the anti-OVA-specific IgE antibody production of the SZ62+CpG administration group (SZ62+CpG in Figure) is lower than that in the Addavax™+CpG administration group (Addavax+CpG in Figure), SZ62 is superior to Addavax™ as a combination adjuvant with lower side effects to being combined with CpG.

### (10) Evaluation of systemic inflammation after adjuvant administration

### [Example 22] Evaluation test of systemic inflammation after primary immunization when a mixture of N-hexadecanoylagmatine (SZ62: compound wherein the group corresponding to R⁷ in the formula (II) is C₁₅ alkyl group) and MPL or CpG is administered

8-Week-old female BALB/c mice were divided into groups (N=6), and each group was primarily immunized by dorsal-subcutaneous administration of (1) ovalbumin (OVA) (5 µg) dissolved in saline as an antigen (OVA single administration group), (2) OVA 10 µg and Addavax™ (mixed with saline at 1:1) dissolved in saline (Addavax™ administration group), (3) OVA (10 µg) and aluminum hydroxide salt dissolved in saline (Alum administration group), (4) OVA (10 µg) and CpG 5 µg dissolved in saline (CpG administration group), (5) OVA (10 µg) and MPL 5 µg dissolved in saline (MPL administration group), (6) OVA (5 µg) and SZ62 (0.256 µmol) dissolved or dispersed in 5% αCD-added saline (SZ62 administration group), (7) OVA (5 µg), SZ62 (0.256 µmol) and CpG 5 µg dissolved or dispersed in 5% αCD-added saline (SZ62+CpG administration group), (8) OVA (10 µg), Addavax™(mixed with saline at 1:1) and CpG 5 µg dissolved in saline (Addavax™+CpG administration group), (9) OVA (5 µg), SZ62 (0.256 µmol) and MPL 5 µg dissolved or dispersed in 5% αCD-added saline (SZ62+MPL administration group), (10) OVA (10 µg), Addavax™ (mixed with saline at 1:1) and MPL 5 µg dissolved in saline (Addavax™+MPL administration group), each per mouse. After 3 hr, blood samples were collected under anesthesia, and the obtained serum samples were subjected to the measurement of TNF-α by ELISA method. The results are shown in Fig. 29.

As is clear from the results shown in Fig. 29, the TNF-α production in the serum of the CpG administration group (CpG in Figure) and Addavax™+CpG administration group (Addavax+CpG in Figure) significantly increased as compared to the OVA single administration group (saline in Figure). While a significant difference was not observed, it was found that the production of TNF-α tended to increase in the Addavax™ administration group (Addavax in Figure), SZ62+CpG administration group (SZ62+CpG in Figure), Addavax™+MPL administration group (Addavax+MPL in Figure). However, these results suggest that, since the TNF-α production of the SZ62+CpG administration group (SZ62+CpG in Figure) and SZ62+MPL administration group (SZ62+MPL in Figure) is respectively lower than that in the Addavax™+CpG administration group (Addavax+CpG in Figure) and Addavax™+MPL administration group (Addavax+MPL in Figure), SZ62 is superior to Addavax™ as a combination adjuvant with lower side effects to be combined with CpG or MPL.

### Industrial Applicability

Since the compound of the present invention has an antigen-specific IgG1 subclass antibody and/or IgG2a subclass antibody production-enhancing effect (immunostimulatory effect), it is useful as an immunostimulating agent. Particularly, since the compound of the present invention has an immunostimulatory effect equivalent to or not less than that of conventional aluminum gel adjuvants, suppresses induction of IgE antibody production, and sometimes suppresses problematic allergy inducing activity of conventional aluminum gel adjuvants, it can be an effective and safe adjuvant. Furthermore, since the compound of the present invention induces IgA antibody production on the mucosa, it can also be a mucosal vaccine adjuvant of which the research and development are ongoing at present. By combining with an existing adjuvant, the development as a combination adjuvant that enhances immune response is also expected.

This application is based on patent application No. 2015-212222 filed in Japan (filing date: October 28, 2015) and patent application No. 2016-091808 filed in Japan (filing date: April 28, 2016), the contents of which are incorporated in full herein.

## Claims

1. An immunostimulating agent comprising at least one kind of a compound represented by the formula (I): 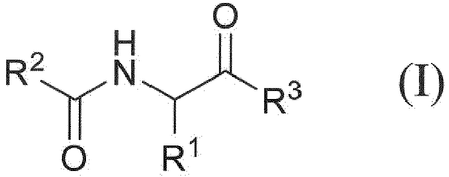 wherein
R¹ is an amino acid side chain excluding a cystine side chain;
R² is a C₁₋₃₇ alkyl group; and
R³ is a hydroxyl group, a C₁₋₆ alkoxy group or -NR⁴R⁵ wherein R⁴ and R⁵ are the same or different and each is a hydrogen atom or a C₁₋₆ alkyl group,
or a salt thereof.

2. The immunostimulating agent according to claim 1, wherein R¹ is selected from the group consisting of an arginine side chain, a glutamine side chain, a glutamic acid side chain, a hydrogen atom, an isoleucine side chain, a leucine side chain, a lysine side chain, a phenylalanine side chain, and a valine side chain.

3. The immunostimulating agent according to claim 2, wherein R¹ is selected from the group consisting of an arginine side chain, and a glutamine side chain.

4. The immunostimulating agent according to claim 1, wherein R¹ is selected from the group consisting of a histidine side chain, a proline side chain, and a serine side chain.

5. The immunostimulating agent according to any one of claims 1 to 4, wherein R² is a C₁₂₋₂₄ alkyl group.

6. The immunostimulating agent according to any one of claims 1, 2 and 5, wherein the compound represented by the formula (I) or a salt thereof is selected from the group consisting of
N²-hexadecanoyl-L-arginine,
N²-hexadecanoyl-L-arginine methyl ester,
N²-octadecanoyl-L-glutamine tert-butyl ester,
N²-octadecanoyl-L-glutamine,
N-docosanoyl glycine methyl ester,
N-docosanoyl-L-leucine methyl ester,
N-docosanoyl-L-phenylalanine methyl ester,
N-docosanoyl-L-glutamic acid 1-methyl ester,
N²-docosanoyl-L-lysine methyl ester,
N-docosanoyl-L-isoleucine methyl ester,
N-docosanoyl-L-valine methyl ester,
N-hexadecanoyl glycine methyl ester,
N-hexadecanoyl-L-leucine methyl ester,
N-hexadecanoyl-L-phenylalanine methyl ester,
N²-hexadecanoyl-L-lysine methyl ester,
N-hexadecanoyl-L-glutamic acid 1-methyl ester,
and a salt thereof.

7. The immunostimulating agent according to claim 1, wherein the compound represented by the formula (I) or a salt thereof is selected from the group consisting of
N-acetyl-L-leucine methyl ester,
N-hexanoyl-L-leucine methyl ester,
N-(2-octadecyleicosanoyl)-L-leucine methyl ester,
N-hexadecanoyl-L-leucine tert-butyl ester,
N-hexadecanoyl-L-leucinamide,
N²-hexadecanoyl-N¹,N¹-diethyl-L-leucinamide,
N-hexadecanoyl-L-histidine methyl ester,
N-hexadecanoyl-L-proline methyl ester,
N-hexadecanoyl-L-serine methyl ester,
N-hexadecanoyl-L-leucine hexyl ester,
N²-hexadecanoyl-L-arginine hexyl ester,
N²-docosanoyl-N¹,N¹-diethyl-L-glutamic acid 1-amide,
N²-hexadecanoyl-N¹,N¹-diethyl-L-arginine amide,
N²-docosanoyl-L-arginine hexyl ester,
N-docosanoyl-L-leucine hexyl ester,
N²-docosanoyl-N¹,N¹-diethyl-L-leucinamide,
N²-docosanoyl-L-arginine amide,
N²-docosanoyl-N¹,N¹-diethyl-L-arginine amide,
N²-hexanoyl-L-arginine hexyl ester,
N-hexadecanoyl-L-glutamic acid hexyl ester,
N-docosanoyl-L-glutamic acid hexyl ester,
N²-hexadecanoyl-L-glutamine methyl ester,
and a salt thereof.

8. A pharmaceutical composition comprising at least one kind of a compound represented by the formula (I): 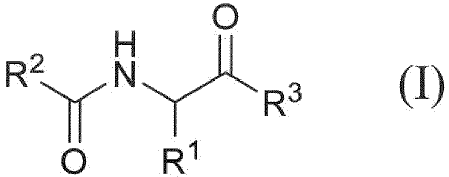 wherein
R¹ is an amino acid side chain excluding a cystine side chain;
R² is a C₁-₃₇ alkyl group; and
R³ is a hydroxyl group, a C₁₋₆ alkoxy group or -NR⁴R⁵ wherein R⁴ and R⁵ are the same or different and each is a hydrogen atom or a C₁₋₆ alkyl group,
or a salt thereof, and
α -cyclodextrin.

9. The pharmaceutical composition according to claim 8, wherein R¹ is selected from the group consisting of an arginine side chain, a glutamine side chain, a glutamic acid side chain, a hydrogen atom, an isoleucine side chain, a leucine side chain, a lysine side chain, a phenylalanine side chain, and a valine side chain.

10. The pharmaceutical composition according to claim 8, wherein R¹ is selected from the group consisting of a histidine side chain, a proline side chain, and a serine side chain.

11. The pharmaceutical composition according to any one of claims 8 to 10, wherein R² is a C₁₂₋₂₄ alkyl group.

12. The pharmaceutical composition according to any one of claims 8, 9 and 11, wherein the compound represented by the formula (I) or a salt thereof is selected from the group consisting of
N²-hexadecanoyl-L-arginine,
N²-hexadecanoyl-L-arginine methyl ester,
N²-octadecanoyl-L-glutamine tert-butyl ester,
N²-octadecanoyl-L-glutamine,
N-docosanoyl glycine methyl ester,
N-docosanoyl-L-leucine methyl ester,
N-docosanoyl-L-phenylalanine methyl ester,
N-docosanoyl-L-glutamic acid 1-methyl ester,
N²-docosanoyl-L-lysine methyl ester,
N-docosanoyl-L-isoleucine methyl ester,
N-docosanoyl-L-valine methyl ester,
N-hexadecanoyl glycine methyl ester,
N-hexadecanoyl-L-leucine methyl ester,
N-hexadecanoyl-L-phenylalanine methyl ester,
N²-hexadecanoyl-L-lysine methyl ester,
N-hexadecanoyl-L-glutamic acid 1-methyl ester,
and a salt thereof.

13. The pharmaceutical composition according to claim 8, wherein the compound represented by the formula (I) or a salt thereof is selected from the group consisting of
N-acetyl-L-leucine methyl ester,
N-hexanoyl-L-leucine methyl ester,
N-(2-octadecyleicosanoyl)-L-leucine methyl ester,
N-hexadecanoyl-L-leucine tert-butyl ester,
N-hexadecanoyl-L-leucinamide,
N²-hexadecanoyl-N¹,N¹-diethyl-L-leucinamide,
N-hexadecanoyl-L-histidine methyl ester,
N-hexadecanoyl-L-proline methyl ester,
N-hexadecanoyl-L-serine methyl ester,
N-hexadecanoyl-L-leucine hexyl ester,
N²-hexadecanoyl-L-arginine hexyl ester,
N²-docosanoyl-N¹,N¹-diethyl-L-glutamic acid 1-amide,
N²-hexadecanoyl-N¹,N¹-diethyl-L-arginine amide,
N²-docosanoyl-L-arginine hexyl ester,
N-docosanoyl-L-leucine hexyl ester,
N²-docosanoyl-N¹,N¹-diethyl-L-leucinamide,
N²-docosanoyl-L-arginine amide,
N²-docosanoyl-N¹,N¹-diethyl-L-arginine amide,
N²-hexanoyl-L-arginine hexyl ester,
N-hexadecanoyl-L-glutamic acid hexyl ester,
N-docosanoyl-L-glutamic acid hexyl ester,
N²-hexadecanoyl-L-glutamine methyl ester,
and a salt thereof.

14. A vaccine comprising at least one kind of a compound represented by the formula (I): 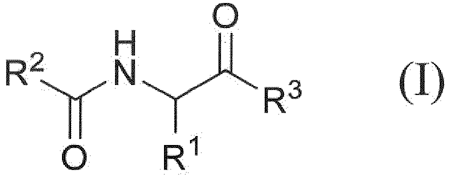 wherein
R¹ is an amino acid side chain excluding a cystine side chain;
R² is a C₁₋₃₇ alkyl group; and
R³ is a hydroxyl group, a C₁₋₆ alkoxy group or -NR⁴R⁵ wherein R⁴ and R⁵ are the same or different and each is a hydrogen atom or a C₁₋₆ alkyl group,
or a salt thereof, and
antigen.

15. The vaccine according to claim 14, wherein R¹ is selected from the group consisting of an arginine side chain, a glutamine side chain, a glutamic acid side chain, a hydrogen atom, an isoleucine side chain, a leucine side chain, a lysine side chain, a phenylalanine side chain, and a valine side chain.

16. The vaccine according to claim 14, wherein R¹ is selected from the group consisting of histidine side chain, a proline side chain, and a serine side chain.

17. The vaccine according to any one of claims 14 to 16, wherein R² is a C₁₂₋₂₄ alkyl group.

18. The vaccine according to any one of claims 14, 15 and 17, wherein the compound represented by the formula (I) or a salt thereof is selected from the group consisting of
N²-hexadecanoyl-L-arginine,
N²-hexadecanoyl-L-arginine methyl ester,
N²-octadecanoyl-L-glutamine tert-butyl ester,
N²-octadecanoyl-L-glutamine,
N-docosanoyl glycine methyl ester,
N-docosanoyl-L-leucine methyl ester,
N-docosanoyl-L-phenylalanine methyl ester,
N-docosanoyl-L-glutamic acid 1-methyl ester,
N²-docosanoyl-L-lysine methyl ester,
N-docosanoyl-L-isoleucine methyl ester,
N-docosanoyl-L-valine methyl ester,
N-hexadecanoyl glycine methyl ester,
N-hexadecanoyl-L-leucine methyl ester,
N-hexadecanoyl-L-phenylalanine methyl ester,
N²-hexadecanoyl-L-lysine methyl ester,
N-hexadecanoyl-L-glutamic acid 1-methyl ester,
and a salt thereof.

19. The vaccine according to claim 14, wherein the compound represented by the formula (I) or a salt thereof is selected from the group consisting of
N-acetyl-L-leucine methyl ester,
N-hexanoyl-L-leucine methyl ester,
N-(2-octadecyleicosanoyl)-L-leucine methyl ester,
N-hexadecanoyl-L-leucine tert-butyl ester,
N-hexadecanoyl-L-leucinamide,
N²-hexadecanoyl-N¹,N¹-diethyl-L-leucinamide,
N-hexadecanoyl-L-histidine methyl ester,
N-hexadecanoyl-L-proline methyl ester,
N-hexadecanoyl-L-serine methyl ester,
N-hexadecanoyl-L-leucine hexyl ester,
N²-hexadecanoyl-L-arginine hexyl ester,
N²-docosanoyl-N¹,N¹-diethyl-L-glutamic acid 1-amide,
N²-hexadecanoyl-N¹,N¹-diethyl-L-arginine amide,
N²-docosanoyl-L-arginine hexyl ester,
N-docosanoyl-L-leucine hexyl ester,
N²-docosanoyl-N¹,N¹-diethyl-L-leucinamide,
N²-docosanoyl-L-arginine amide,
N²-docosanoyl-N¹,N¹-diethyl-L-arginine amide,
N²-hexanoyl-L-arginine hexyl ester,
N-hexadecanoyl-L-glutamic acid hexyl ester,
N-docosanoyl-L-glutamic acid hexyl ester,
N²-hexadecanoyl-L-glutamine methyl ester,
and a salt thereof.

20. The vaccine according to any one of claims 14 to 19, which is for subcutaneous administration or transnasal administration.
